# EUROPEAN PATENT SPECIFICATION

(11) **EP 3 177 733 B1**
(45) Date of publication and mention of the grant of the patent: **26.09.2018**
(21) Application number: 15747477.6
(22) Date of filing: 05.08.2015
(51) Int. Cl.: C12Q 1/68

(54) **SINGLE-STRANDED OLIGONUCLEOTIDE PROBES FOR CHROMOSOME OR GENE COPY ENUMERATION**
EINZELSTRÄNGIGE OLIGONUKLEOTIDSONDEN FÜR CHROMOSOMEN- ODER GENKOPIEZÄHLUNG
SONDES OLIGONUCLÉOTIDIQUES MONOCATÉNAIRES DESTINÉES AU DÉNOMBREMENT DE CHROMOSOMES OU DE COPIES DE GÈNES

(30) Priority: 06.08.2014 US 201462034035 P
(43) Date of publication of application: 14.06.2017
(73) Proprietor: Ventana Medical Systems, Inc., Tucson, Arizona 85755 (US)
(72) Inventor: HUBBARD, Antony, Tucson, Arizona 85719 (US); TANG, Lei, Oro Valley, Arizona 85737 (US); ZHANG, Wenjun, Tucson, Arizona 85737 (US); BACA PARKINSON, Lesli, Tucson, Arizona 85705 (US)
(74) Representative: Burger, Alexander
(86) International application number: PCT/EP2015/068041
(87) International publication number: WO 2016/020429

(56) References cited:
- ZHANG WENJUN ET AL: "Oligonucleotide PIK3CA/Chromosome 3 Dual in Situ Hybridization Automated Assay with Improved Signals, One-Hour Hybridization, and No Use of Blocking DNA.", THE JOURNAL OF MOLECULAR DIAGNOSTICS : JMD SEP 2015, EPUB 09 JULY 2015;, vol. 17, no. 5, September 2015 (2015-09), pages 496-504, XP002745036, ISSN: 1943-7811
- NAKAYAMA KENTARO ET AL: "Sequence mutations and amplification of PIK3CA and AKT2 genes in purified ovarian serous neoplasms.", CANCER BIOLOGY & THERAPY JUL 2006, vol. 5, no. 7, July 2006 (2006-07), pages 779-785, XP002745037, ISSN: 1538-4047 cited in the application
- DE MARCO CARMELA ET AL: "Multiple genetic alterations within the PI3K pathway are responsible for AKT activation in patients with ovarian carcinoma.", PLOS ONE 2013, E55362, vol. 8, no. 2, 2013, pages 1-18, XP002745038, ISSN: 1932-6203, DOI: 10.1371/journal.pone.0055362 cited in the application
- ALEXANDROV I A ET AL: "Segment Substitutions in Alpha Satellite DNA - Unusual Structure of Human Chromosome 3-specific Alpha Satellite Repeat Unit", JOURNAL OF MOLECULAR BIOLOGY, ACADEMIC PRESS, UNITED KINGDOM, vol. 231, no. 2, 20 May 1993 (1993-05-20), pages 516-520, XP024008992, ISSN: 0022-2836, DOI: 10.1006/JMBI.1993.1302 [retrieved on 1993-05-20] cited in the application
- REDON R ET AL: "A simple specific pattern of chromosomal aberrations at early stages of head and neck squamous cell carcinomas: PIK3CA but not p63 gene as a likely target of 3q26-qter gains.", CANCER RESEARCH 15 MAY 2001, vol. 61, no. 10, 15 May 2001 (2001-05-15), pages 4122-4129, XP002745039, ISSN: 0008-5472
- SPOERKE JILL M ET AL: "Phosphoinositide 3-kinase (PI3K) pathway alterations are associated with histologic subtypes and are predictive of sensitivity to PI3K inhibitors in lung cancer preclinical models.", CLINICAL CANCER RESEARCH : AN OFFICIAL JOURNAL OF THE AMERICAN ASSOCIATION FOR CANCER RESEARCH 15 DEC 2012, vol. 18, no. 24, 15 December 2012 (2012-12-15), pages 6771-6783, XP002745040, ISSN: 1078-0432 cited in the application

## Description

### SEQUENCE LISTING

The instant application contains a Sequence Listing which has been submitted electronically in ASCII format and is hereby incorporated by reference in its entirety. Said ASCII copy, created on July 17, 2015, is named 32190_WO_SL.txt and is 887,822 bytes in size.

### FIELD

This disclosure relates to oligonucleotide probes, systems, kits, and methods for using said probes and systems for chromosome enumeration, for detection of nucleic acid target sequences (e.g., genomic DNA or RNA), for gene copy number enumeration, and/or for tissue diagnostics.

### BACKGROUND

Probes have been developed for a variety of diagnostic and research purposes. Hybridization of chromosome or gene-specific probes has made possible detection of chromosomal abnormalities associated with numerous diseases and syndromes, including constitutive genetic anomalies, such as microdeletion syndromes, chromosome translocations, gene amplification and aneuploidy syndromes, neoplastic diseases, as well as pathogen infections. Detection of genetic changes in these regions can provide diagnostic and prognostic information for patients and in some cases, inform treatment decisions.

However, existing probes for detection and enumeration of human chromosome 3 (CHR3) require long assay times and are not desirable. For example, chromosome 3 probes are typically generated from plasmid pHS05, which targets chromosome 3 alpha satellite sequences, or from BACs for a site-specific region (Yurov, Mitkevich et al. 1987; Woenckhaus, Steger et al. 2007; Agell, Hernandez et al. 2011; Jang, Yonescu et al. 2006; De Marco, Rinaldo et al. 2013; Nakayama et al. 2006). Unfortunately, these double-stranded probes have repetitive sequences that are common to centromere regions of other human chromosome. As such, blocking DNA is used in conjunction with these probes to help reduce non-specific binding. Further, assays employing these probes require extensive hybridization time to achieve sufficient hybridization, e.g., about 12 to 18 hours.

The use of single strand probes has a distinct advantage over the use of double probes. Single-stranded probes generally have higher sensitivity than double-stranded probes because a proportion of the denatured double-stranded probe renatures to form probe homoduplexes, thus preventing their capture of genomic targets in the test samples [Taneja K and Singer RH. (1987), Lewis ME, Sherman TG, Watson SJ. (1985); Strachan T, Read AP. (1999); Kourilsky P, Mercereau O, Gros D, Tremblay G. (1974)]. Several laboratories have reported that single-stranded probes provide higher sensitivity on hybridization than double-stranded probes (An SF, Franklin D, Fleming KA. (1992); Hannon K, Johnstone E, Craft LS, et al (1993); Cox KH, DeLeon DV, Angerer LM, Angerer RC. (1984).

Despite the appeal of the use of a single strand probe in the detection and enumeration of human chromosome 3, the general consensus by workers in this field is that it is not possible to make single strand probes that are specific enough to chromosome 3. The major concern is the off-target hits of a short single strand probe to other chromosome targets. Another concern is the robustness of a small number of short single strand probes. It was found that human chromosome 3-specific alpha satellite contained a ∼2,900 base pair repeat unit that consists of 17 monomers (Alexandrov, I. A., et al. (1993). Each individual may carry different combinations of these 17 monomers and their related variants. A limited number of short single strand probes (e.g., 2-5) may not be robust enough to detect chromosome 3 polymorphism in populations (Yurov, Y. B., et al. 1987). As such, prior to the present invention, probes for detection and enumeration of human chromosome 3 are not desirable and not time efficient.

Lung cancer is the leading cause of cancer-related mortality worldwide, with an estimate of 1.4 million deaths in 2010. In the United States, more than 220,000 new cases and more than 157,000 deaths annually with lung cancer, non-small cell lung cancer (NSCLC) accounts for 85% of all lung cancers. Adenocarcinoma and squamous-cell carcinoma (SCC) are the most frequent histological subtypes accounting for 50% and 30% of NSCLC cases respectively (Minna, Roth et al. 2002). Historically, approaches to the treatment of NSCLC were uniform, and histologic subtypes within NSCLC did not significantly affect treatment decisions. In the past ten years, knowledge of genetic aberrances of lung adenocarcinoma has been identified in most cases of lung adenocarcinoma; targeted drugs like EGFR tyrosine kinase inhibitors and ALK inhibitors are now applied to about one third of the patients (Zochbauer-Muller, Gazdar et al. 2002, Cooper, Lam et al. 2013, Villaruz, Burns et al. 2013). Unlike adenocarcinoma of the lung, SCC remains a relatively poorly understood disease that lacks a consensus molecular profile, although recent work is overcoming these challenges (Ji, Guan et al. 2011, Lockwood, Wilson et al. 2012). Gain of chromosome 3q25-qter was revealed almost exclusively in SCC, among them the PIK3CA which encodes the catalytic alpha subunit (p110α) of phosphatidylinositol (PI) 3-kinase (PI3K) (Kok, Geering et al. 2009, Massion, Kuo et al. 2002, Okudela, Suzuki et al. 2007). Moreover, PIK3CA was found to be amplified in up to 45% of SCC cancer patients (Yamamoto, Shigematsu et al. 2008, Spoerke, O'Brien et al. 2012). A strong correlation was found between PIK3CA amplification and the increased PI3K pathway activity such as cell proliferation, survival, oncogenic RAS signaling and transformation (Woenckhaus, Steger et al. 2002). These features make PI3K an attractive target for therapeutic intervention on SCC of the lung (Wee, Lengauer et al. 2008).

Currently, several inhibitors directed against PI3K are being clinically evaluated for NSCLC treatment, where distinct candidate predictive biomarkers strategies might be needed for SCC patient populations (Salphati, Belvin et al. 2009, Blumenthal, Orbach et al. 2011, Salphati, Pang et al. 2011). In a recent study by Spoerke JM et al. (Spoerke, O'Brien et al. 2012), PIK3CA amplification was detected with fluorescent *in situ* hybridization (FISH) in 37% of SCC and only 5% of adenocarcinomas. Cell lines harboring PI3K amplification were exquisitely sensitive to the PI3K inhibitor GDC-0941. In addition, Angulo B et al. (Angulo, Suarez-Gauthier et al. 2008) demonstrated a strong statistically significant association between increased levels of PIK3CA mRNA expression and gene amplification predominantly in SCC. On the protein level, Scrima M et al. (Scrima, De Marco et al. 2012) showed the majority of NSCLCs with increased copy number of PIK3CA showed moderate or high expression of p110α.

Currently, all studies on PIK3CA copy number gain employ fluorescent *in situ* hybridization (FISH) technology. These PIK3CA FISH probes are derived from bacterial artificial chromosomes (BACs) that cover PIK3CA gene region; while most CHR3 probes are generated from plasmid pHS05 that targets chromosome 3 alpha satellite sequences or BACs for site-specific region. These double stranded probes require 12 to 18 hours hybridization in order to achieve sufficient hybridization; and require blocking DNA due to the presence of repetitive sequence common to other centromere regions which would result in non-specific binding without the addition of the blocking DNA.

### SUMMARY

The present inventors have surprisingly discovered a set of 18 unique single strand probes that are highly specific for chromosome 3. In fact, these newly discovered oligonucleotide probes are so highly specific that the use of blocking DNA can be eliminated in the assays. Furthermore, it was surprisingly discovered that these oligonucleotide probes have much enhanced hybridization kinetics that they require a significantly reduced hybridization time.

Also surprisingly, these single-stranded oligonucleotide probes to chromosome 3 resulted in discrete enumerable rounded signals. This is in contrast to the nick-translation labeled double-stranded probe, which generate signals with a wide range of size and shape.

The single-stranded oligonucleotide probes to chromosome 3 of the present invention may be used in combination with one or more target probes directed to a target gene of interest. This allows for gene copy enumeration (e.g., determination of the ratio of a target gene to its corresponding chromosome), which may be important for tissue diagnostics. As an example, PIK3CA is a gene found on chromosome 3. Commercial products and research reagents for PIK3CA gene copy number use bacterial artificial chromosomes (BACs) that cover the PIK3CA gene region (Angulo, Suarez-Gauthier et al. 2008); Shayesteh, Lu et al, 1999; Psyrri, Papageorgiou et al 2009). The BACs are double-stranded DNA probes, which require about 12 to 18 hours hybridization time. The present invention also features the use of single-stranded oligonucleotide probes to detect (and enumerate gene copy number) the PIK3CA gene on chromosome 3 in combination with chromosome 3 detection and enumeration using the aforementioned single-stranded oligonucleotide probes. This is the first and only demonstration of gene copy number enumeration using single-stranded oligonucleotide probes for both the gene and chromosome targets.

In illustrative embodiments, systems for *in situ* hybridization may comprise a control probe specific to a control region of chromosome 3, wherein the control probe is labeled with at least one first label, wherein the control probe is a first plurality of single-stranded oligonucleotide probes, each oligonucleotide probe comprising a sequence selected from the group consisting of SEQ ID NOs: 1-18, or a sequence selected from the group consisting of a truncated version of SEQ ID NOs: 1-18, the truncated version being at least 40 contiguous basepairs (bp) of said SEQ ID NOs:1-18, or a sequence selected from the group consisting of a sequence that has at least 70% sequence identity to one of SEQ ID NOs: 1-18.

In some embodiments, the systems may further comprise a target probe specific to a target region of chromosome 3, wherein the target probe is labeled with at least one second label.

In further embodiments, methods using and kits pertaining to the aforementioned systems are disclosed.

In illustrative embodiments, a kit may comprise a vessel containing the aforementioned systems and instructions for use.

In illustrative embodiments, a slide may comprise a plurality of nuclei chromogenically stained for human chromosome 3, wherein the slide is made using the aforementioned systems.

In illustrative embodiments, a method for *in situ* hybridization of a tissue sample may comprise contacting the tissue sample with the aforementioned systems.

In illustrative embodiments, a method of scoring for a chromosome for PIK3CA gene copy amplification may comprise obtaining a tissue sample having undergone the aforementioned *in situ* hybridization, wherein a control probe and target probe are used, identifying an area of neoplastic nuclei with most copy numbers, and counting enumerable signals for PIK3CA signal in 50-100 neoplastic nuclei and either 50 adjacent mesenchymal nuclei or 50 adjacent normal epithelial nuclei, wherein scoring criteria comprises: no staining or <1 dot/ 10 cells is scored as 0; 1-3 dots/cell is scored as 1; 4-9 dots/cell, none or very few dot clusters is scored as 2; 10-15 dots/cell and <10% dots are in clusters is scored as 3; and >15 dots/cell and >10% dots are in clusters is scored as 4.

Additional features of the present disclosure will become apparent to those skilled in the art upon consideration of the following detailed description of illustrative embodiments exemplifying the best mode of carrying out the disclosure as presently perceived.

### BRIEF DESCRIPTION OF THE DRAWINGS

**FIG. 1** is a schematic view of the 3q26.32 region of chromosome 3. The PIK3CA oligo probe covers the region between 178,922,283 and 179,682,019.
**FIG. 2** shows PIK3CA and CHR3 DISH staining on metaphase spread chromosomes.
**FIG. 3** shows PIK3CA and CHR3 DISH staining on Calu 3 cells.
**FIG. 4** is a hybridization time course study.
**FIG. 5** shows cross-reactive signals in a normal lung tissue stained with pHS05 plasmid (for chromosome 3) in the absence of human blocking DNA.
**FIG. 6** compares CHR3 staining with either probes of the present invention or with the pHS05 plasmid. Staining with probes of the present invention offers discrete CHR3 signals with generally uniform shape and size.
**FIG. 7** shows that the PIK3CA/CHR3 ratio from the scores of 50 nuclei was consistent with the scores from the entire 100 nuclei enumeration when choosing 50 nuclei with the highest counts.
**FIG. 8** shows the PIK3CA/CHR3 ratio, the average PIK3CA copy number, and the average CHR3 copy number profiles on the tested tissue types of the 102 lung specimens.
**FIG. 9** shows cases with PIK3CA copy number gain evaluated by PIK3CA/CHR3 ratio (>2) and average PIK3CA copy number (>4) per nuclei.
**FIG. 10** shows p110α IHC on Calu-3 xenograft (A), p110α IHC staining (B), and PIK3CA mRNA ISH (C) staining results on the 102 lung tissues.

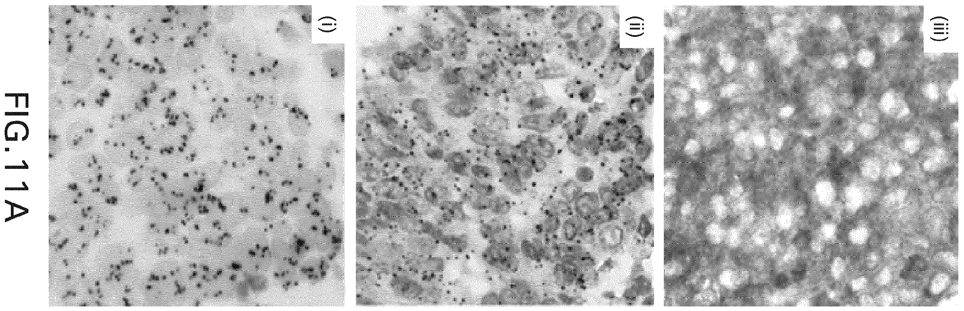

**FIG. 11A**(i-iii) shows (i) PIK3CA/CHR3 DISH, (ii) PIK3CA mRNA ISH and (iii) p110α IHC staining on a SCC (F2) with gene copy gain, increased mRNA and protein expression levels.

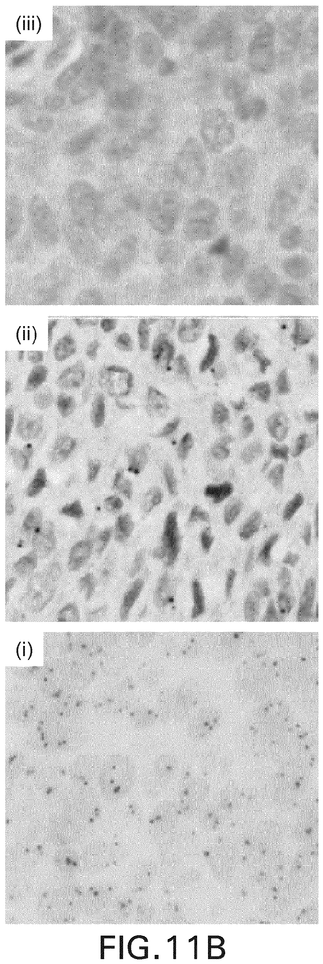

**FIG. 11B** shows (i) PIK3CA/CHR3 DISH, (ii) PIK3CA mRNA ISH and (iii) p110α IHC staining on a SCC (F3). PIK3CA gene copy number is 2.16 with ratio 0.98, mRNA level is slightly elevated (H score 75), protein expression is normal (IHC intensity 1).

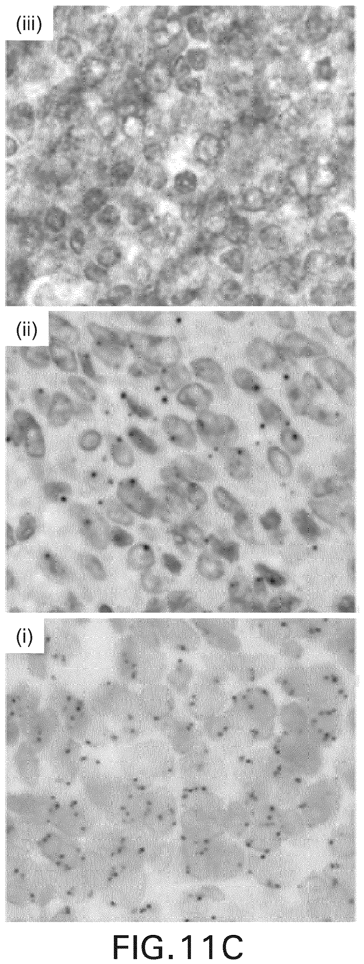

**FIG. 11C** shows (i) PIK3CA/CHR3 DISH, (ii) PIK3CA mRNA ISH and (iii) p110α IHC staining on a SCC (F5). PIK3CA gene copy number is 2.58 with ratio 1.04, mRNA level is within normal range (H score 65), but protein is overexpressed (IHC intensity 3, 50%).
**FIG. 12** shows the distribution of the CHR3 signal radii and the statistical analysis of the data.
**FIG. 13** shows the signals used for measurements (radii) in FIG. 12 and FIG. 13
**FIG. 14** shows examples of concentric circles and simple closed curves used for evaluating enumerable signals.

### SEQUENCES

The nucleic acid sequences provided herein are shown using standard letter abbreviations for nucleotide bases. Only one strand of each nucleic acid sequence is shown, but the complementary strand is understood as included by any reference to the displayed strand. In the provided sequences:
SEQ ID NOs: 1-18 are nucleic acid sequences of probes to human chromosome 3.
SEQ ID NOs: 19-1230 are nucleic acid sequences of probes to the human PIK3CA gene locus or nearby regions.

### DETAILED DESCRIPTION

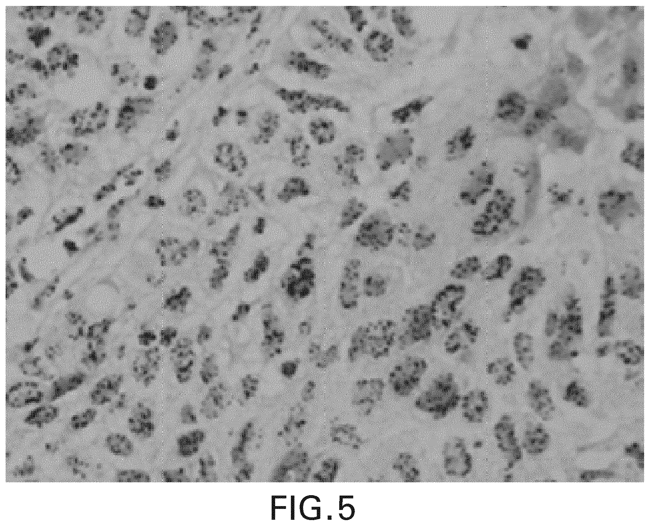

The present inventors are not aware of any successful work thus far by anyone to improve the reliability and conditions for detection and enumeration of human chromosome 3 using pHS05 plasmid-derived chromosome 3 probe. In the inventors' hands, extensive studies have been performed on the pHS05 plasmid-derived chromosome 3 probe, trying to optimize target retrieval steps, stringency wash step, and chromogen development steps, but they were not able to achieve improved quality and reliability for these assays. Additionally, the present inventors have been unable to develop assay steps to reduce or eliminate the requirement to use blocking DNA in such assays. FIG. 5 shows the excessive background signals in a hybridization assay using the pHSO5 plasmid CHR3 probe without the use of blocking DNA.

Additionally, overwhelming evidence exists that single stranded probes cannot be developed for the detection and enumeration of human chromosome 3. For example, as shown in Table 1, bioinformatics research revealed that the 80nt sequences derived from the pHS05 plasmid/chromosome 3 probe (the sequences of the centromere region of chromosome 3) had high homology to several other chromosomes (e.g., chromosome 5, 10, 12, 14, 16, 21, 22 etc.).

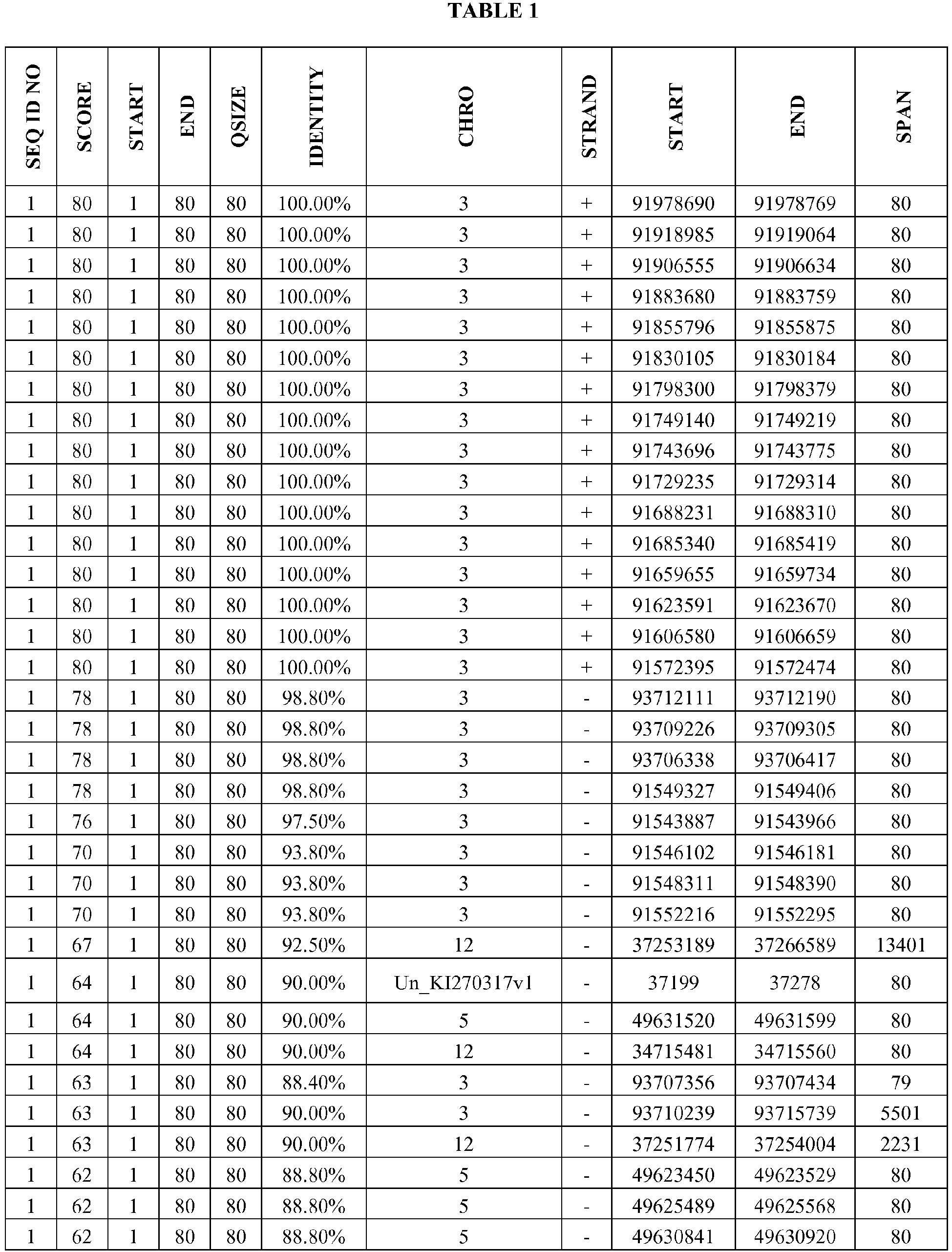

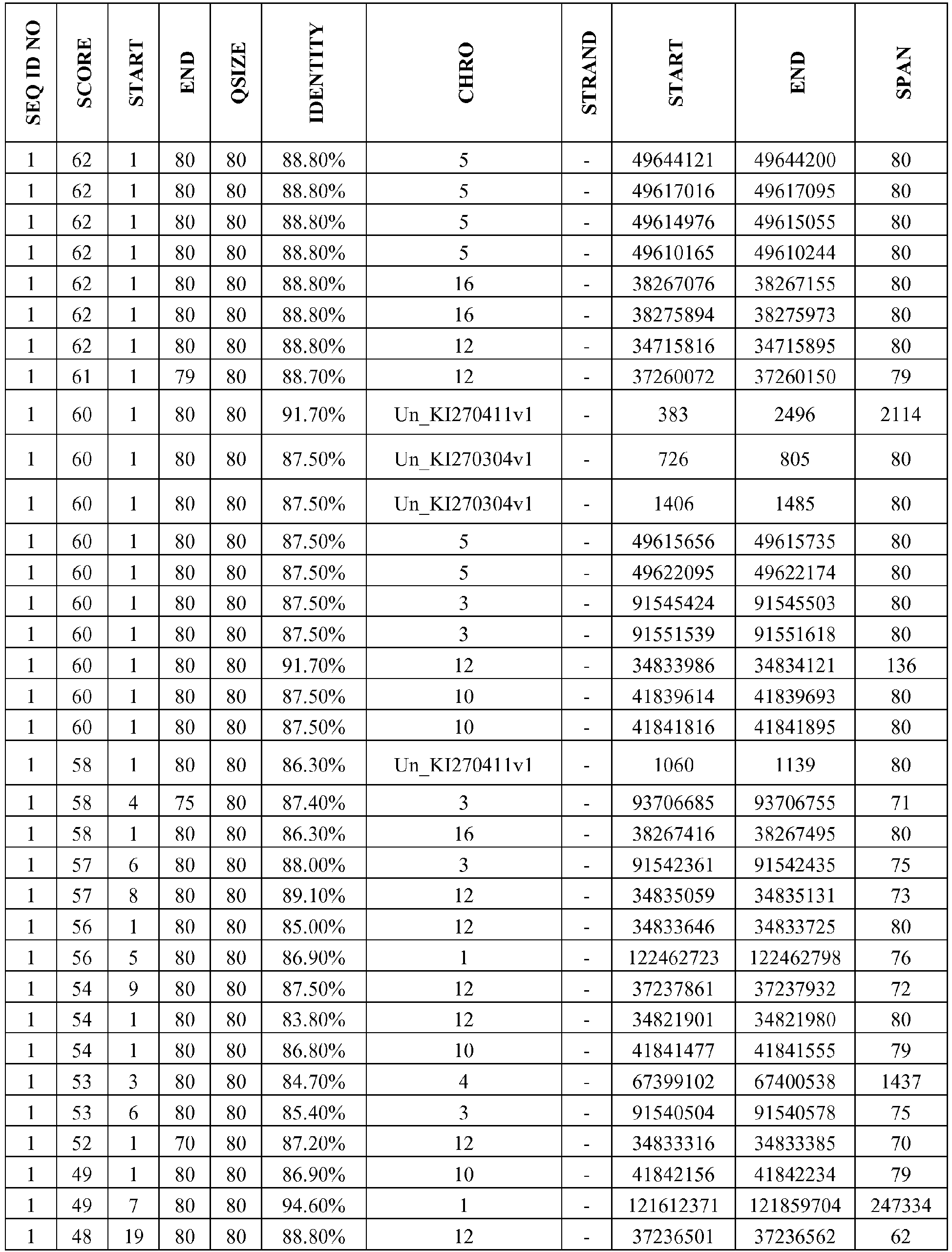

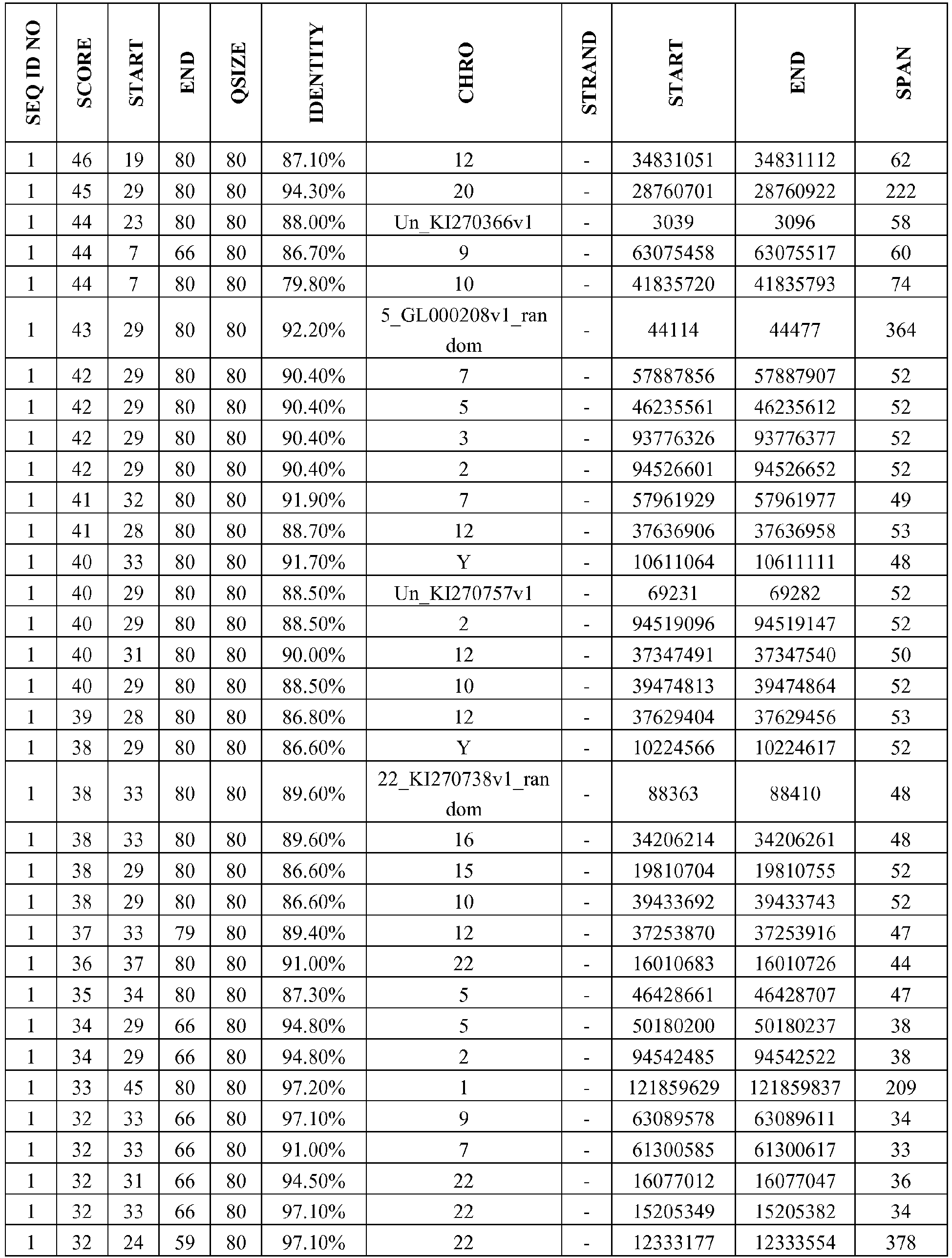

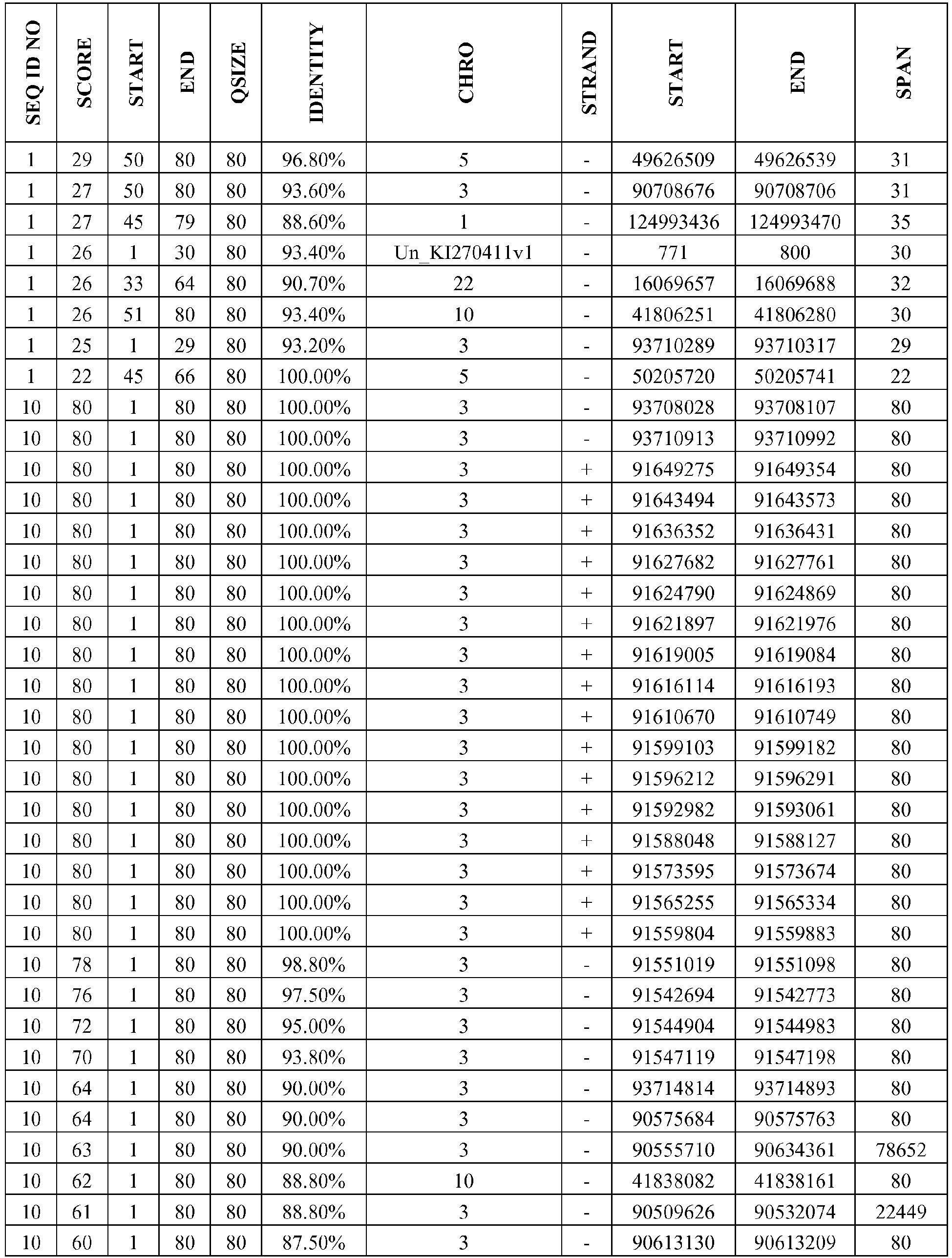

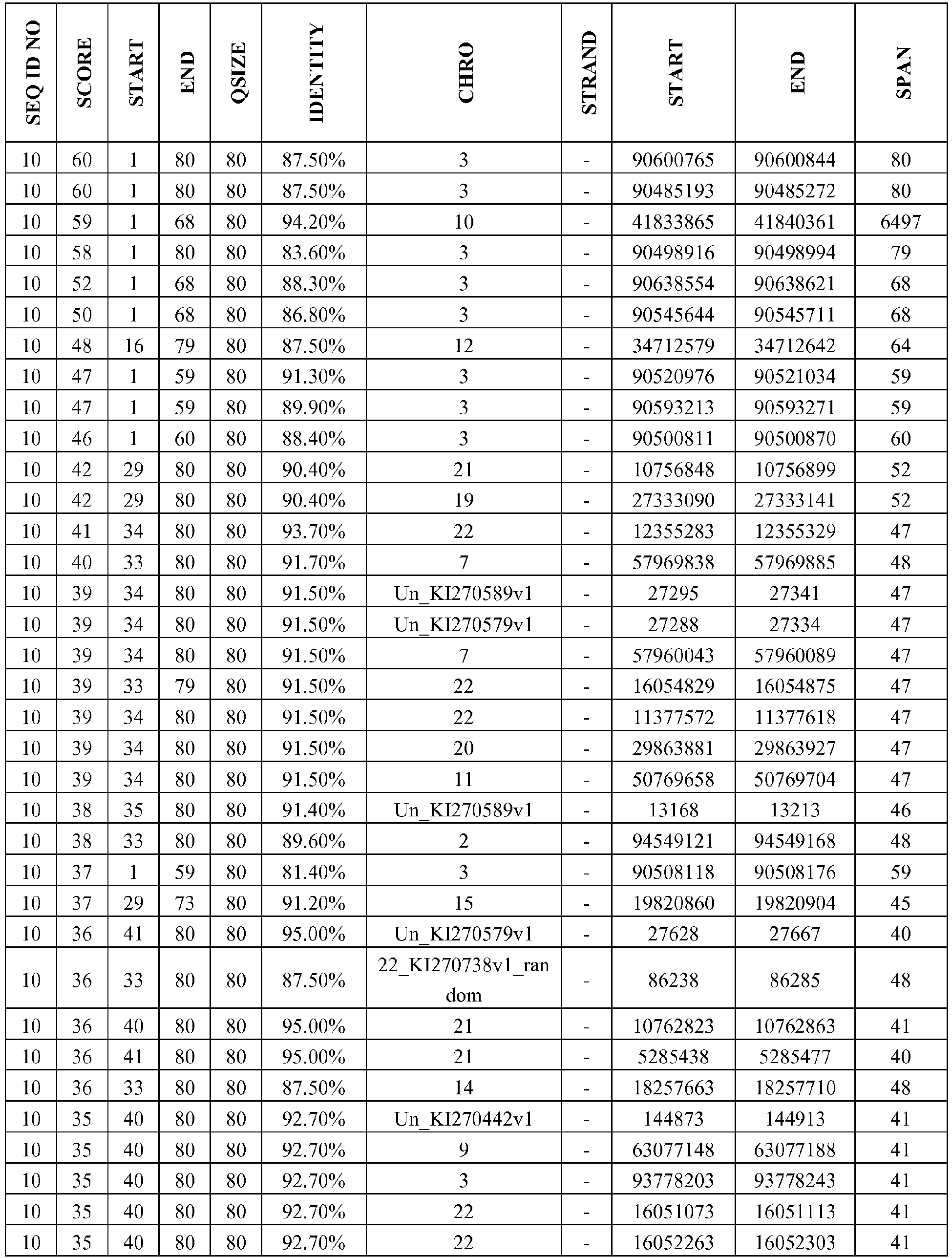

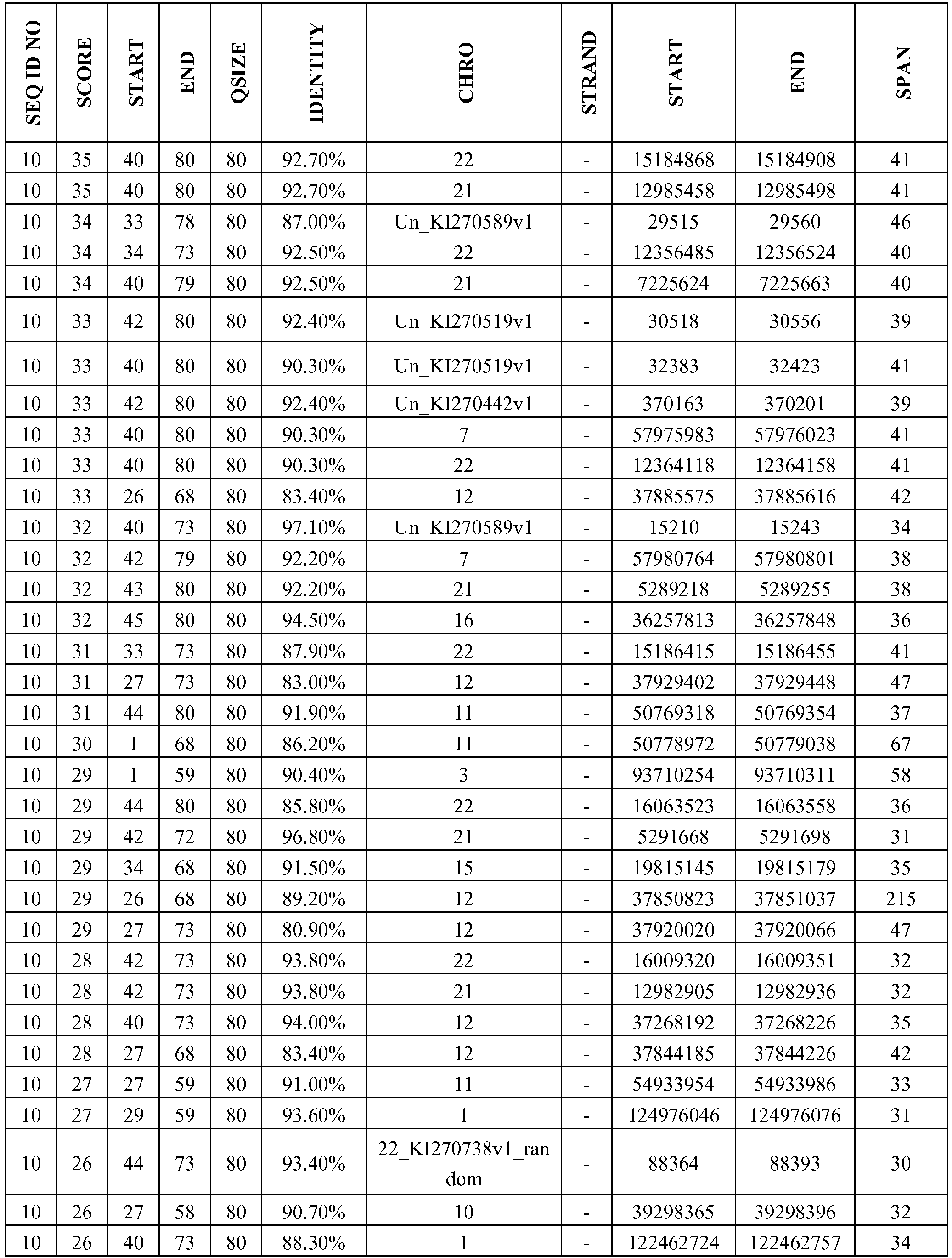

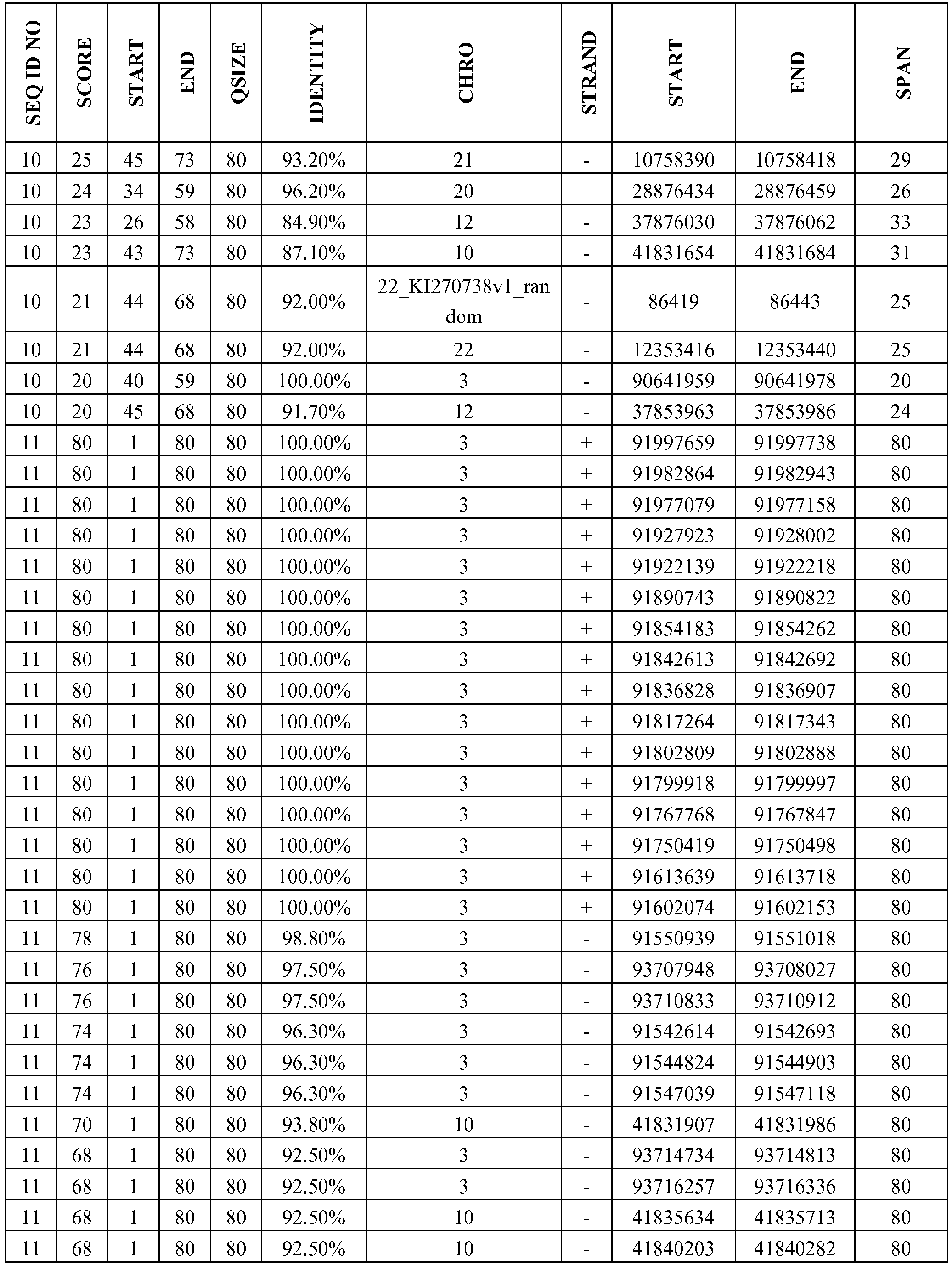

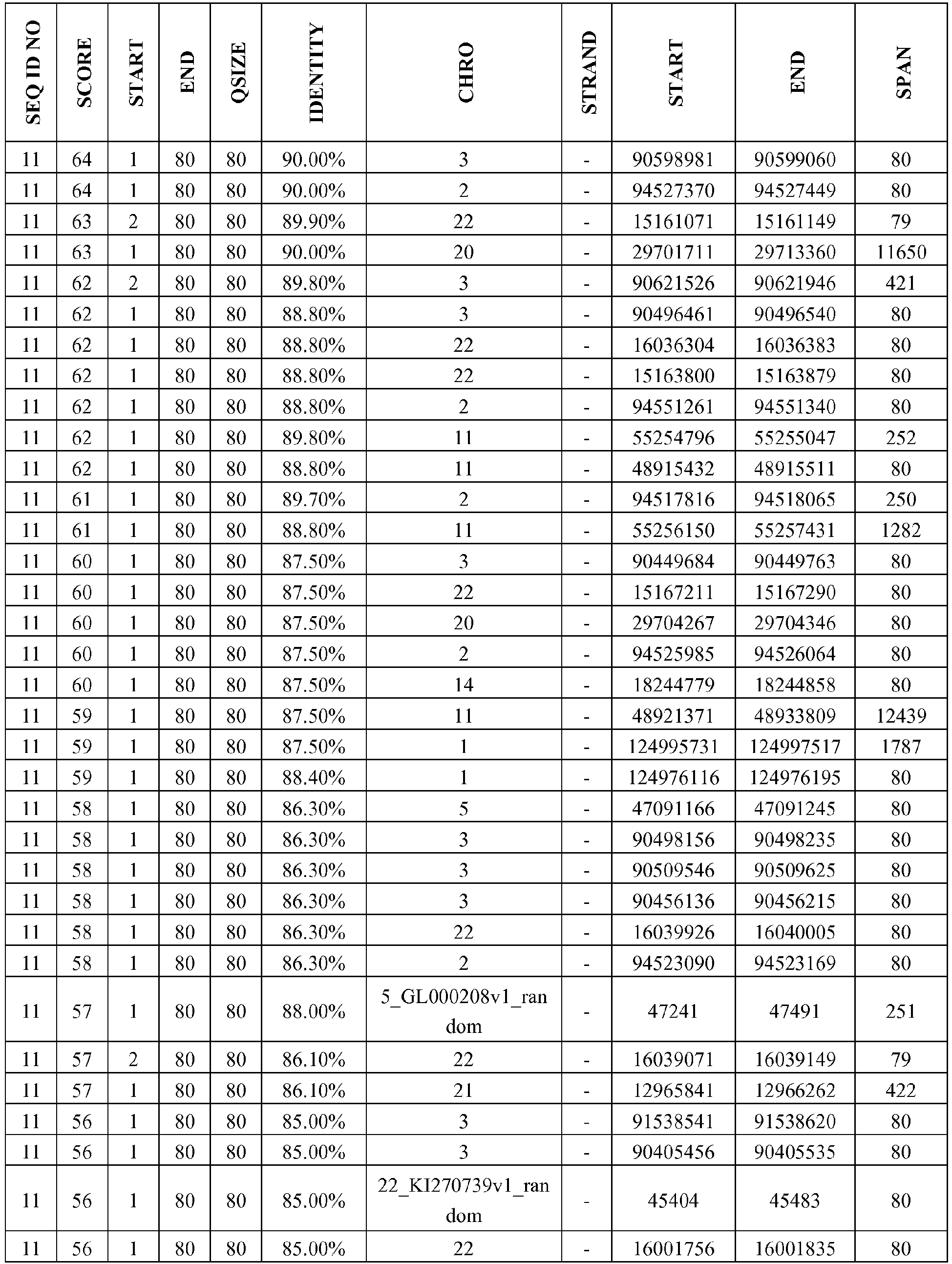

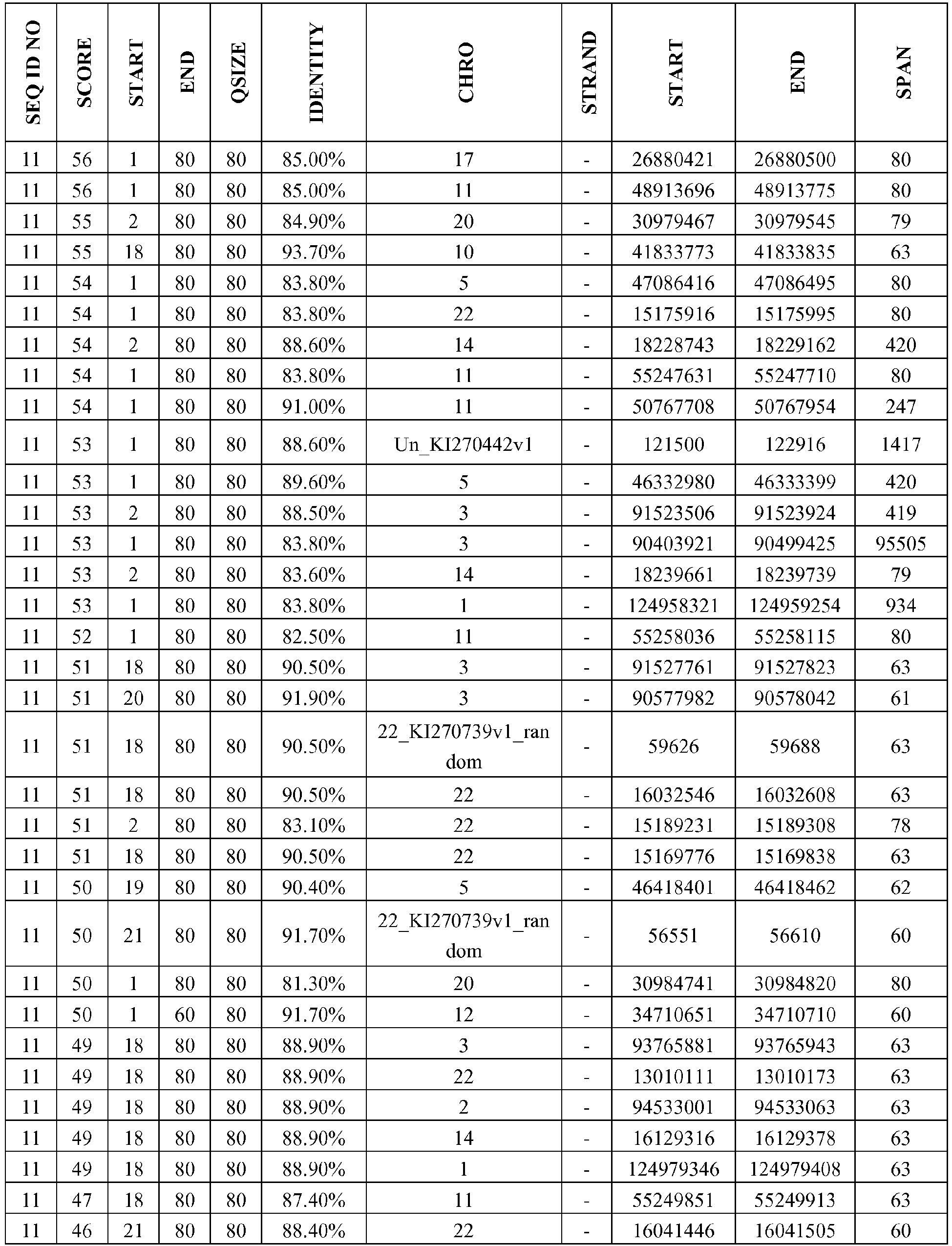

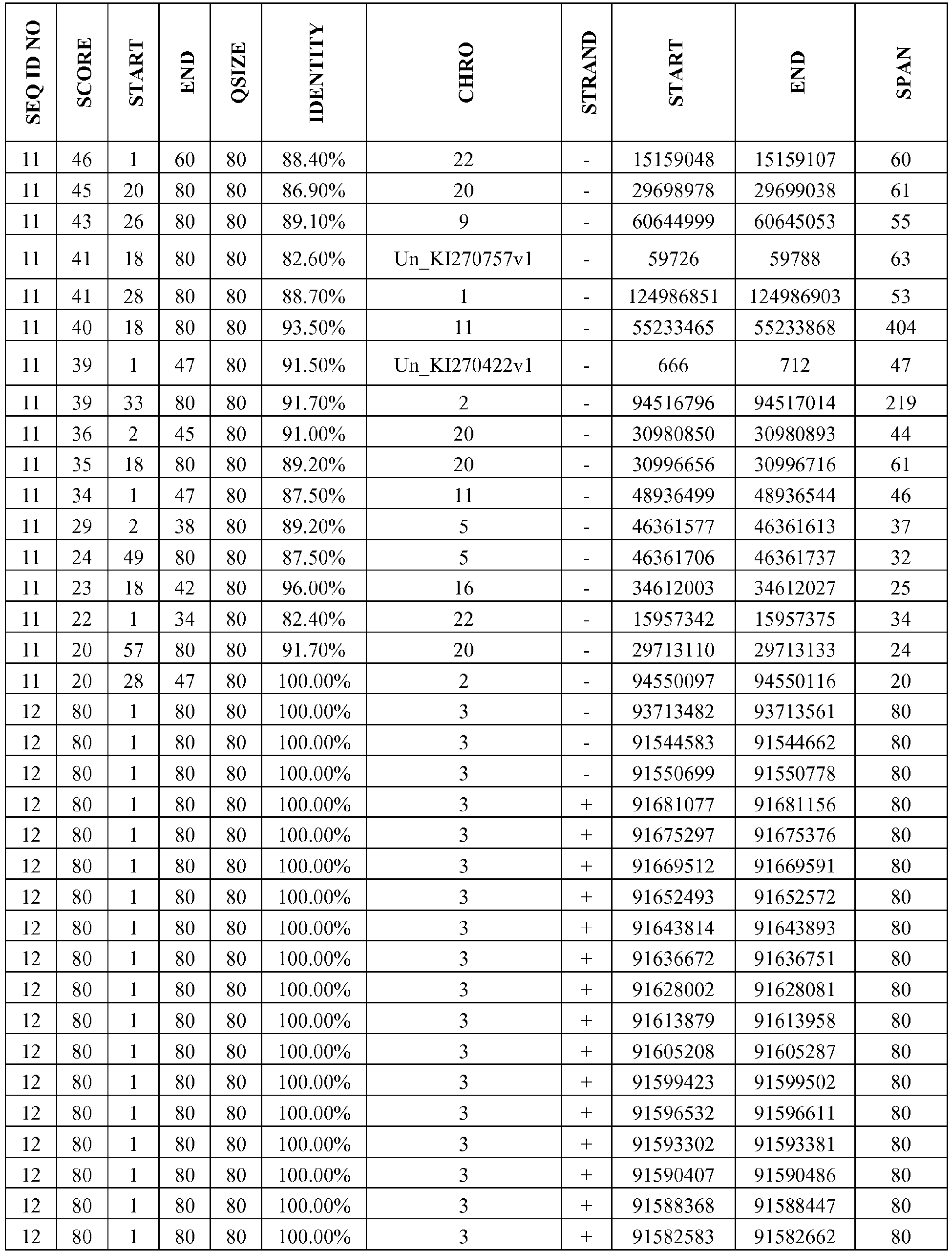

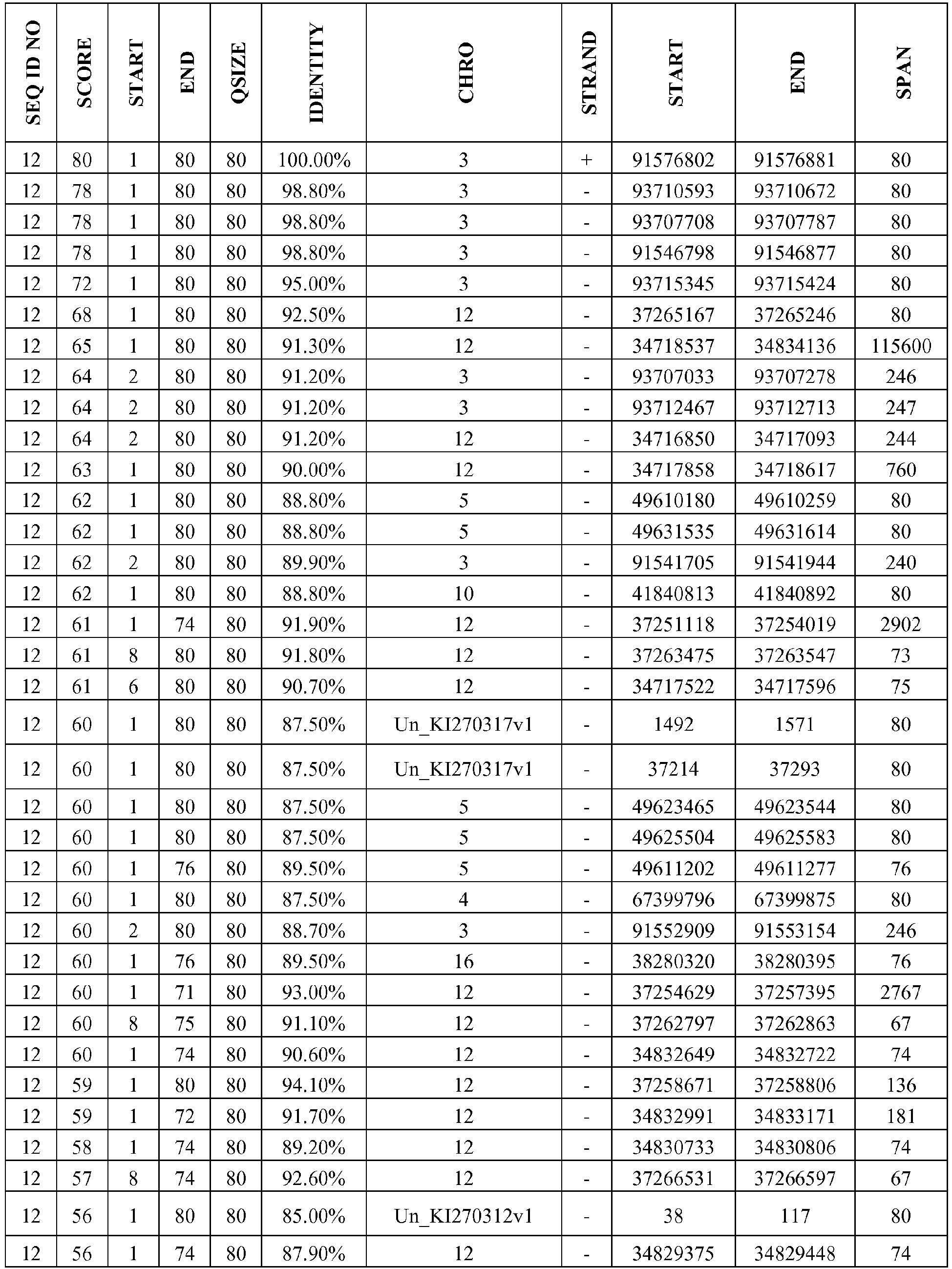

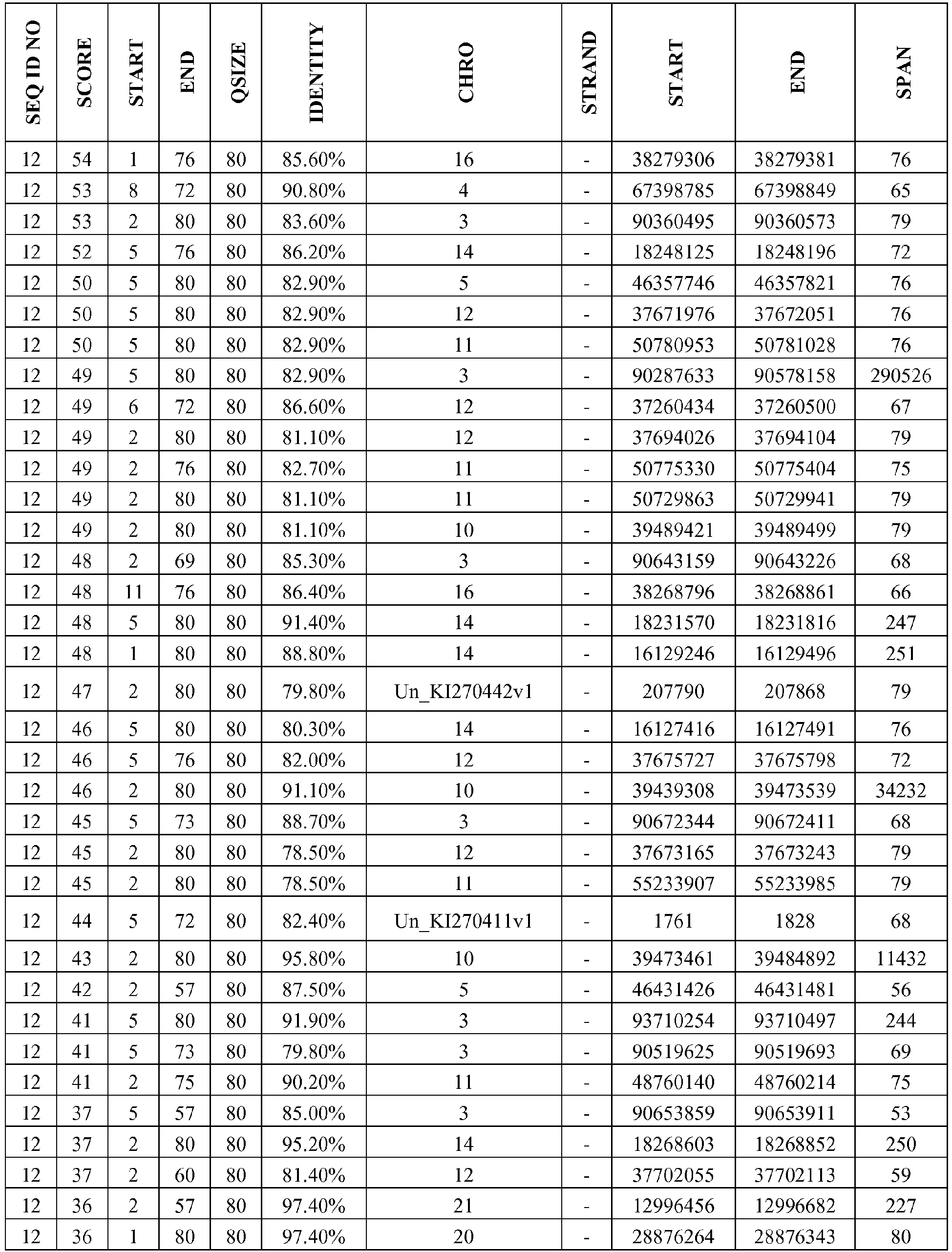

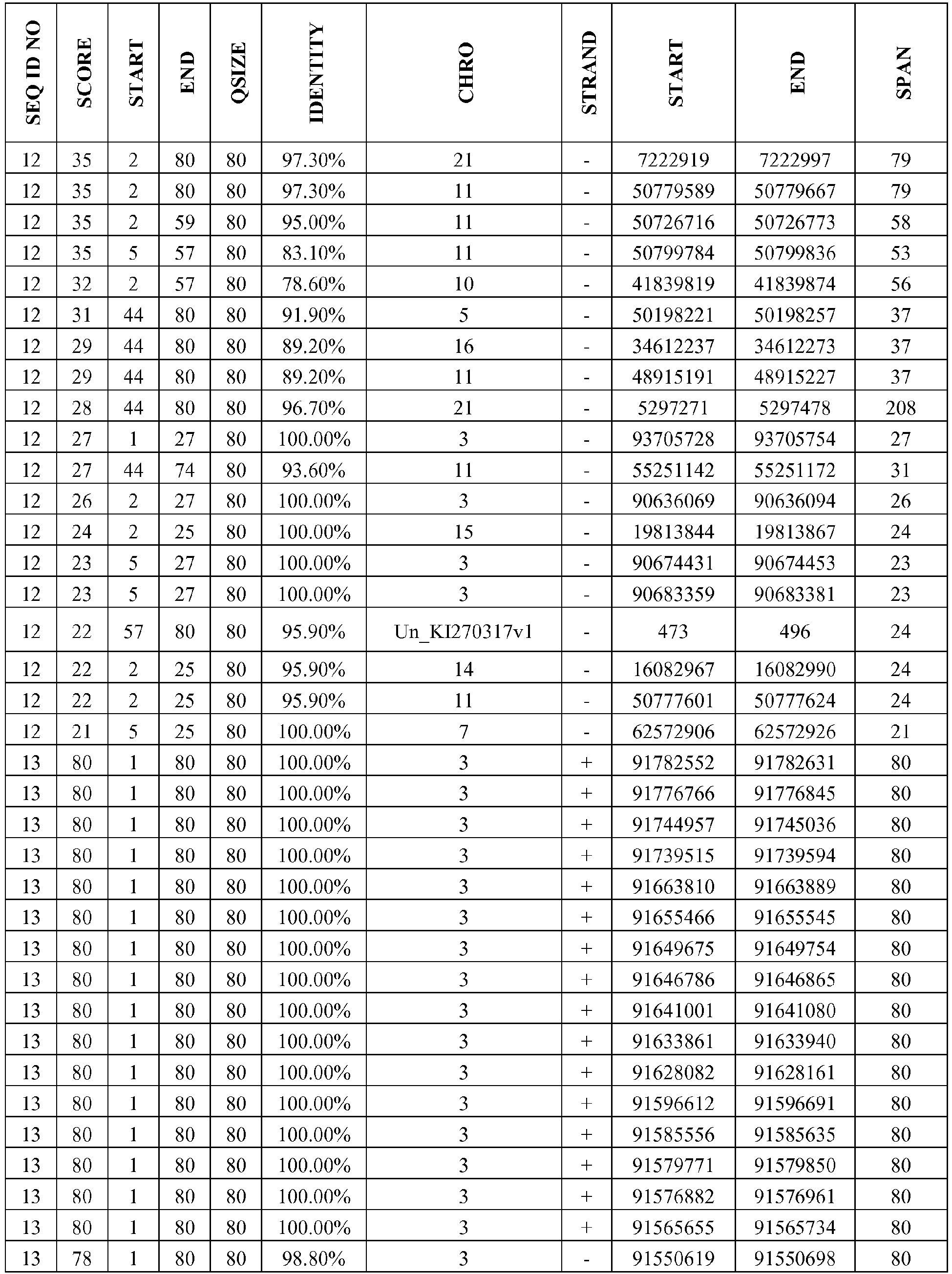

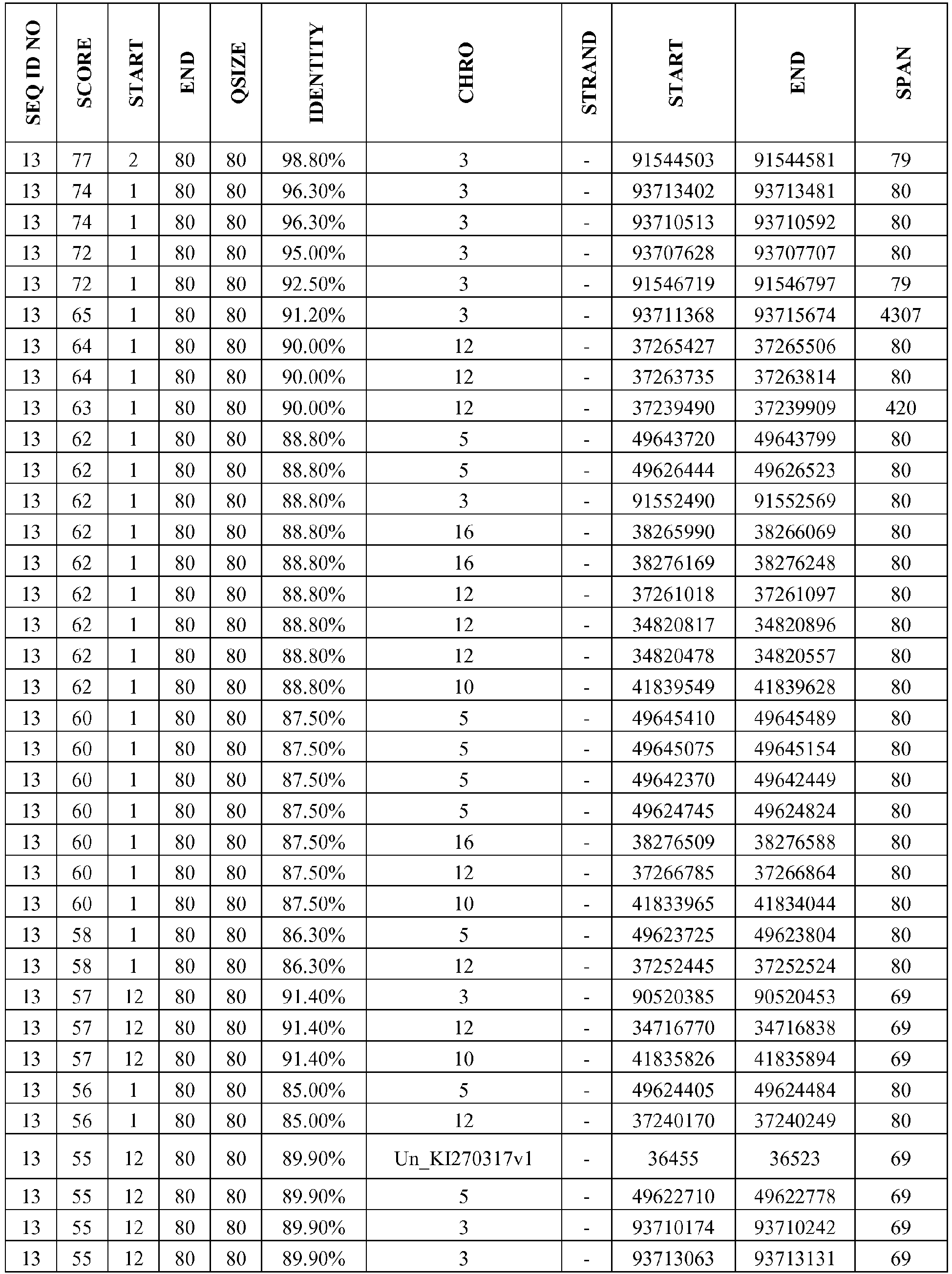

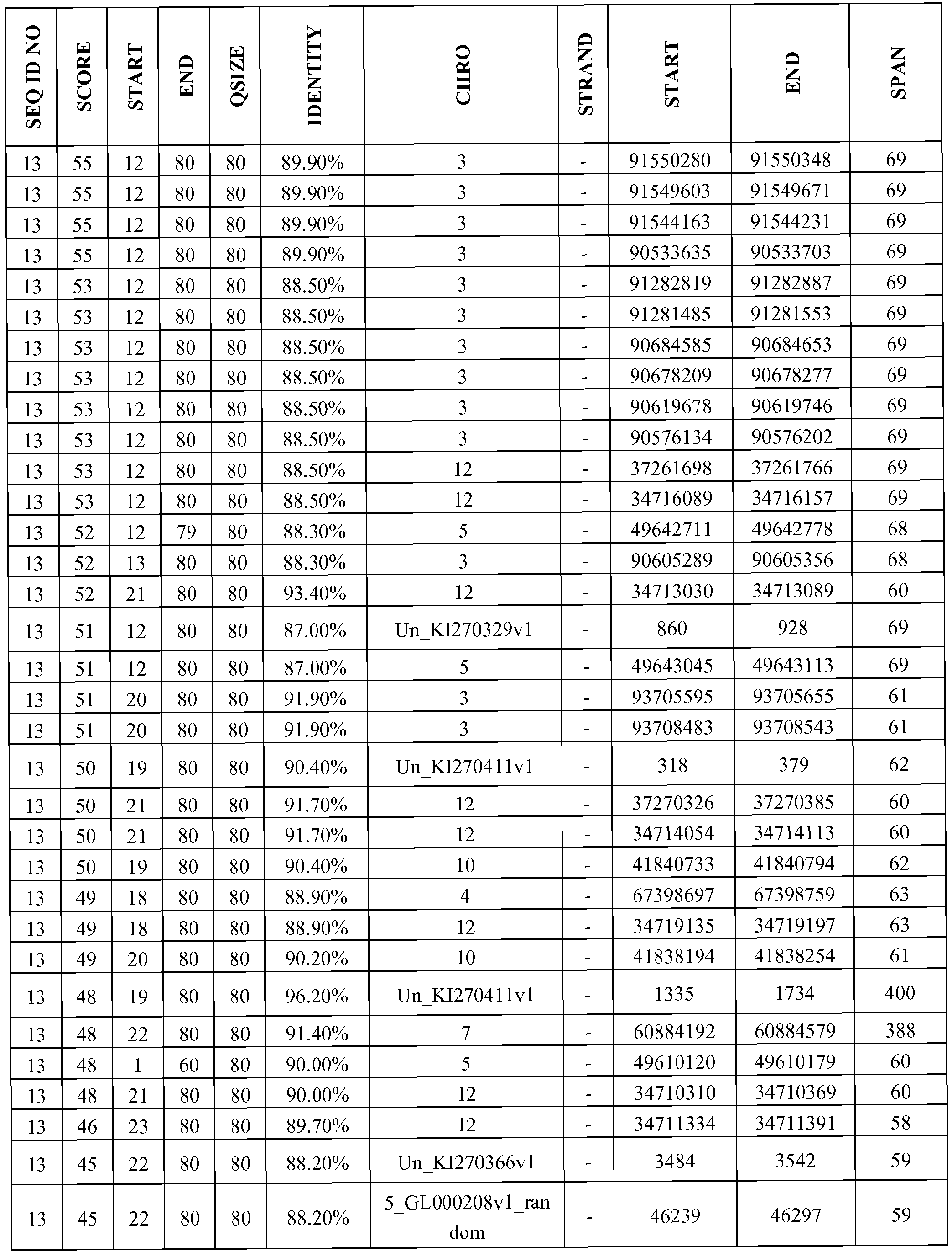

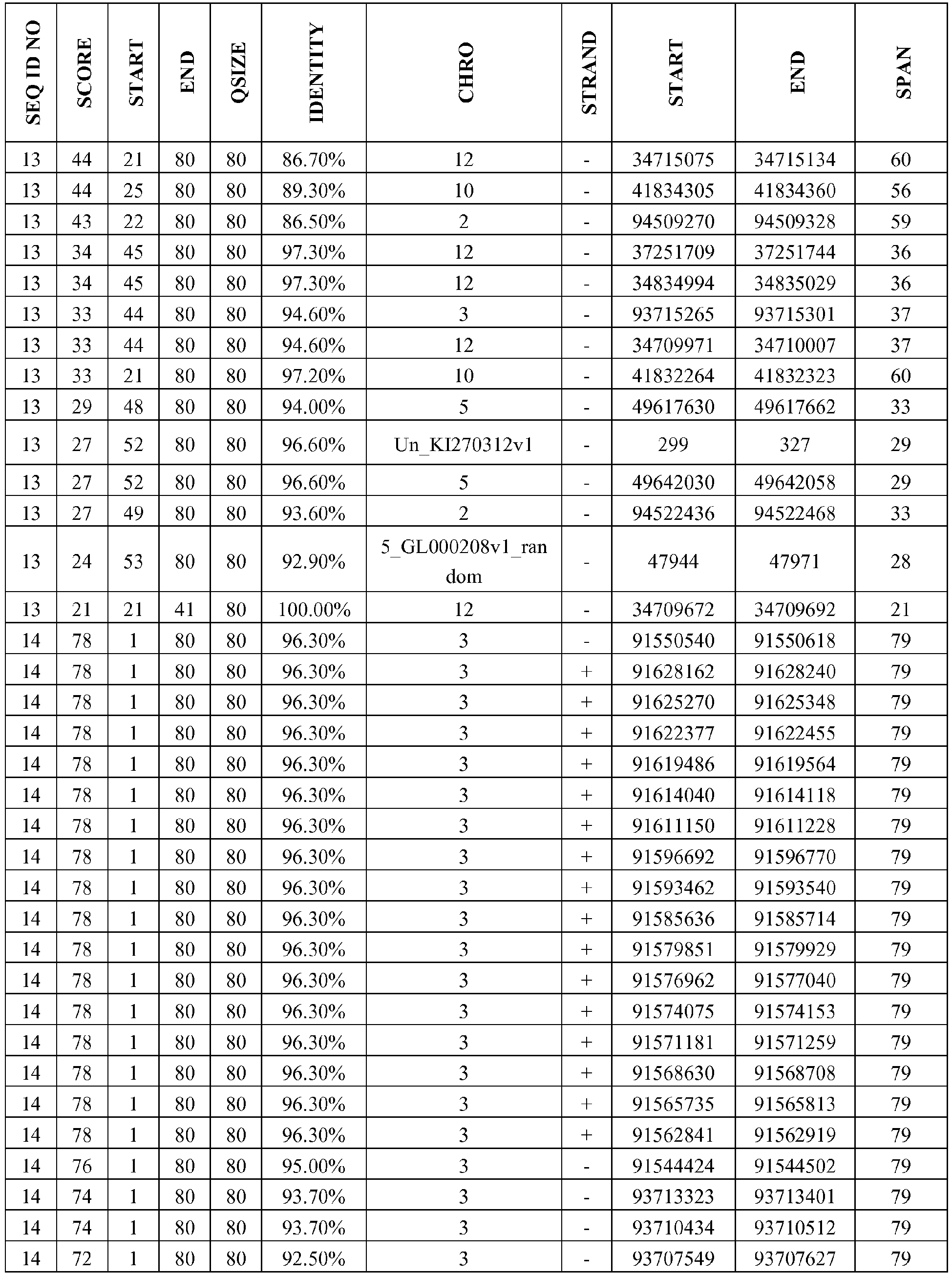

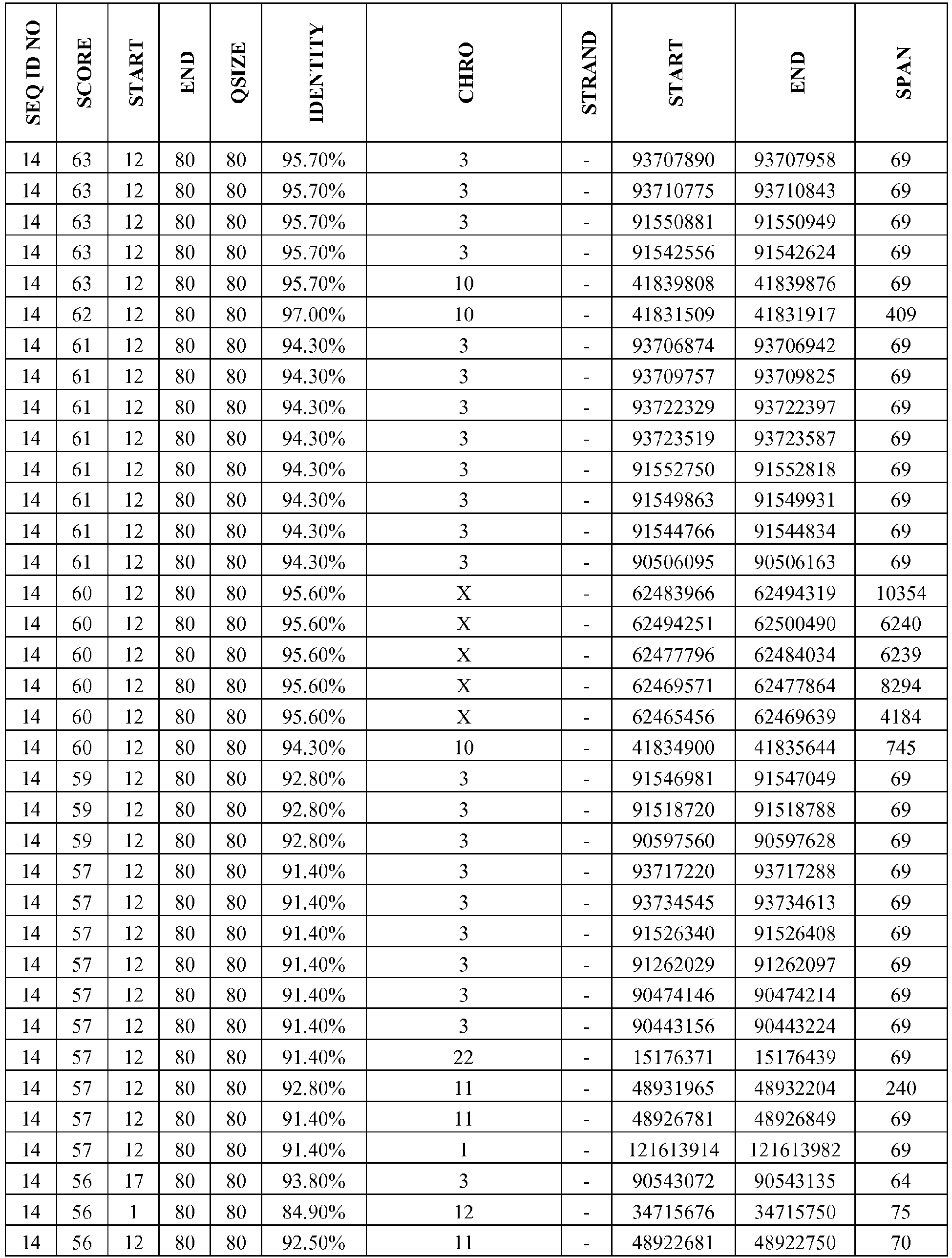

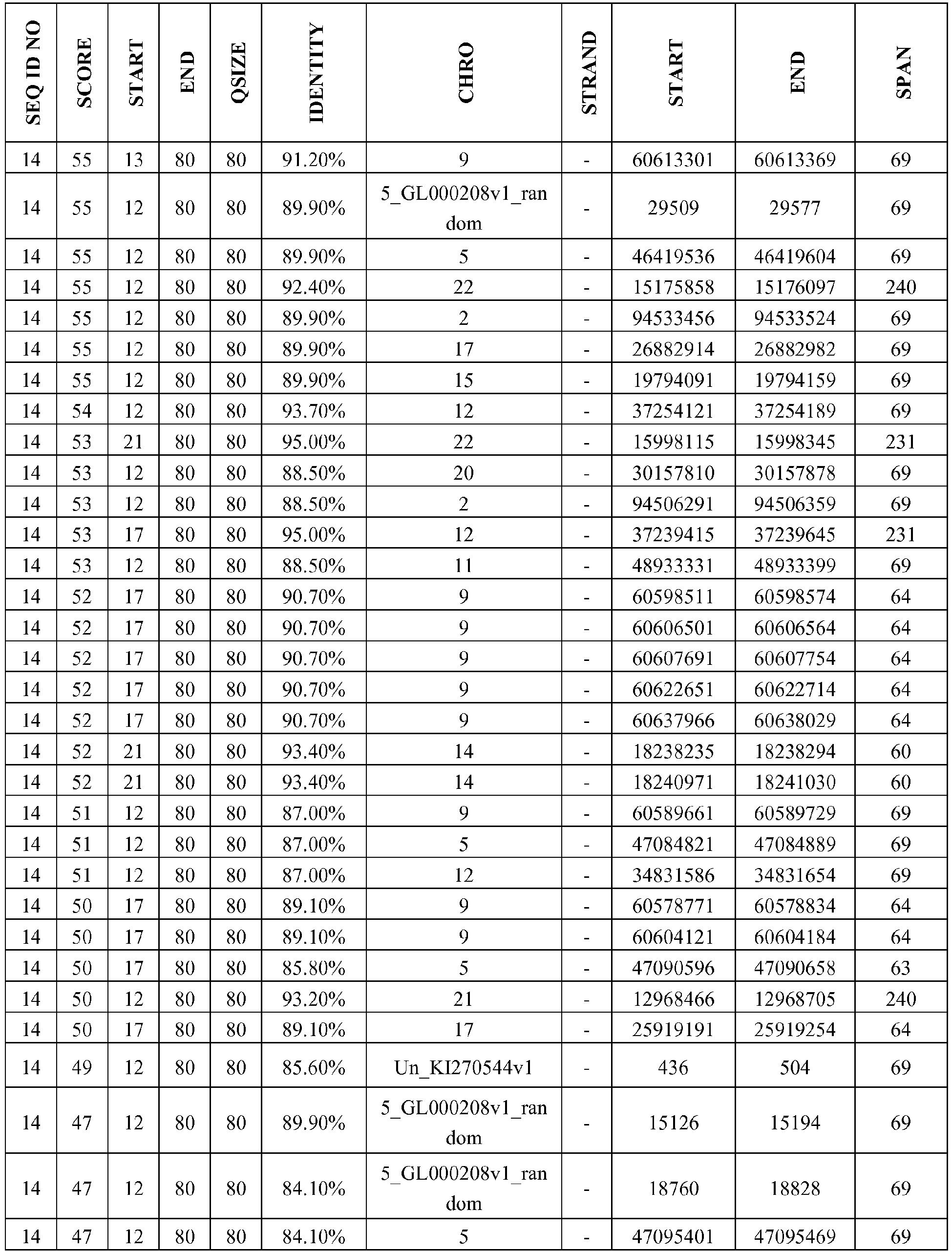

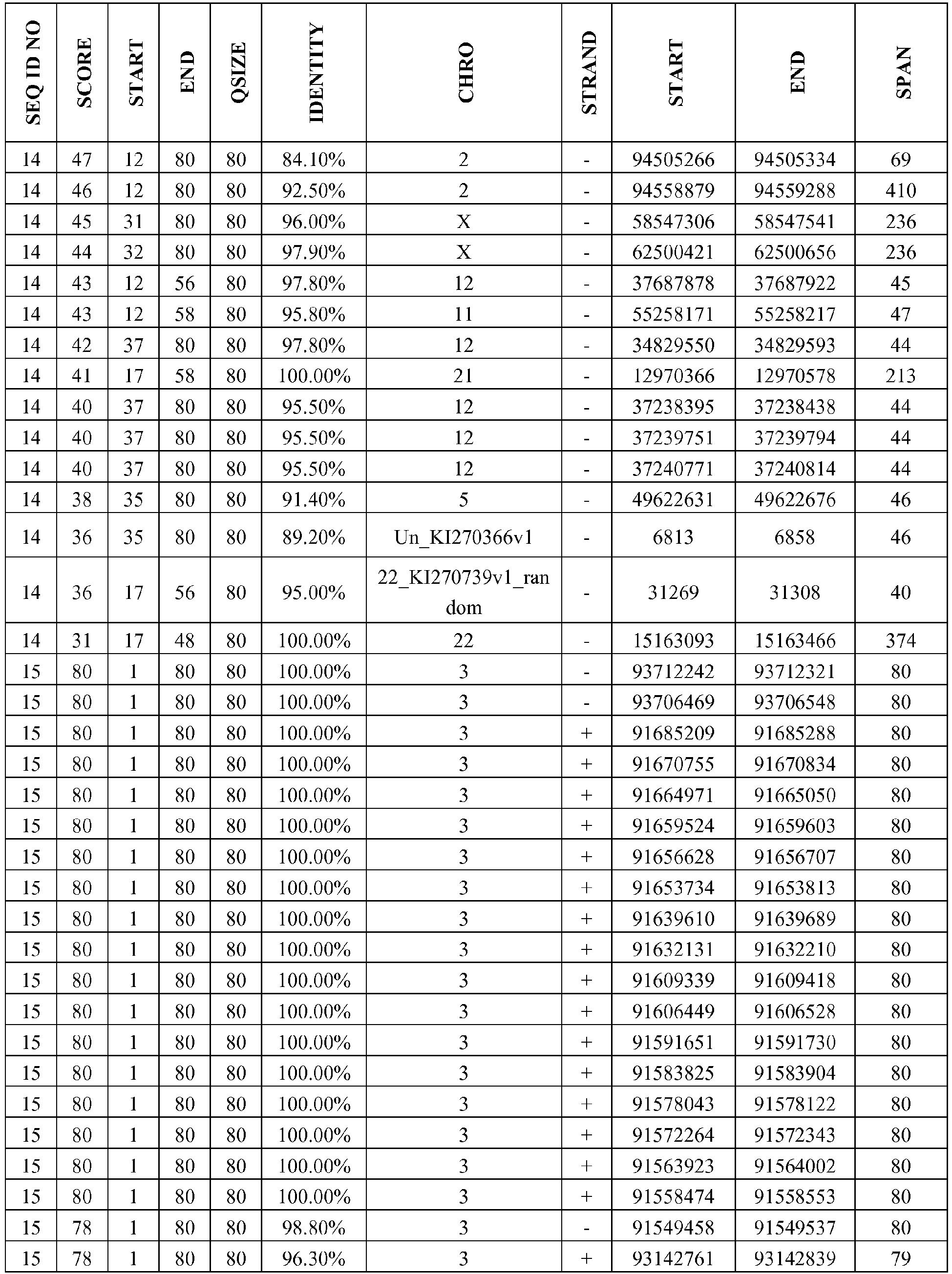

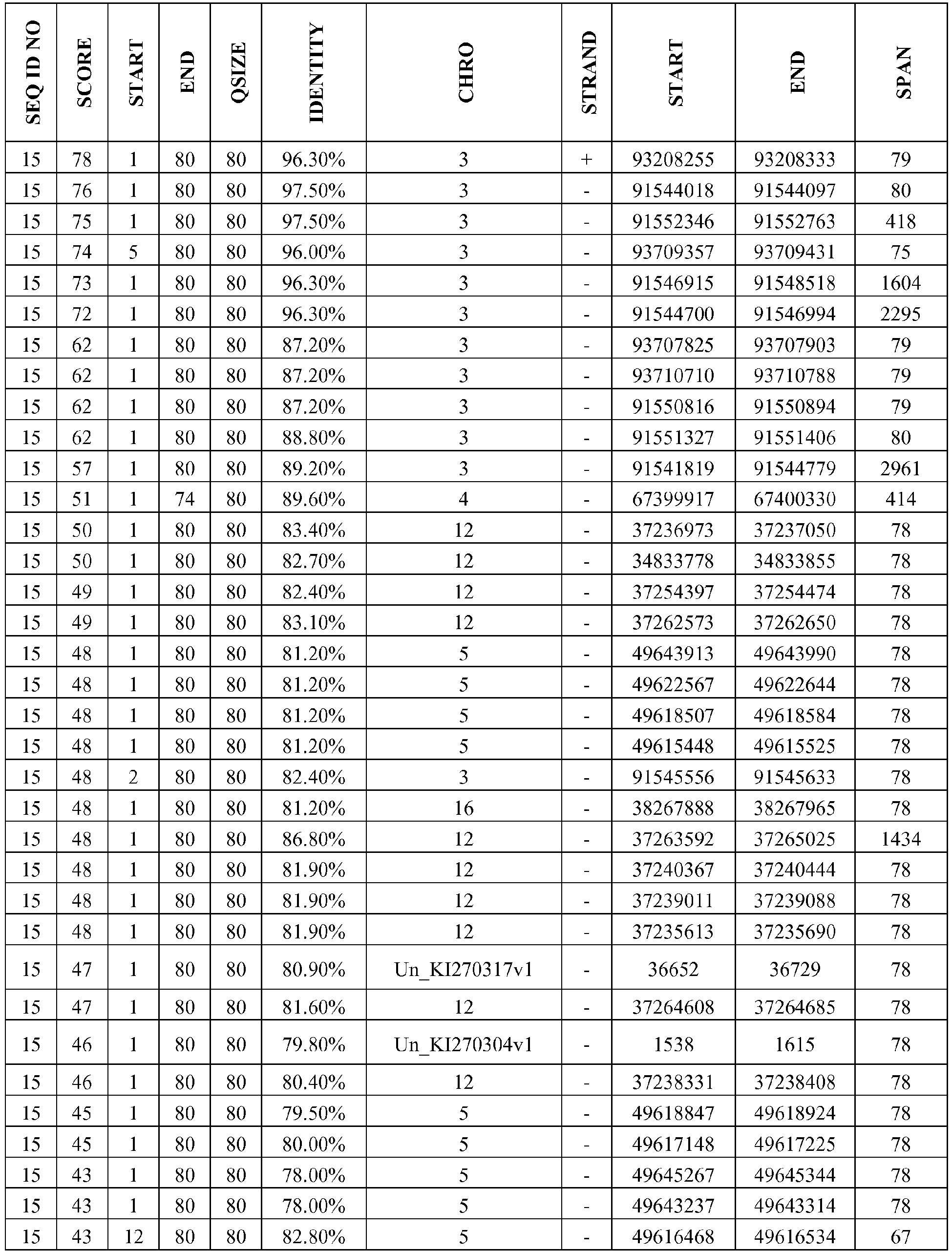

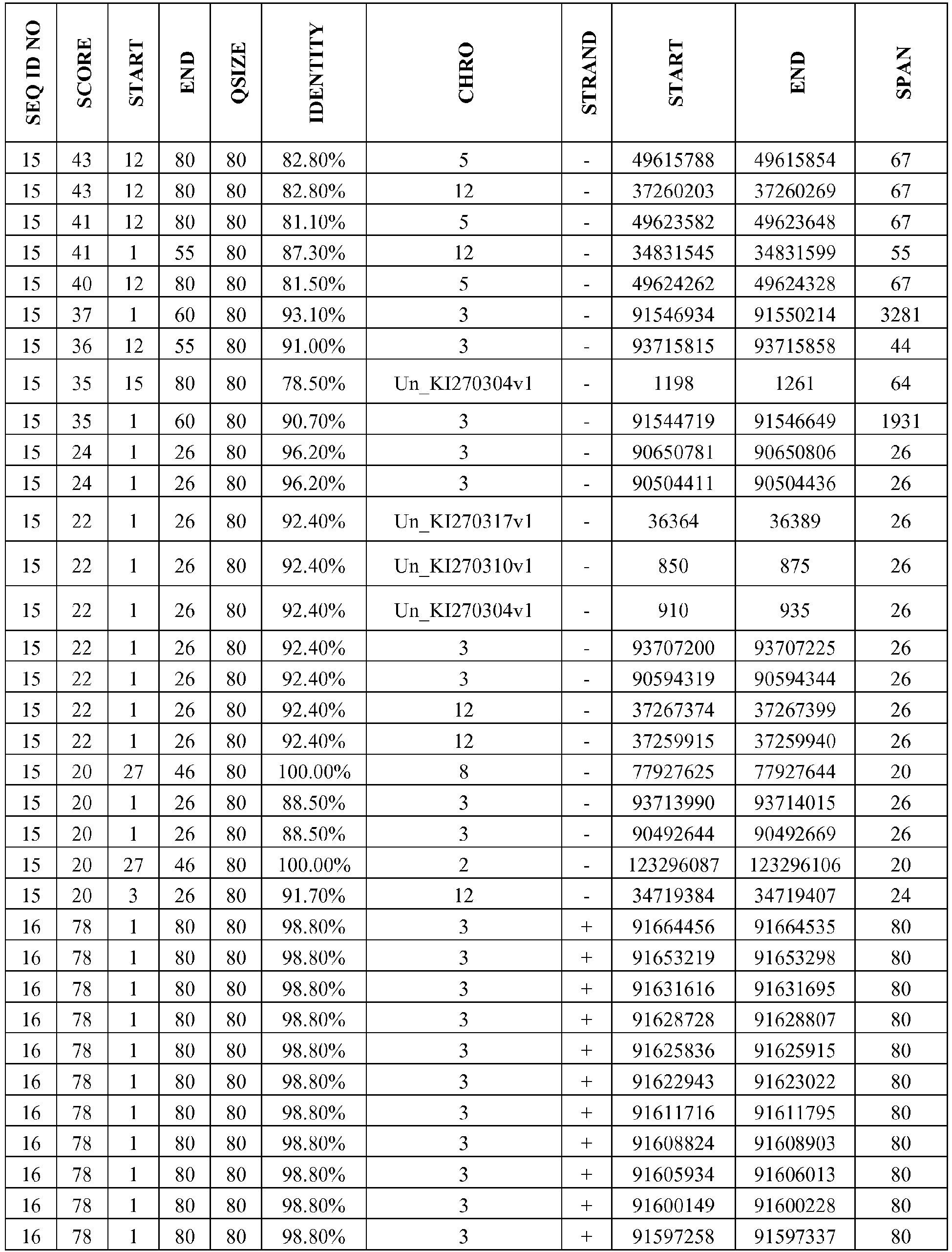

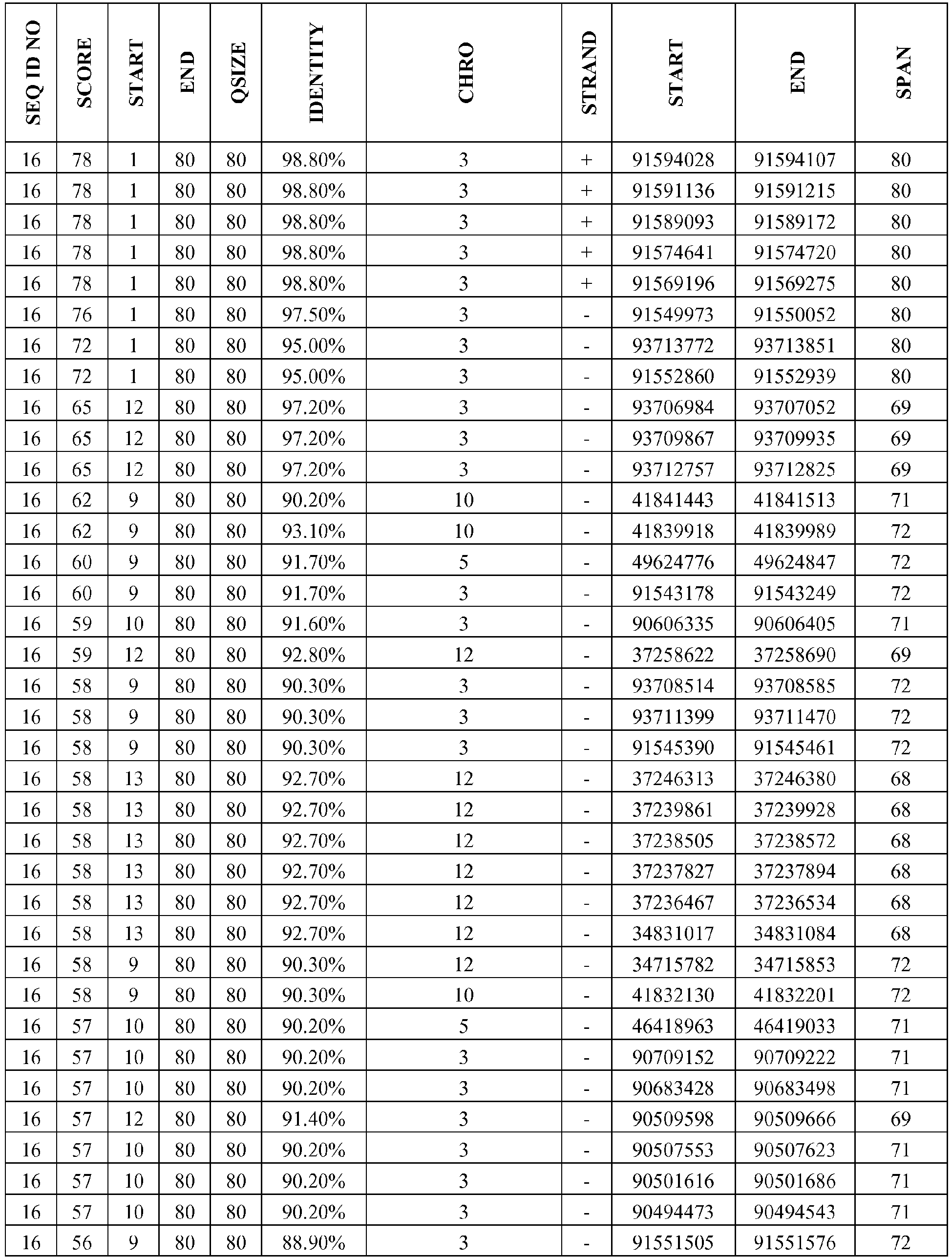

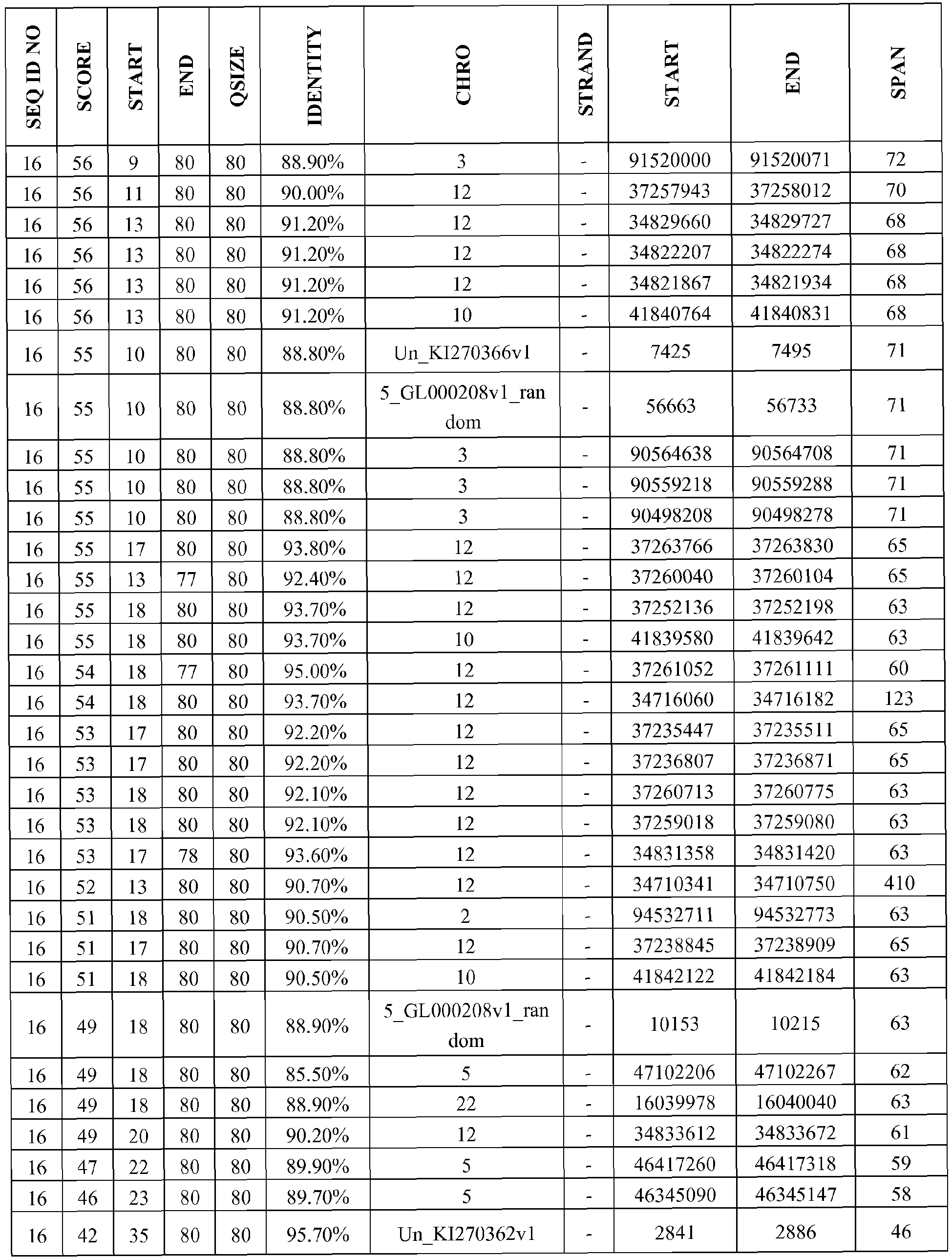

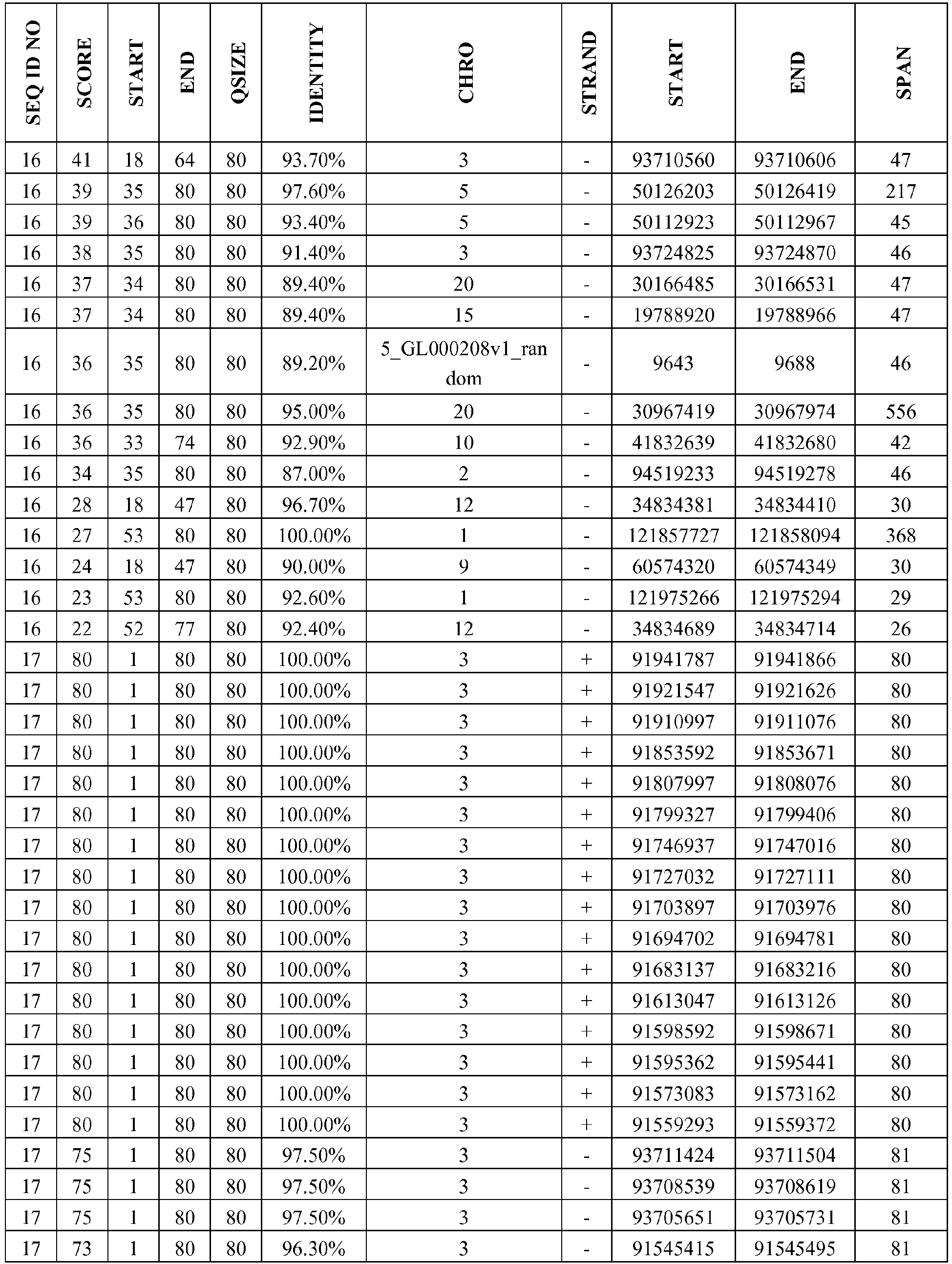

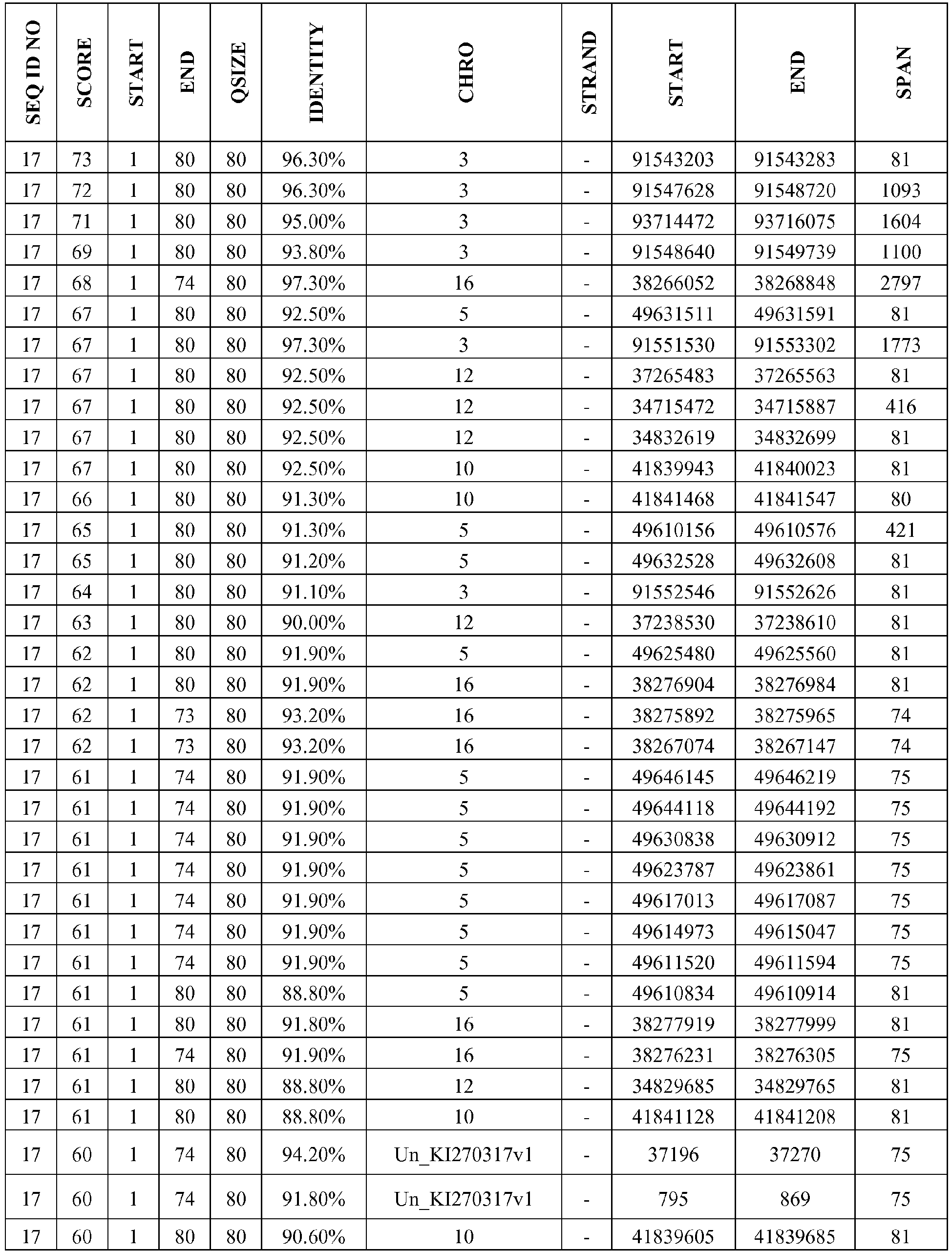

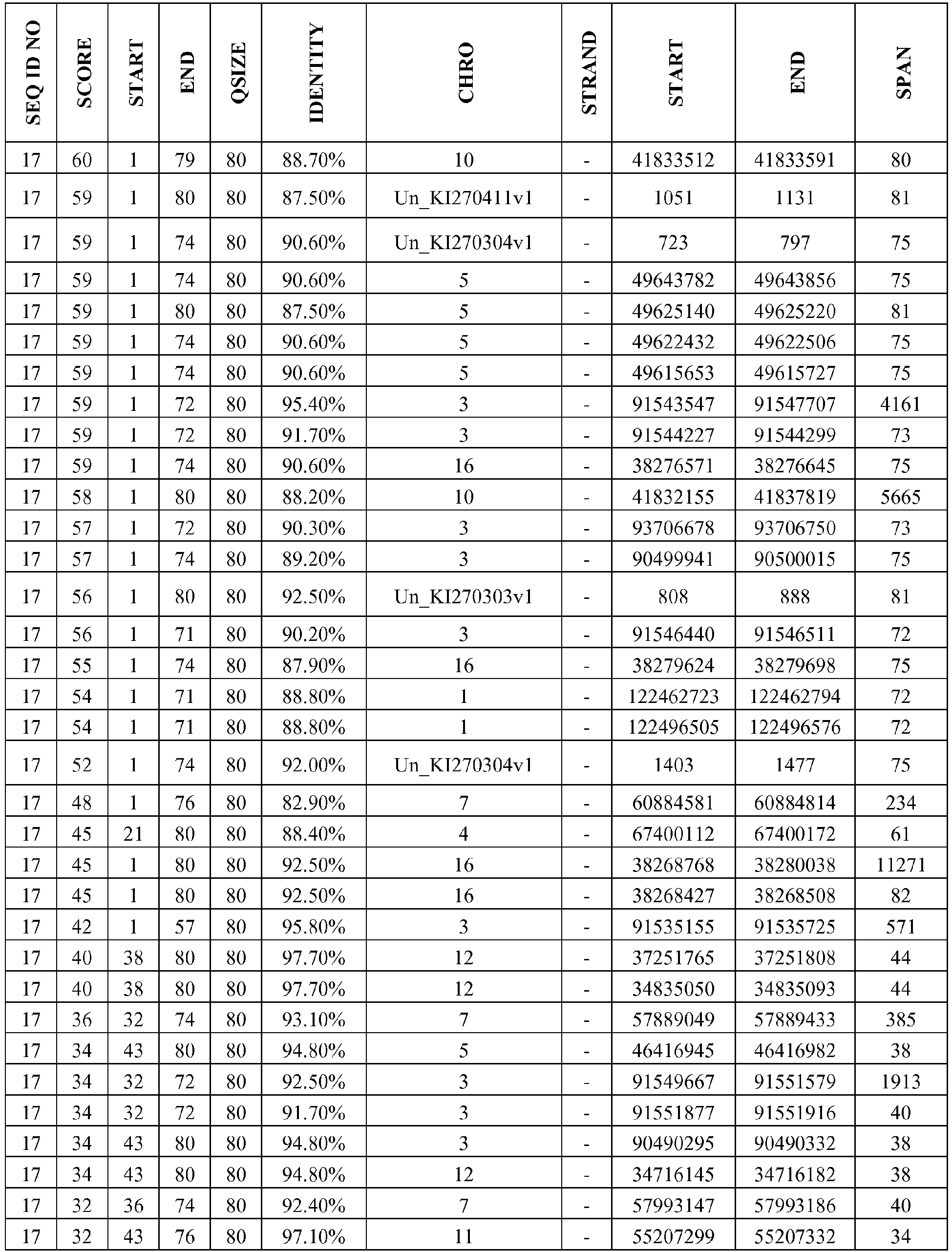

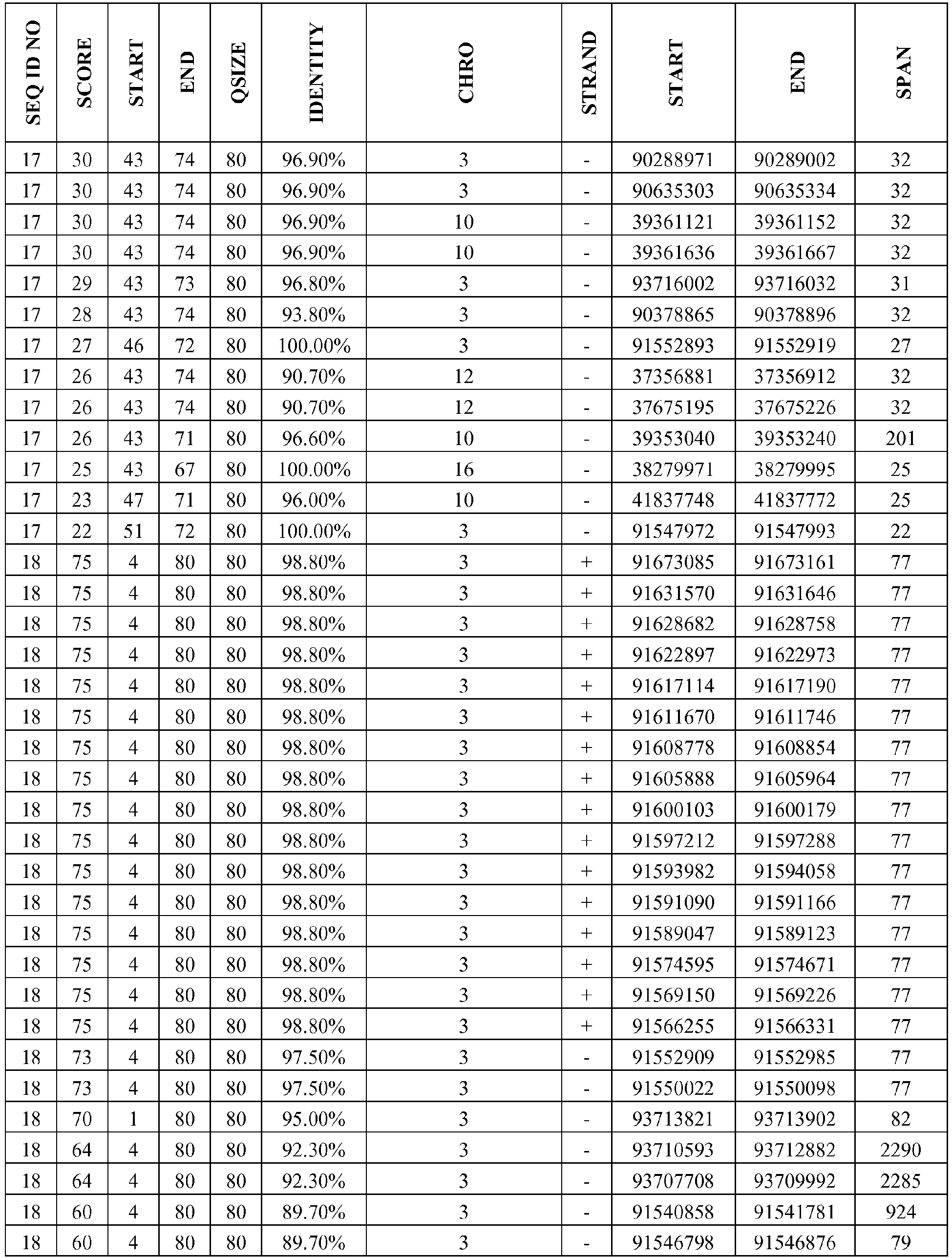

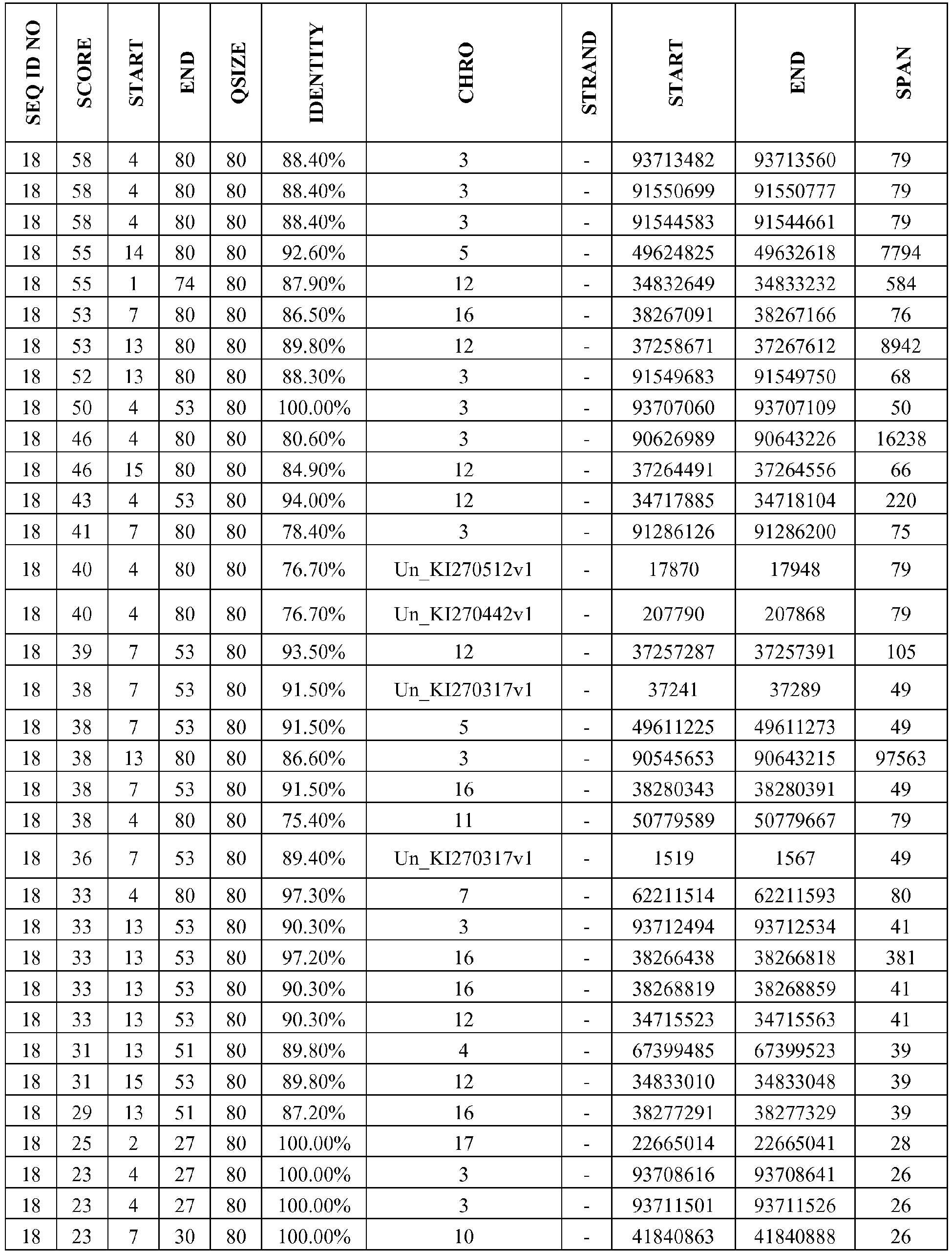

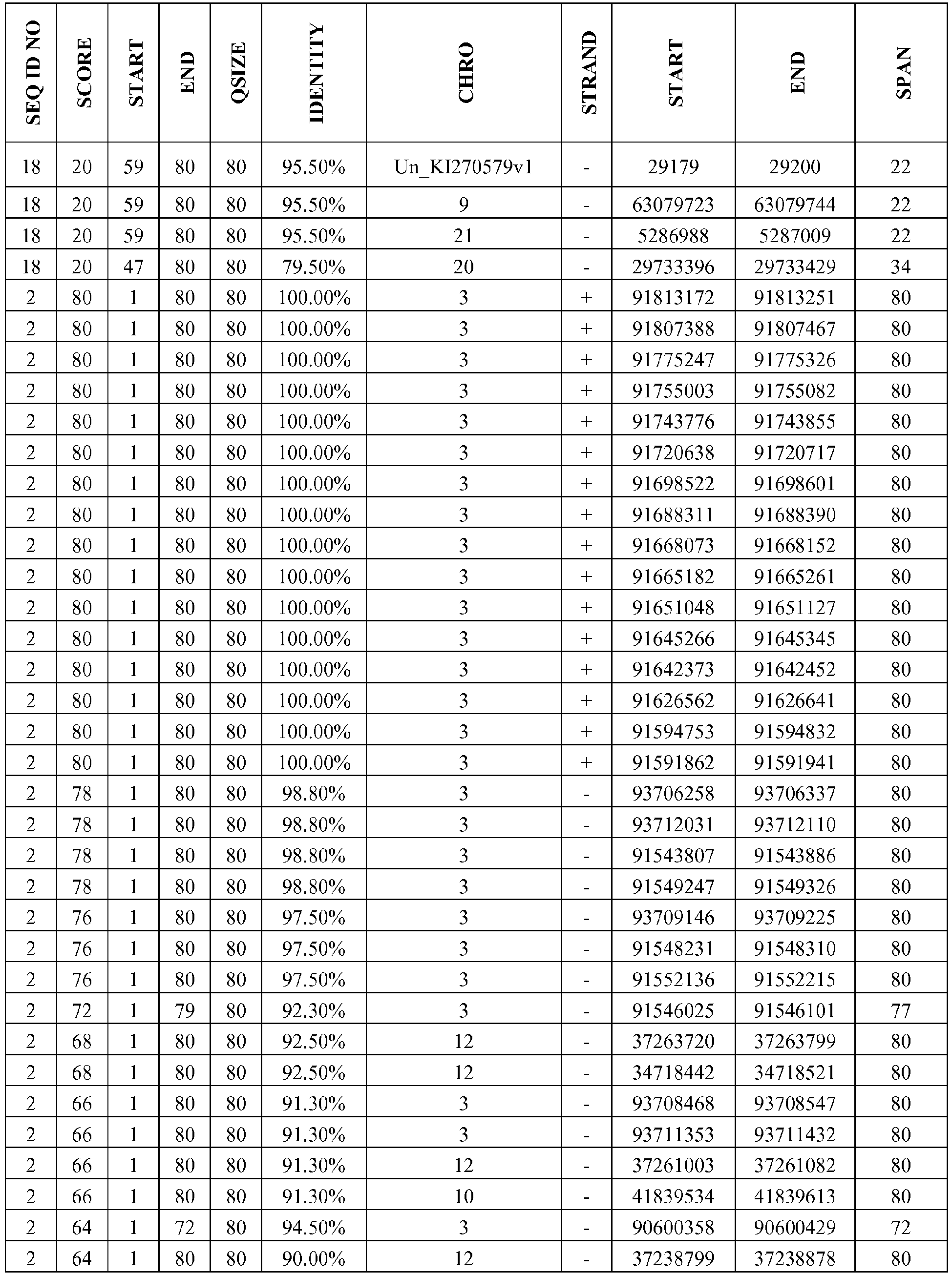

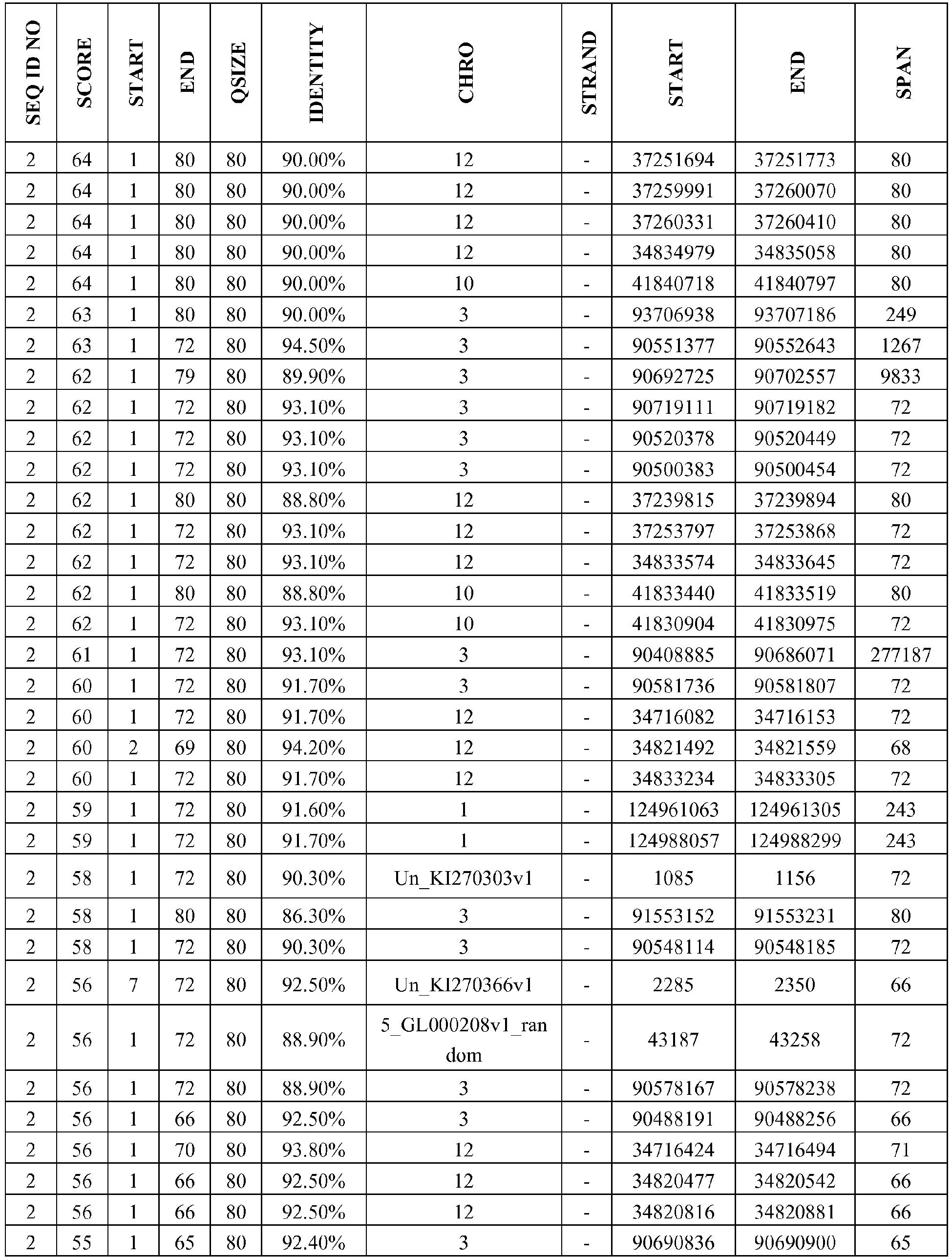

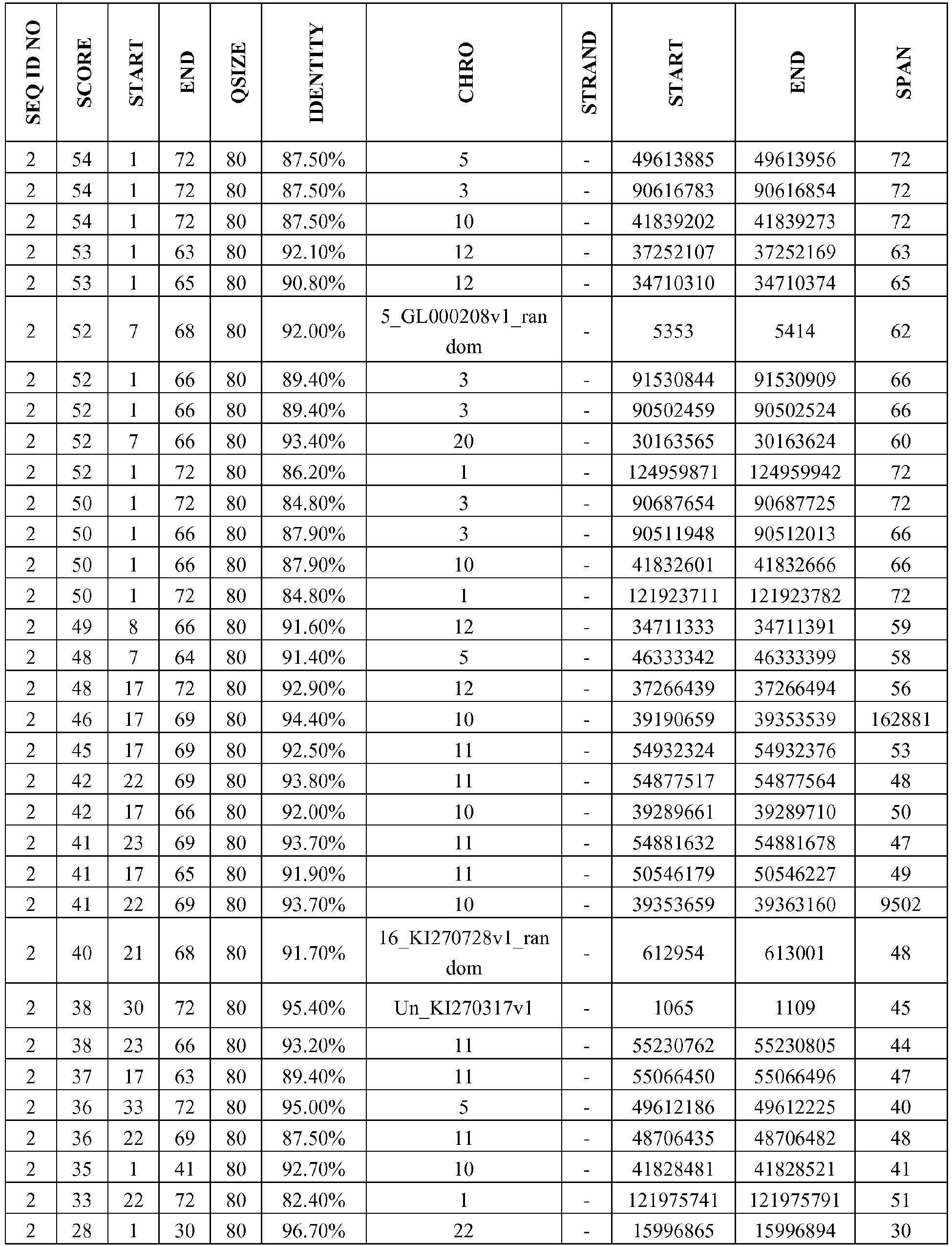

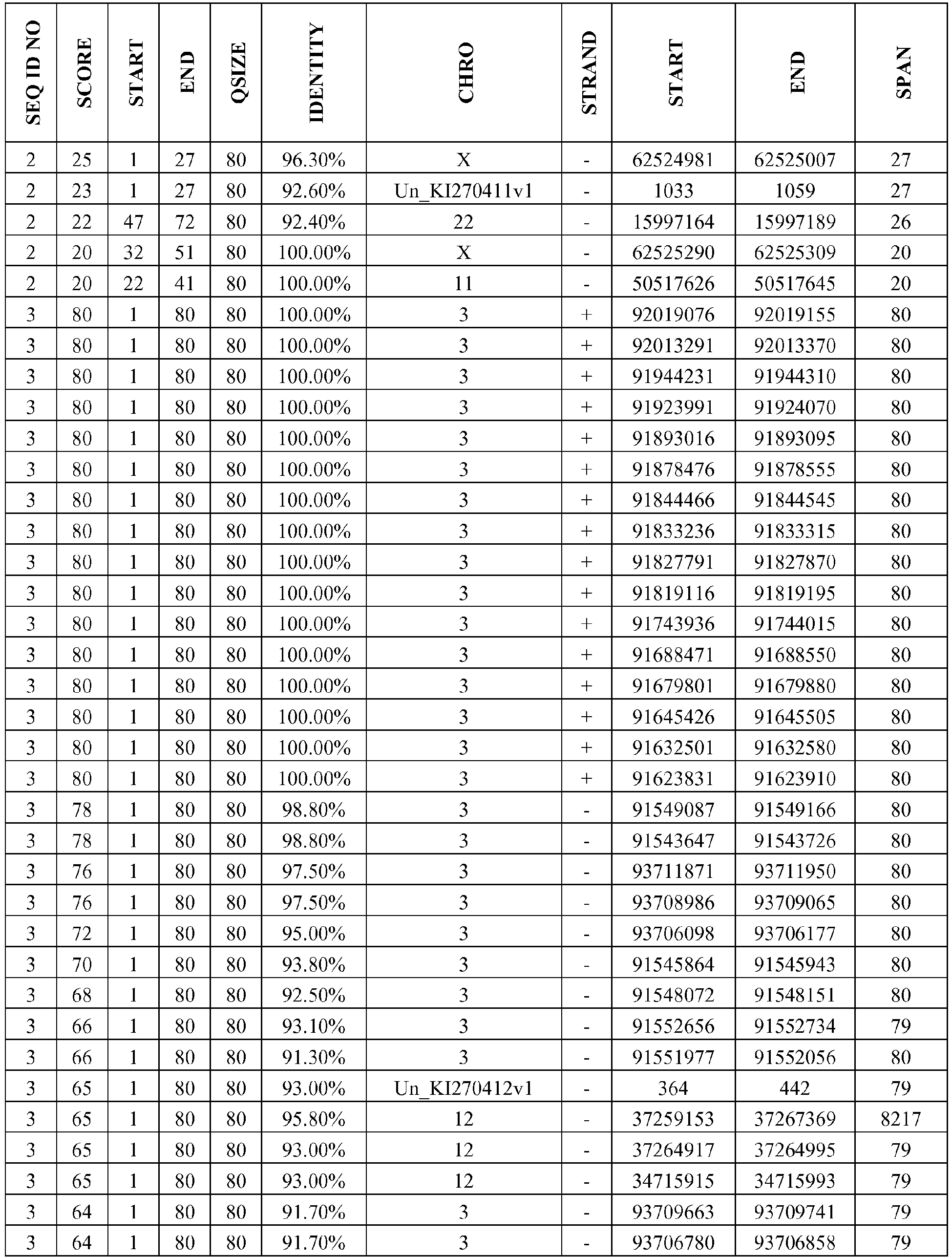

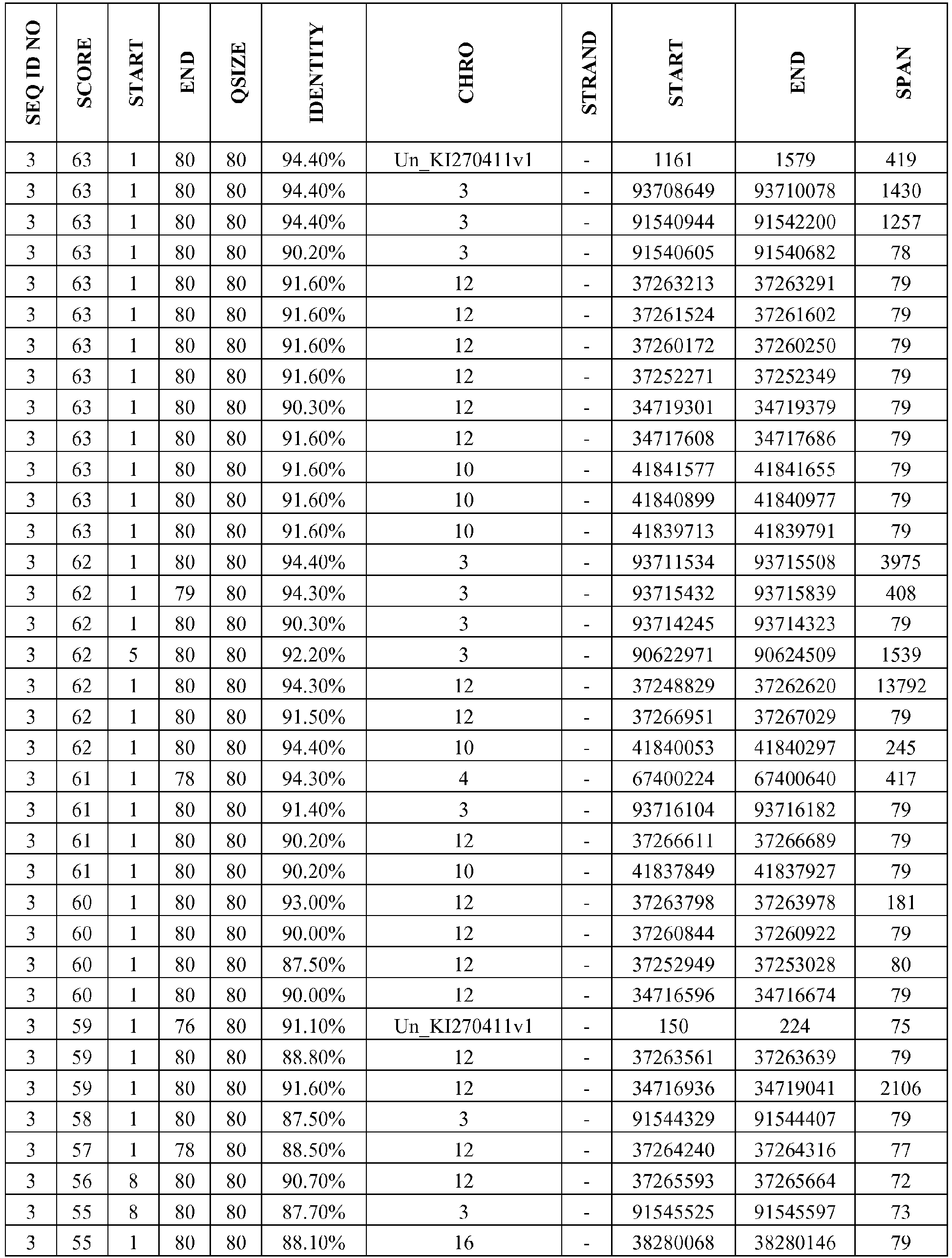

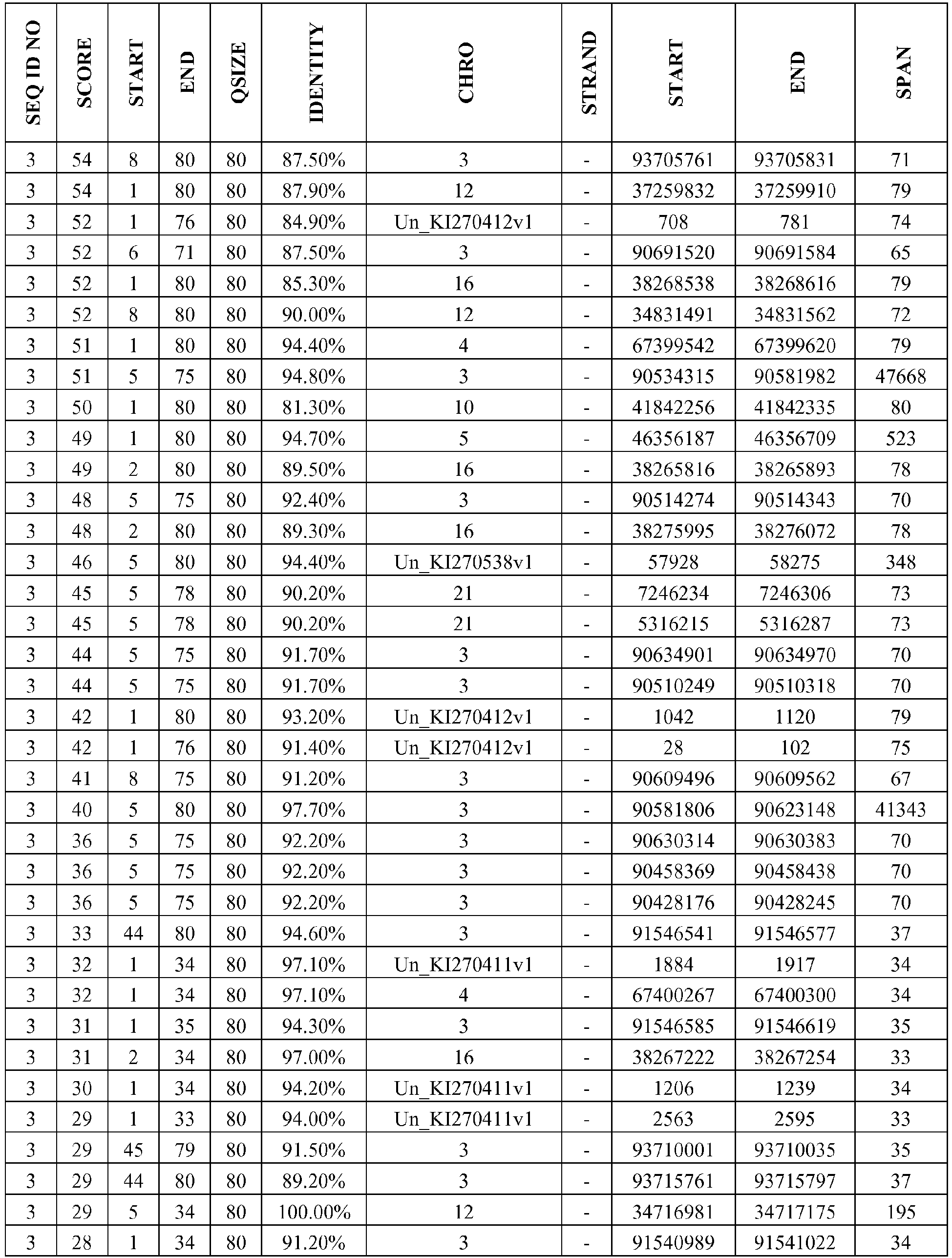

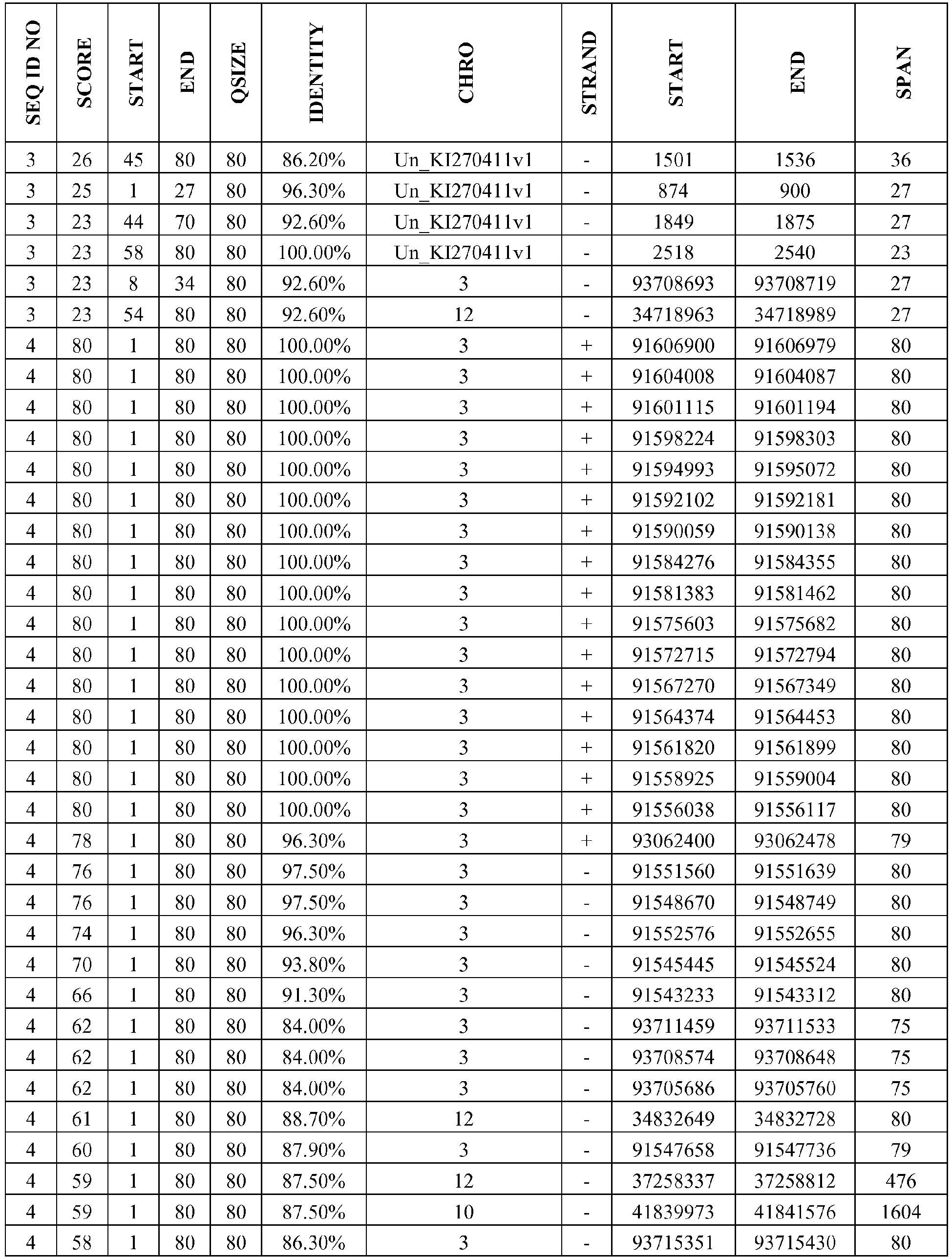

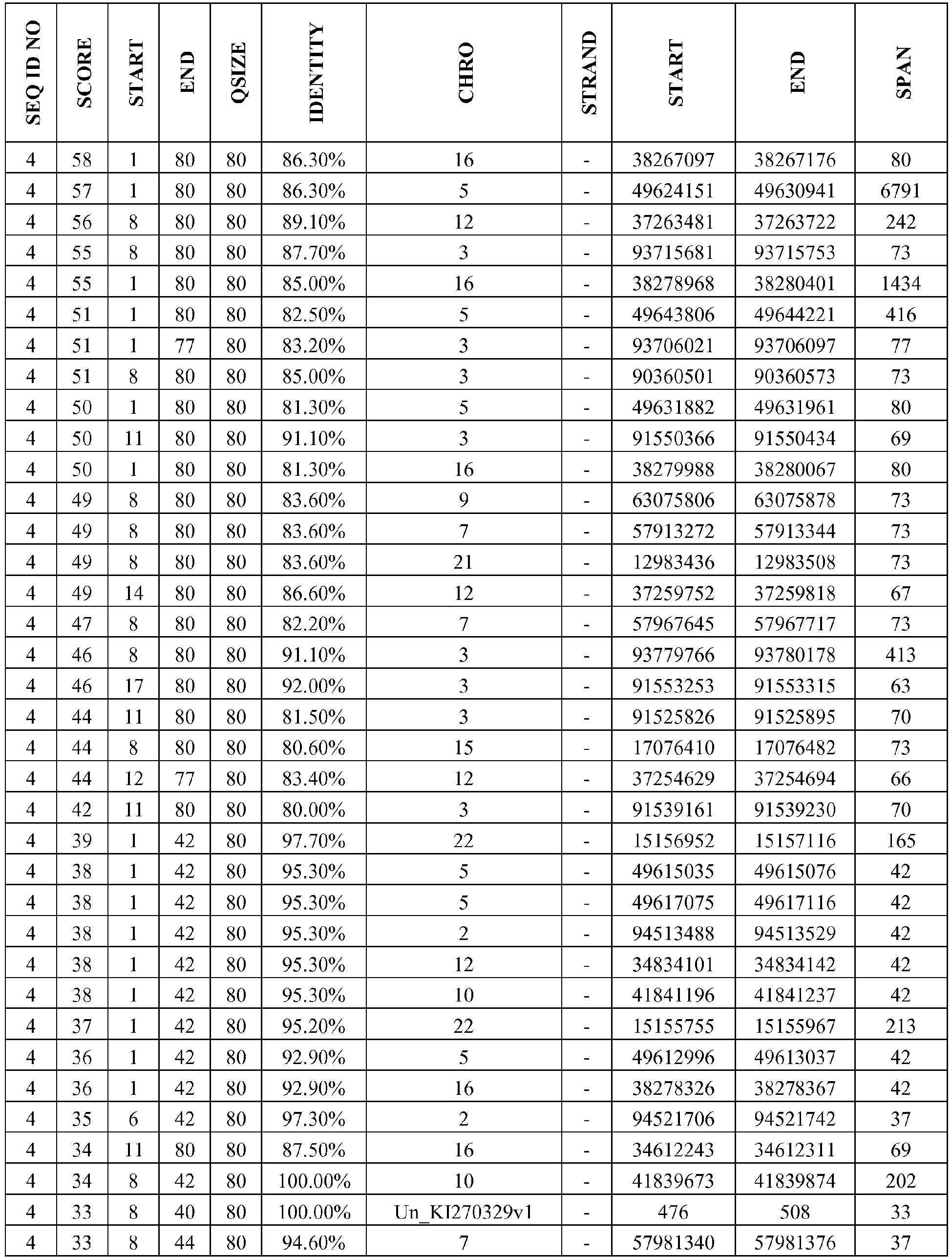

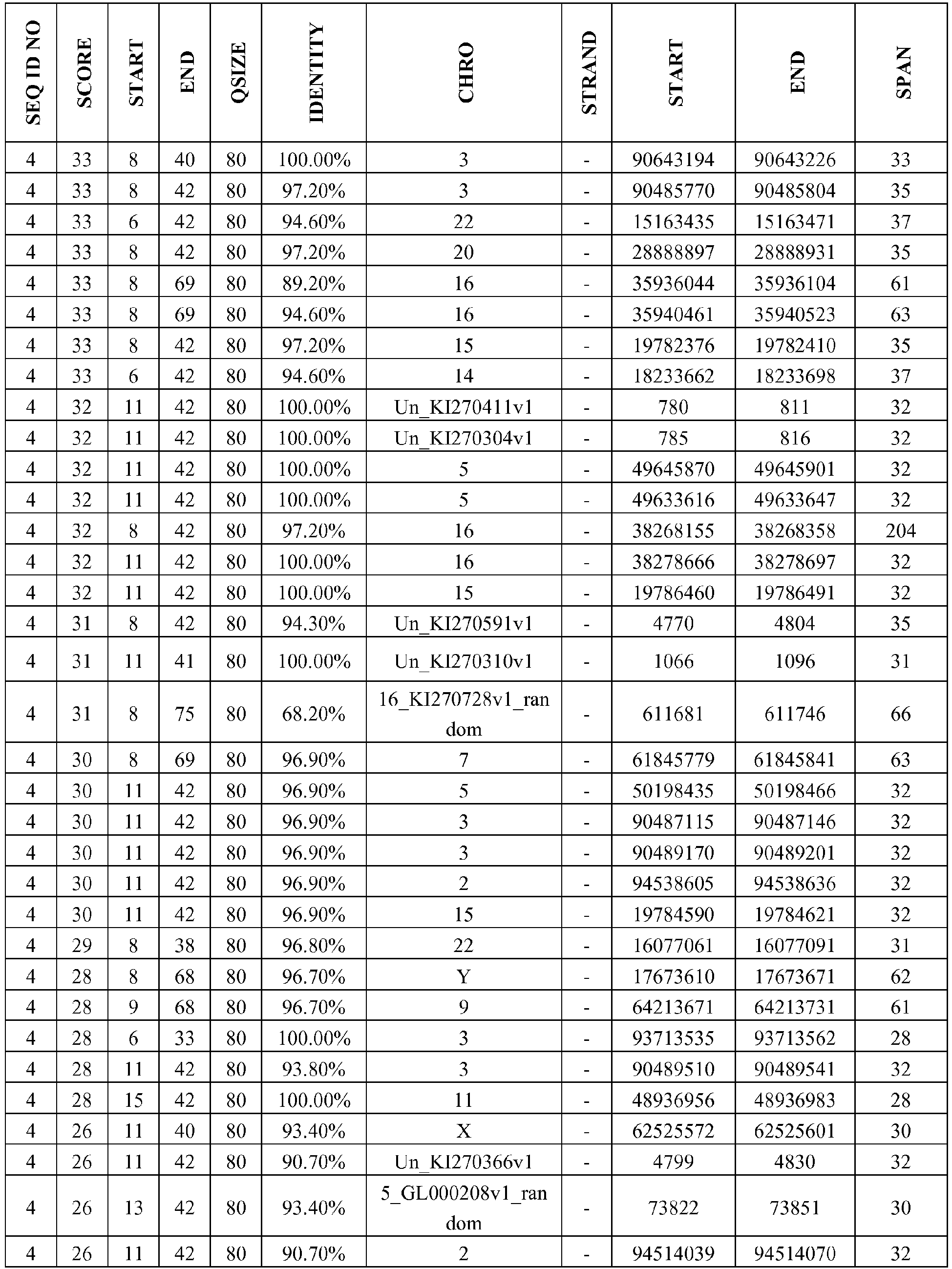

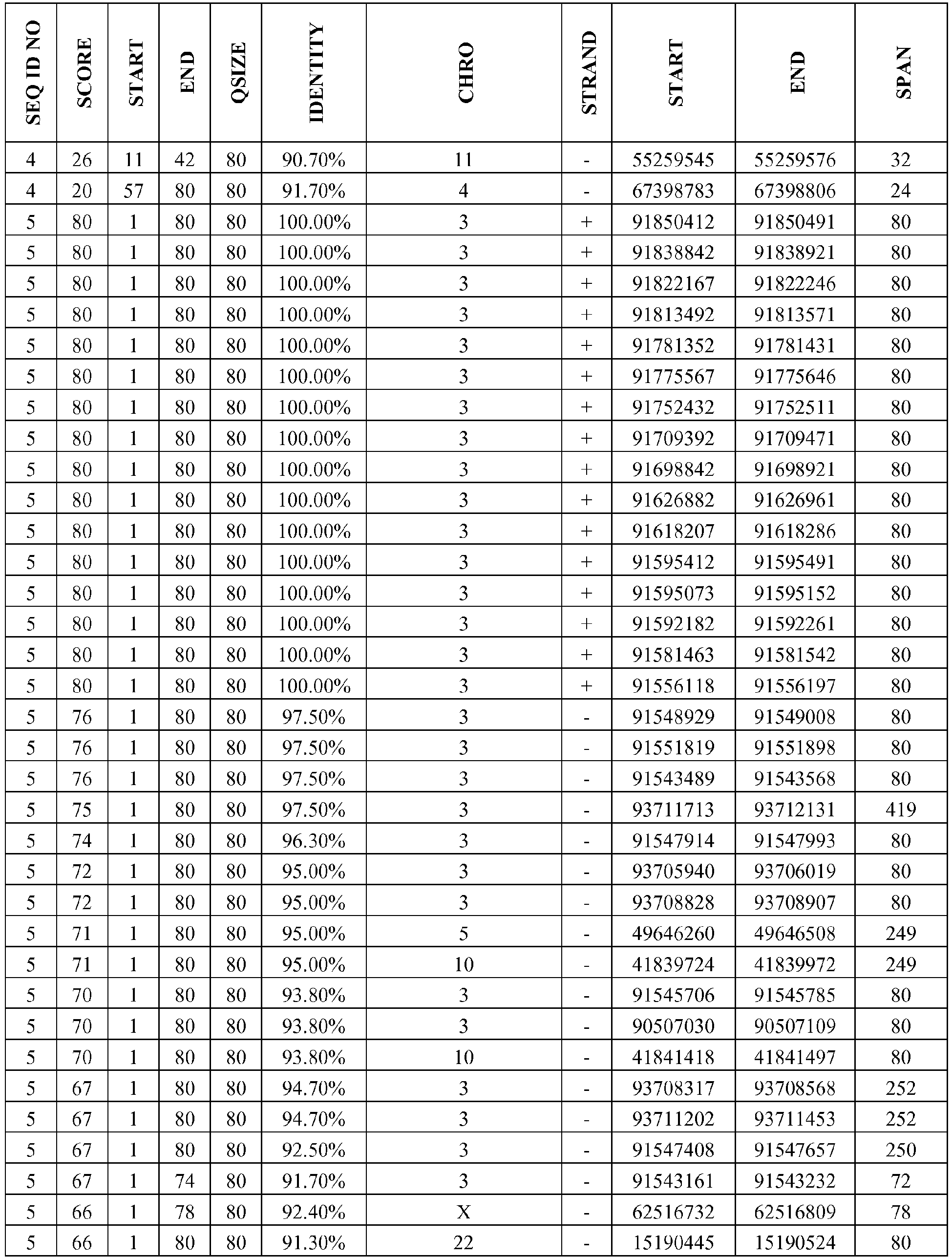

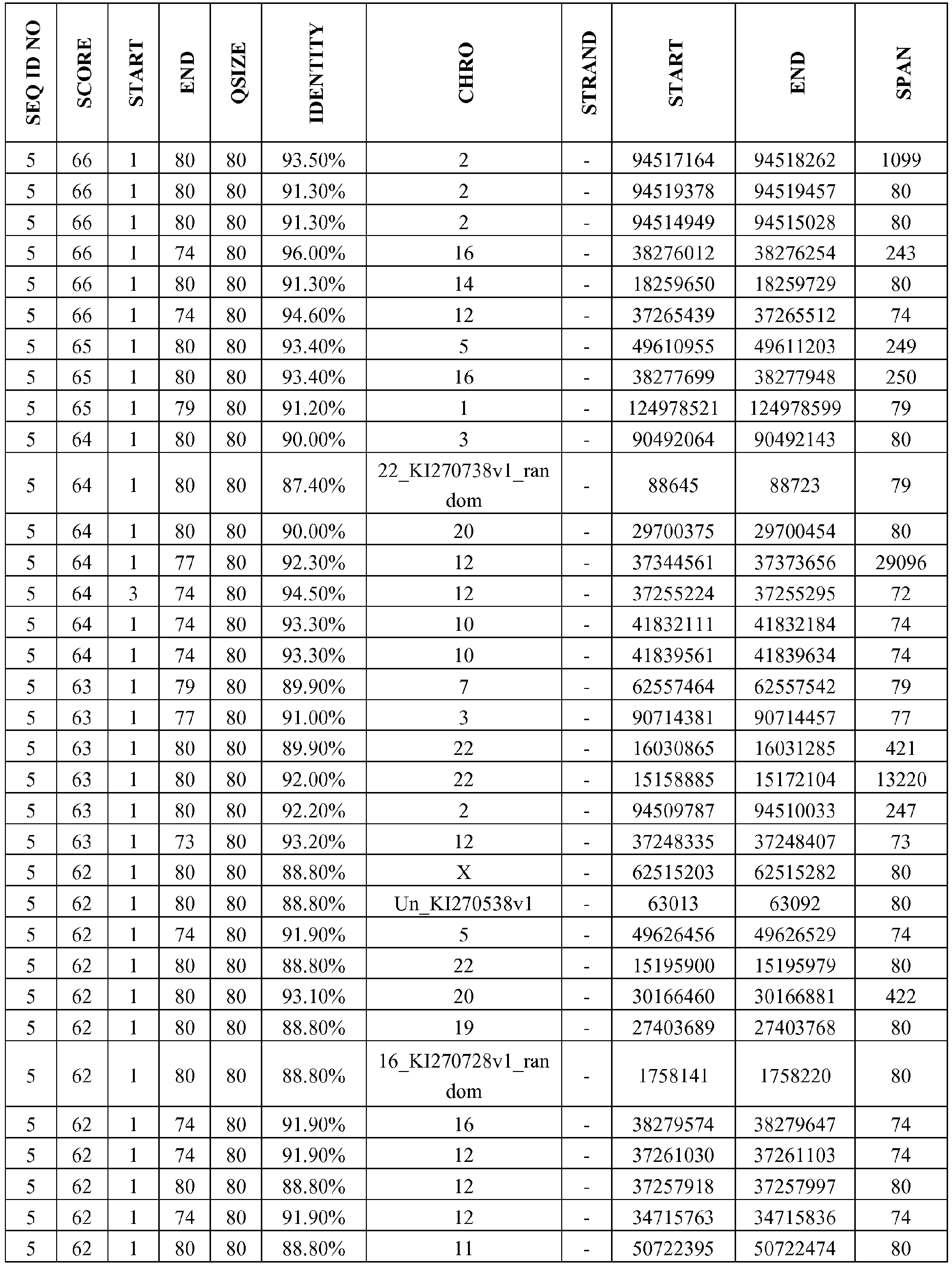

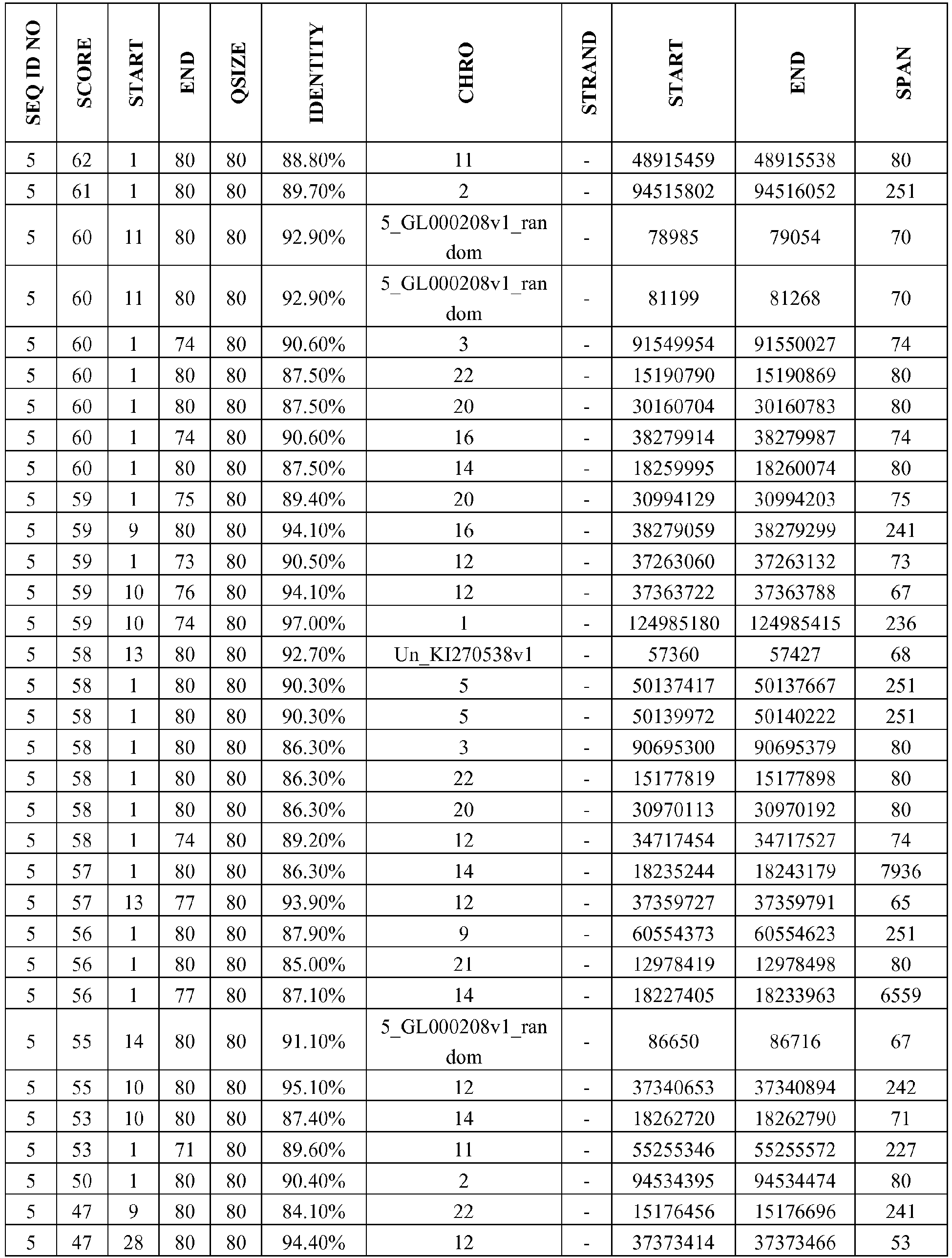

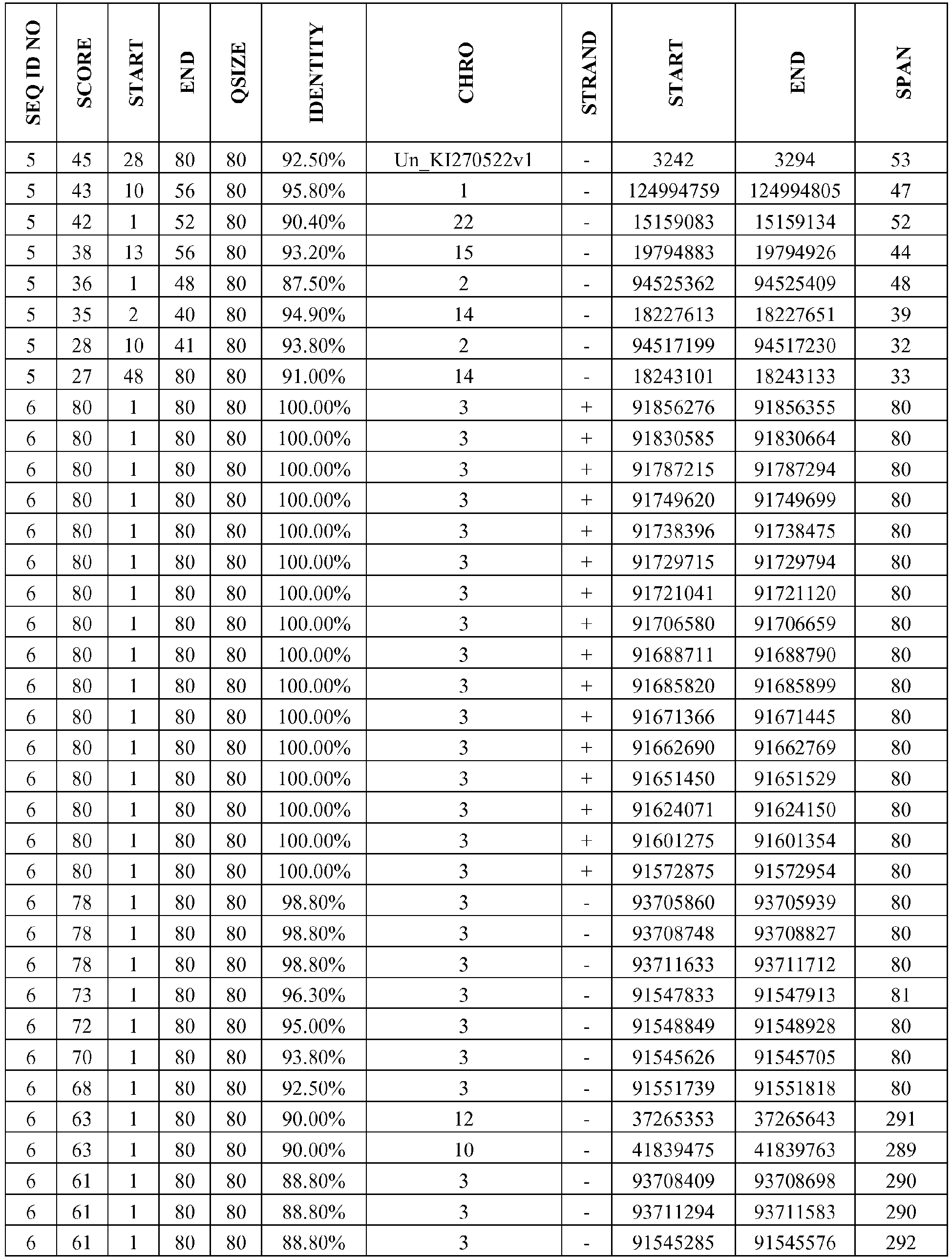

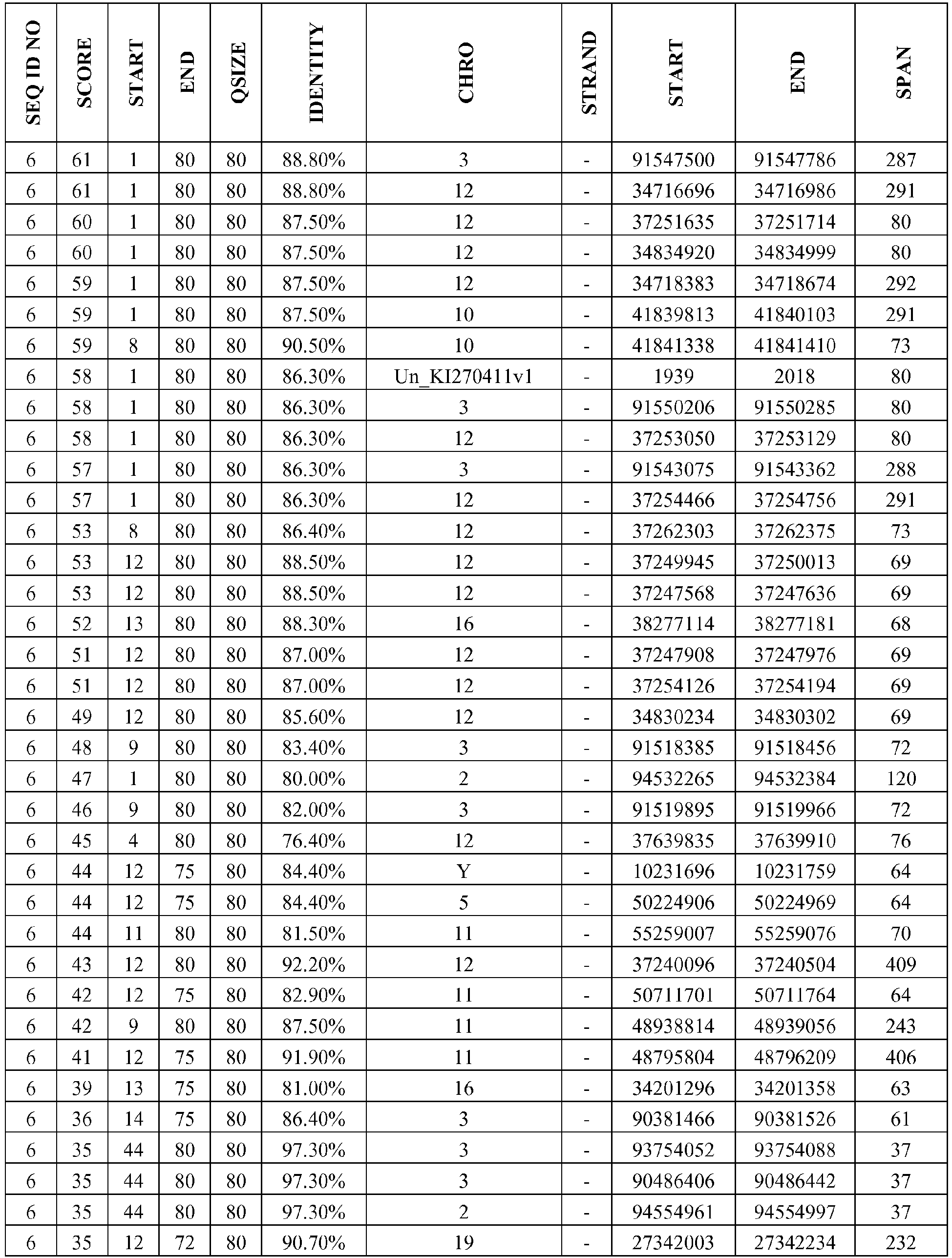

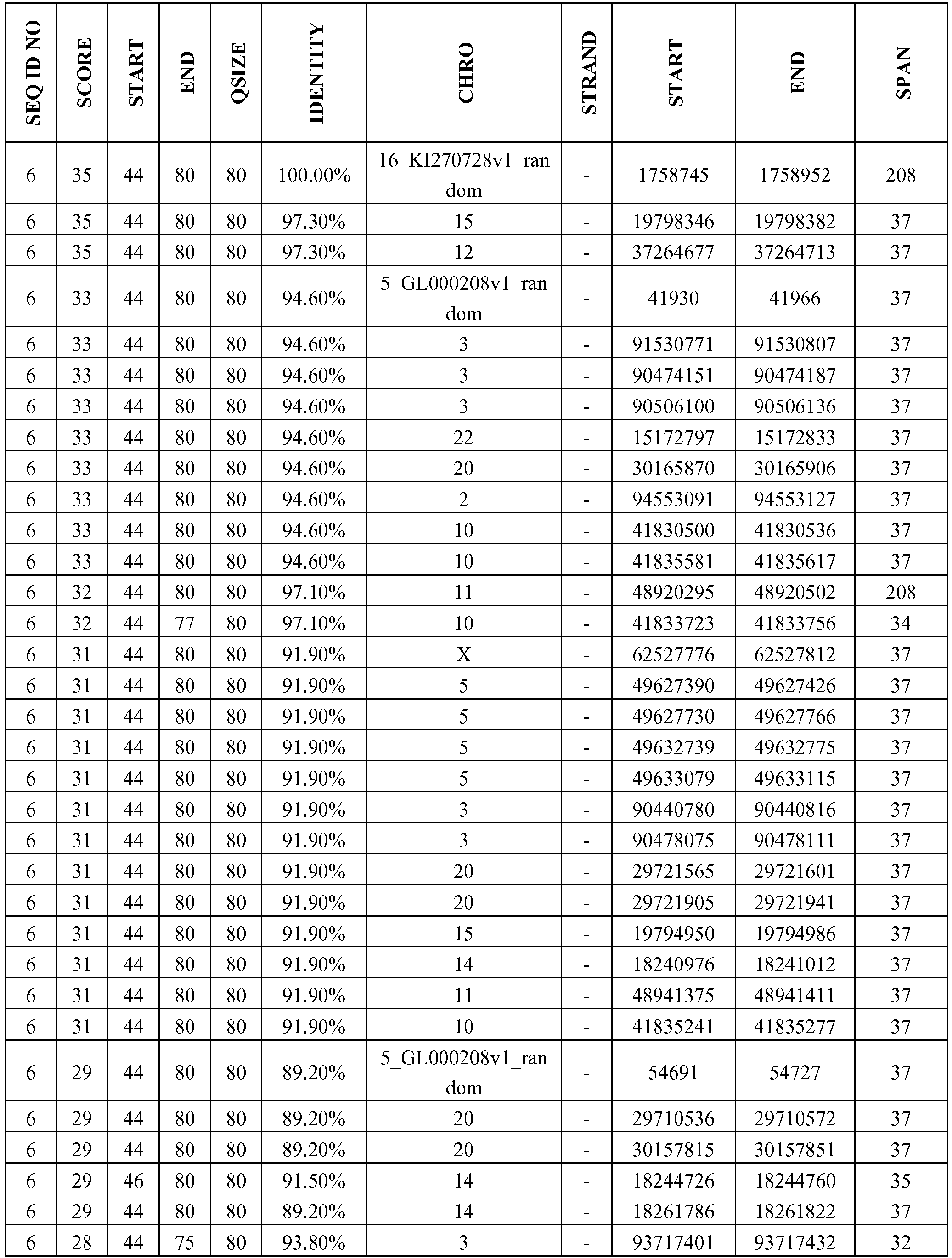

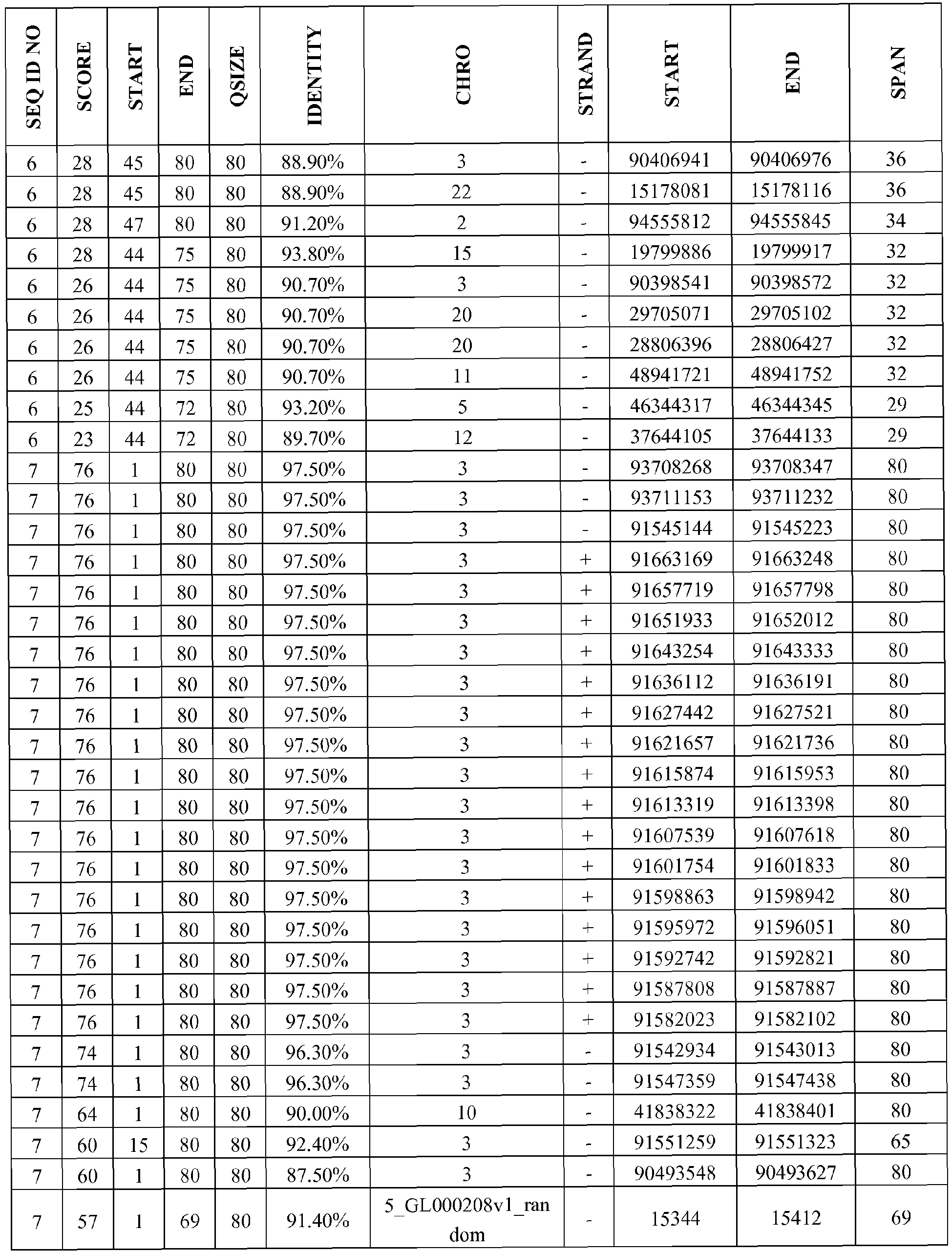

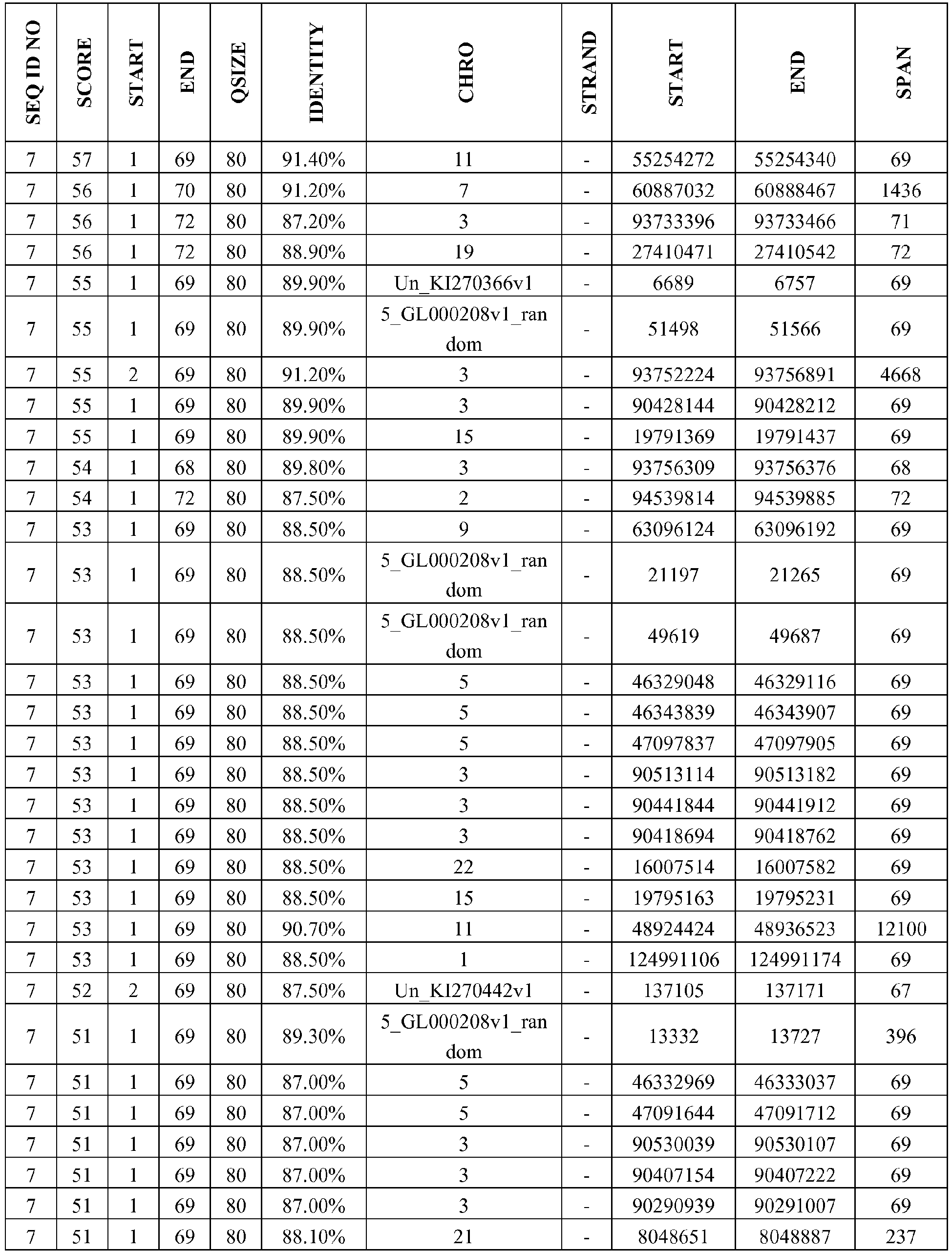

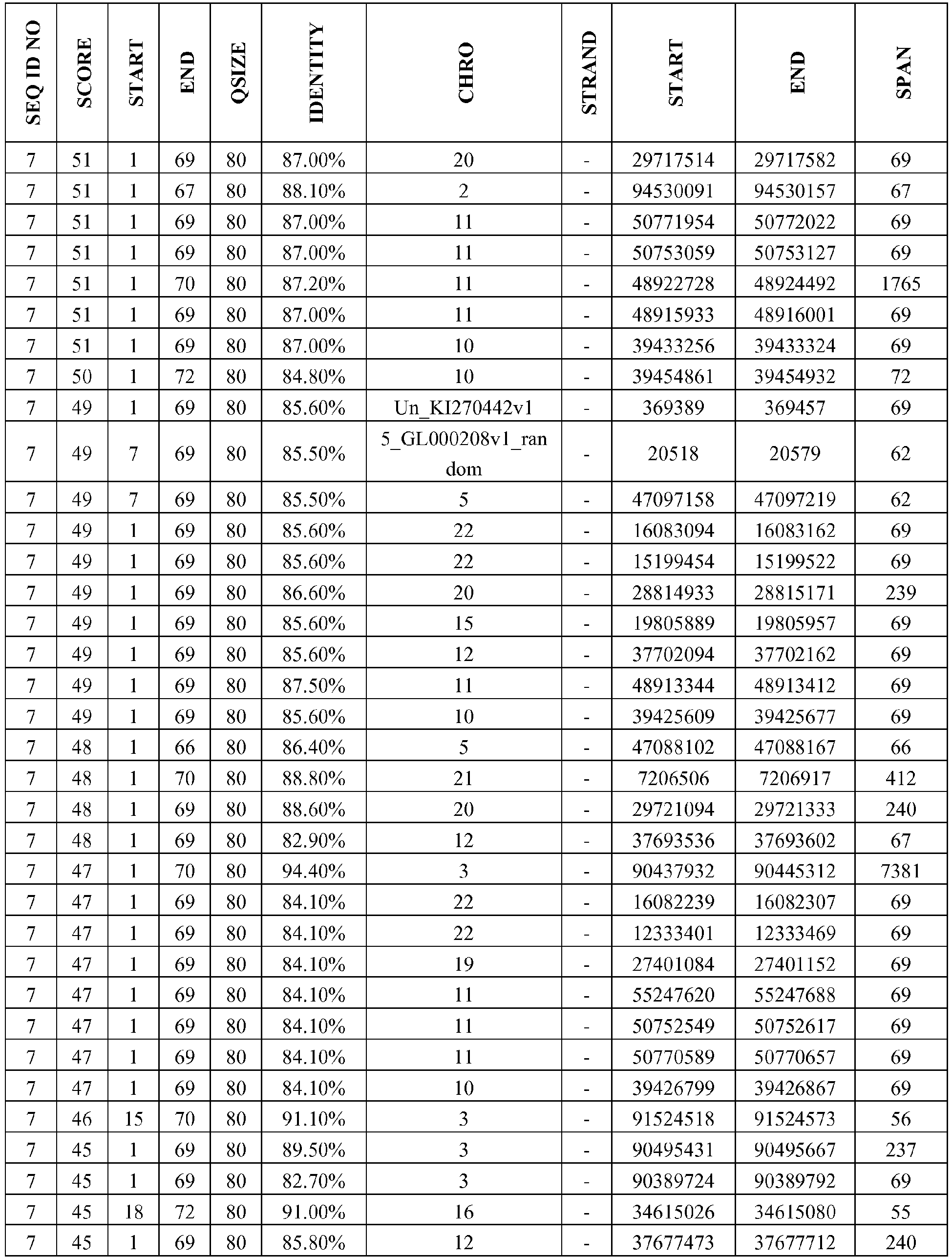

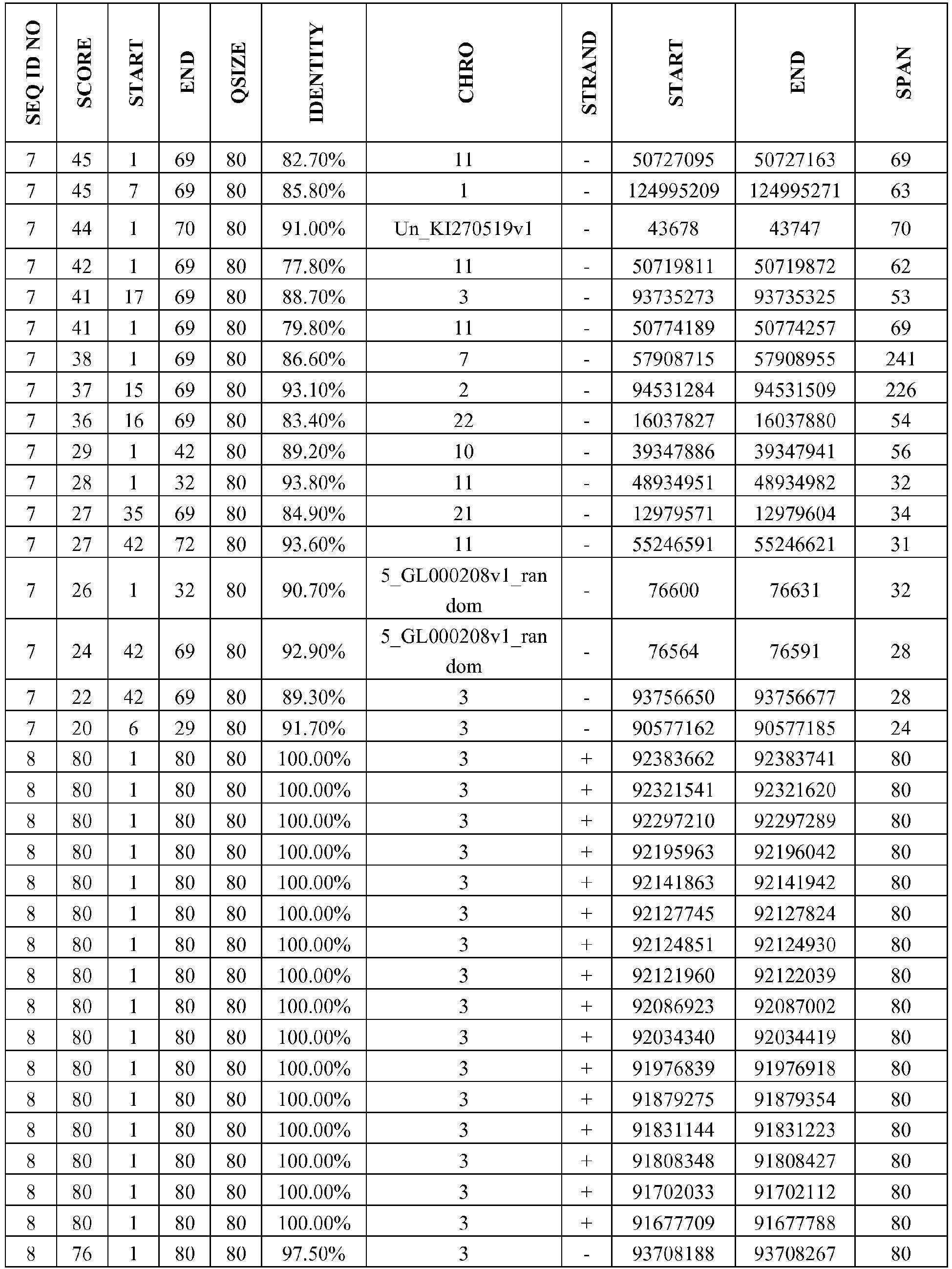

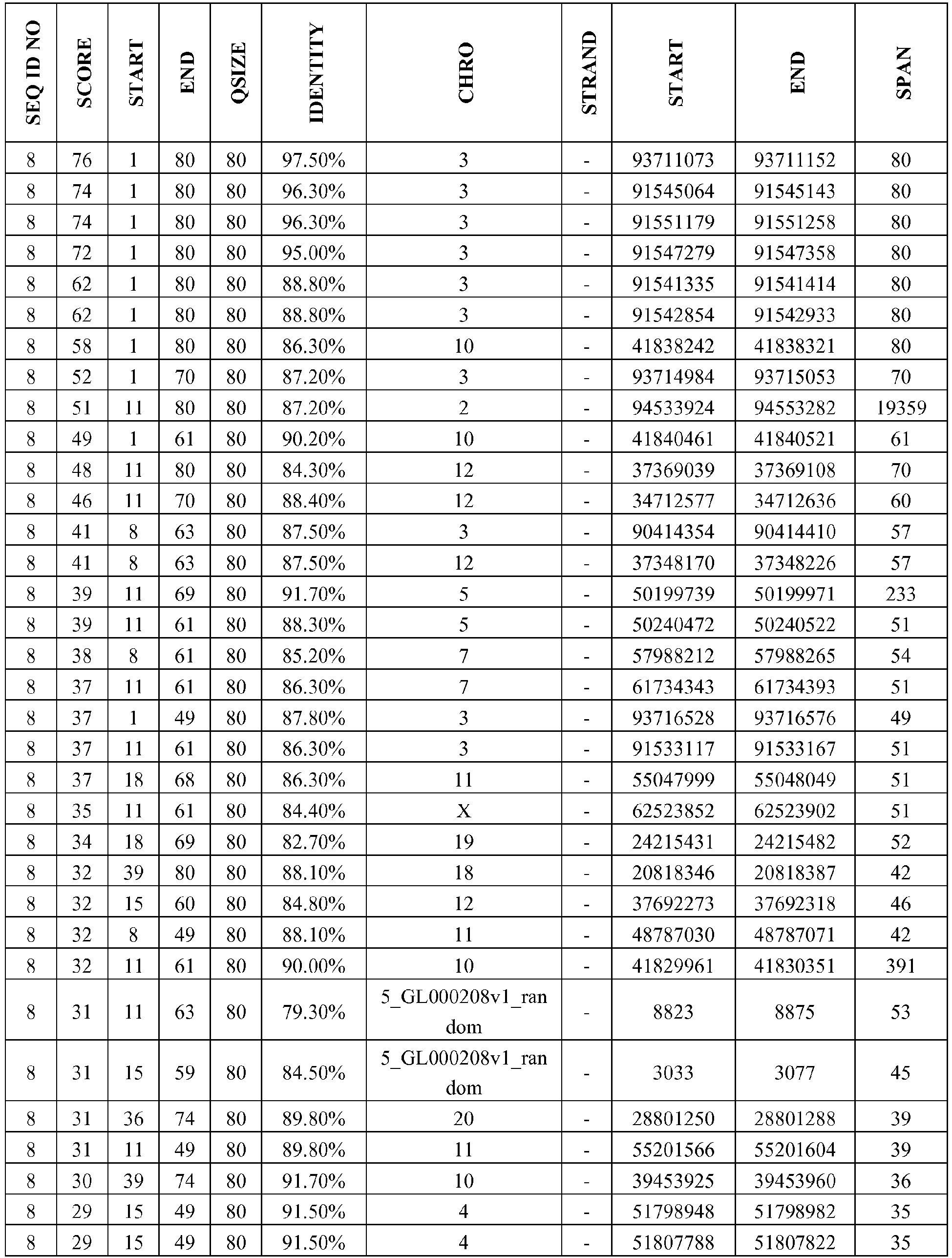

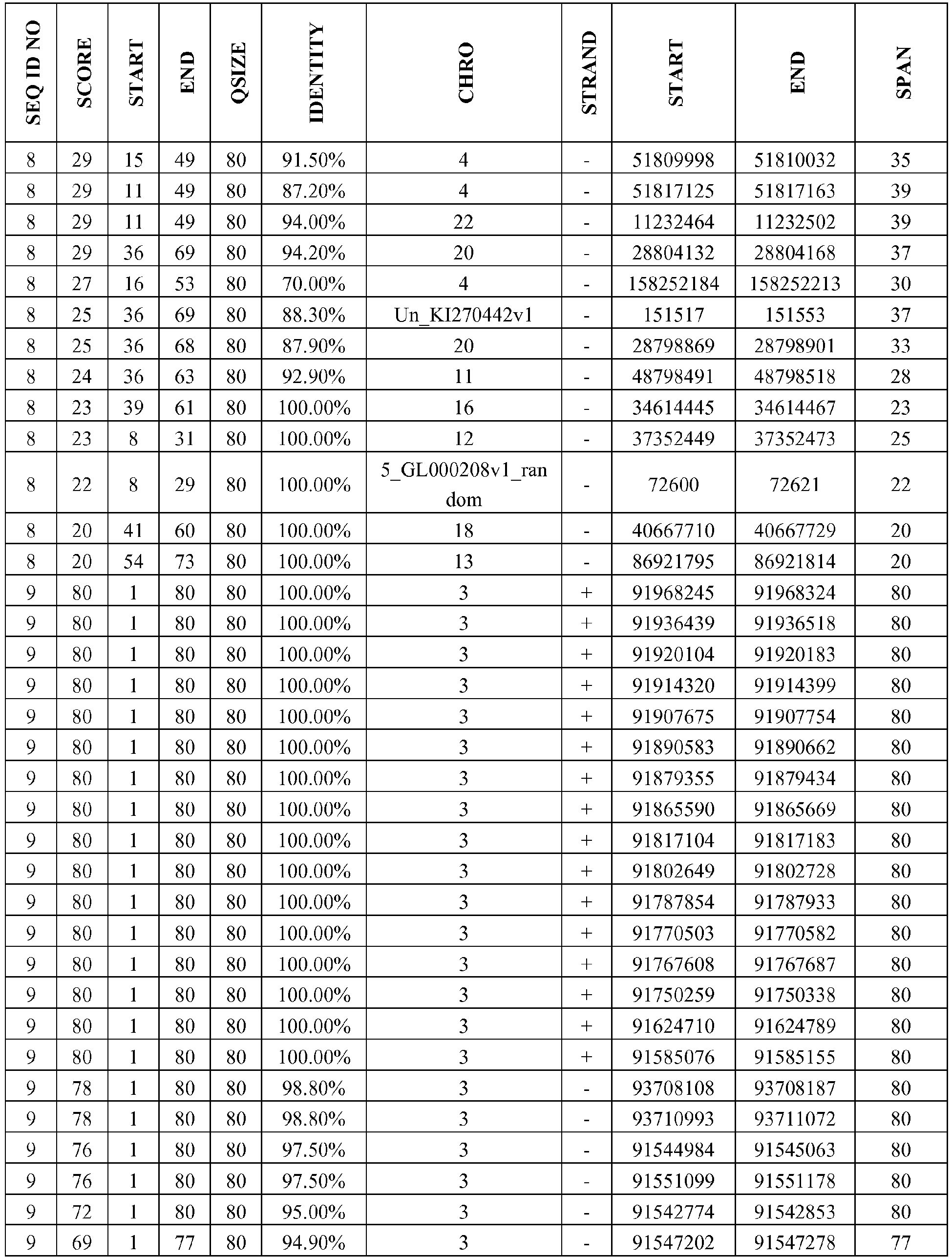

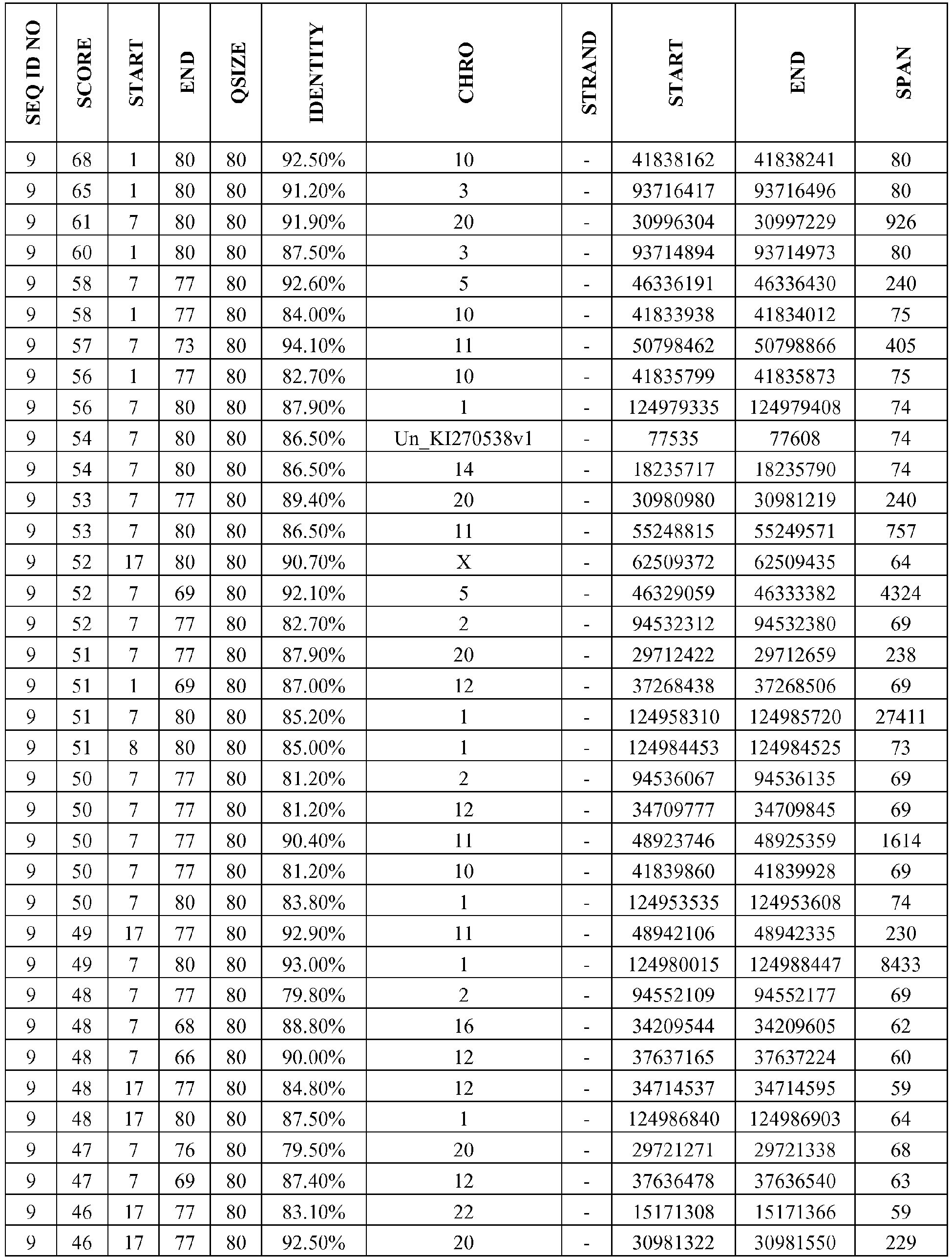

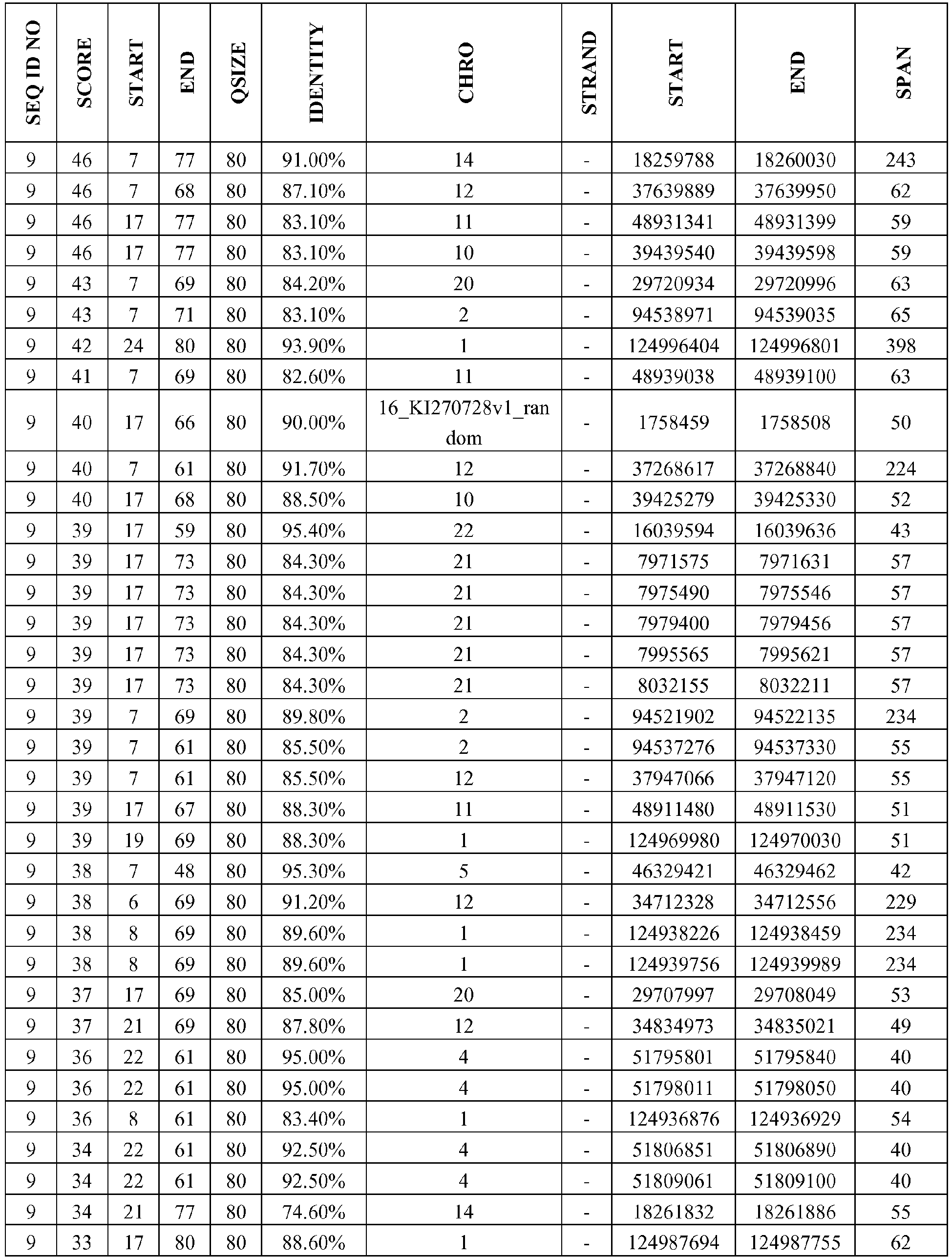

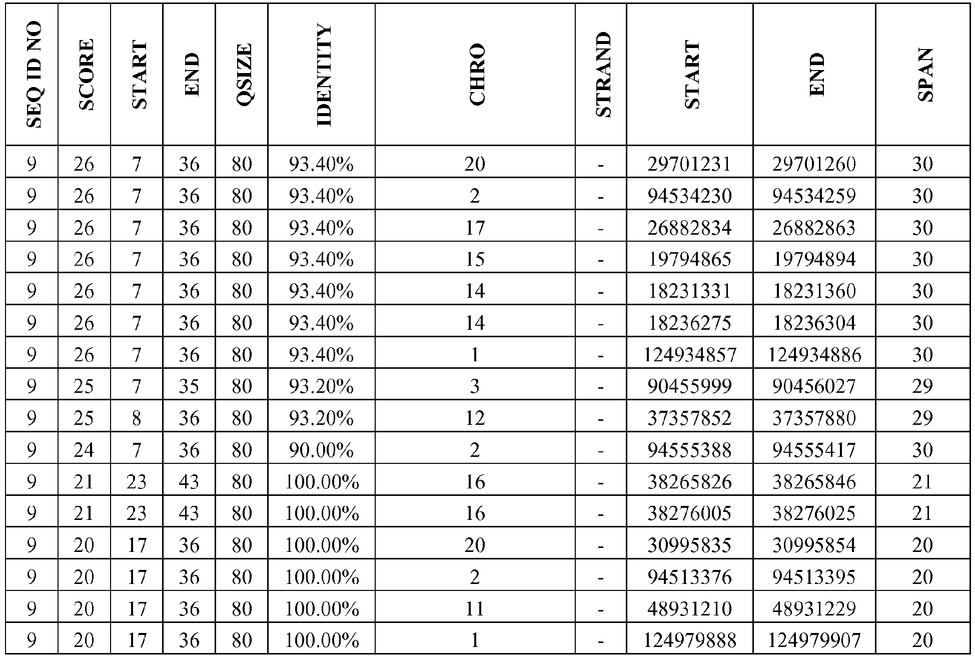

Although a number of sequences of each oligo had 100% homology to chromosome 3, there are also many off-target hits. For example, as shown in Table 2, Oligo 1 had 16 on-target hits, but also 24 off-target hits; Oligo 3 had 16 on-target hits, but also 33 off-target hits, etc.

**TABLE 2**

| SEQ ID NO | # on-target hits (CHR3) | # off-target hits (on other chromosomes) |
|---|---|---|
| | 100% unless otherwise specified | >85% identity (>70nt) |
| 1 | 16 | 24 |
| 2 | 16 | 20 |
| 3 | 16 | 33 |
| 4 | 16 | 8 |
| 5 | 16 | 34 |
| 6 | 15 | 5 |
| 7 | 18 (no 100%, >97%) | 3 |
| 8 | 16 | 2 |
| 9 | 16 | 3 |
| 10 | 18 | 1 |
| 11 | 15 | 20 |
| 12 | 19 | 15 |
| 13 | 16 | 23 |
| 14 | 18 (no 100%, >95%) | 2 |
| 15 | 18 | 0 |
| 16 | 17 (no 100%, >98%) | 6 |
| 17 | 16 | 33 |
| 18 | 16 (no 100%, >98%) | 1 |

These results strongly suggest that the centromere region of chromosome 3 may not contain specific sequences because there are many off-target hits to other chromosomes. Notwithstanding this, the present inventors have surprisingly identified 18 oligonucleotide sequences that hybridize highly specifically to the centromere region of chromosome 3, such that blocking DNA can be eliminated in hybridization assays if so desired.

### I. Definitions

Unless otherwise explained, all technical and scientific terms used herein have the same meaning as commonly understood by one of ordinary skill in the art to which a disclosed invention belongs. The singular terms "a," "an," and "the" include plural referents unless context clearly indicates otherwise. Similarly, the word "or" is intended to include "and" unless the context clearly indicates otherwise. "Comprising" means "including." Hence "comprising A or B" means "including A" or "including B" or "including A and B."

Suitable methods and materials for the practice and/or testing of embodiments of the disclosure are described below. Such methods and materials are illustrative only and are not intended to be limiting. Other methods and materials similar or equivalent to those described herein can be used. For example, conventional methods well known in the art to which the disclosure pertains are described in various general and more specific references, including, for example, Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2000); Ausubel et al., Short Protocols in Molecular Biology: A Compendium of Methods from Current Protocols in Molecular Biology, 4th ed., Wiley & Sons, 1999; Harlow and Lane, Antibodies: A Laboratory Manual, Cold Spring Harbor Laboratory Press, 1990; and Harlow and Lane, Using Antibodies: A Laboratory Manual, Cold Spring Harbor Laboratory Press, 1999.

In order to facilitate review of the various embodiments of the disclosure, the following explanations of specific terms are provided:
**Conjugating, joining, bonding or linking:** Covalently linking one molecule to another molecule to make a larger molecule. For example, making two polypeptides into one contiguous polypeptide molecule, or covalently attaching a mass tag, hapten, nucleic acid, or other molecule to a polypeptide, such as a scFv antibody.

**Detectable label:** A compound or composition that is conjugated directly or indirectly to another molecule (such as a nucleic acid probe) to facilitate detection of that molecule. Specific, non-limiting examples of labels include fluorescent and fluorogenic moieties, chromogenic moieties, haptens, affinity tags, and radioactive isotopes. The label can be directly detectable (e.g., optically detectable) or indirectly detectable (for example, via interaction with one or more additional molecules that are in turn detectable). Exemplary labels in the context of the probes disclosed herein are described below. Methods for labeling nucleic acids, and guidance in the choice of labels useful for various purposes, are discussed, e.g., in Sambrook and Russell, in Molecular Cloning: A Laboratory Manual, 3rd Ed., Cold Spring Harbor Laboratory Press (2001) and Ausubel et al., in Current Protocols in Molecular Biology, Greene Publishing Associates and Wiley-Intersciences (1987, and including updates).

**Hapten:** A molecule, typically a small molecule that can combine specifically with an antibody, but typically is substantially incapable of being immunogenic except in combination with a carrier molecule

**Hybridization:** To form base pairs between complementary regions of two strands of DNA, RNA, or between DNA and RNA, thereby forming a duplex molecule. Hybridization conditions resulting in particular degrees of stringency will vary depending upon the nature of the hybridization method and the composition and length of the hybridizing nucleic acid sequences. Generally, the temperature of hybridization and the ionic strength (such as the Na+ concentration) of the hybridization buffer will determine the stringency of hybridization. The presence of a chemical which decreases hybridization (such as formamide) in the hybridization buffer will also determine the stringency (Sadhu et al., J. Biosci. 6:817-821, 1984). Calculations regarding hybridization conditions for attaining particular degrees of stringency are discussed in Sambrook et al., (1989) Molecular Cloning, second edition, Cold Spring Harbor Laboratory, Plainview, NY (chapters 9 and 11). Hybridization conditions for ISH are also discussed in Landegent et al., Hum. Genet. 77:366-370, 1987; Lichter et al., Hum. Genet. 80:224-234, 1988; and Pinkel et al., Proc. Natl. Acad. Sci. USA 85:9138-9142, 1988.

**Isolated:** An "isolated" biological component (such as a nucleic acid molecule, protein, or cell) has been substantially separated or purified away from other biological components in a preparation, a cell of an organism, or the organism itself, in which the component occurs, such as other chromosomal and extra-chromosomal DNA and RNA, proteins and cells. Nucleic acid molecules and proteins that have been "isolated" include nucleic acid molecules and proteins purified by standard purification methods. The term also embraces nucleic acid molecules and proteins prepared by recombinant expression in a host cell as well as chemically synthesized nucleic acid molecules and proteins. In some examples, the nucleic acid probes disclosed herein are isolated nucleic acid probes.

**Linker:** As used herein, a linker is a molecule or group of atoms positioned between two moieties. For example, a mass tag conjugate may include a linker between the mass tag and the specific binding moiety. Typically, linkers are bifunctional, i.e., the linker includes a functional group at each end, wherein the functional groups are used to couple the linker to the two moieties. The two functional groups may be the same, i.e., a homobifunctional linker, or different, i.e., a heterobifunctional linker.

**Multiplex, -ed, -ing:** Embodiments of the present invention allow multiple targets in a sample to be detected substantially simultaneously, or sequentially, as desired, using plural different conjugates. Multiplexing can include identifying and/or quantifying nucleic acids generally, DNA, RNA, peptides, proteins, both individually and in any and all combinations. Multiplexing also can include detecting two or more of a gene, a messenger and a protein in a cell in its anatomic context.

**Phosphatidylinositol 3-kinase, p110 subunit (PIK3CA):** Also known as phosphoinositide-3-kinase, catalytic, alpha polypeptide. Human phosphatidylinositol 3-kinase (EC 2.7.1.137) is composed of 85-kD and 110-kD subunits. The 85-kD subunit lacks phosphatidylinositol 3-kinase activity and acts as an adaptor, coupling the 110-kD subunit (p110) to activated protein tyrosine kinases. The human p110 subunit is referred to herein as PIK3CA. Hiles et al. (Cell 70:419-429, 1992) found that the human cDNA for p110 predicts a 1,068-amino acid protein related to a protein which in S. cerevisiae is involved in the sorting of proteins to the vacuole. In COS-1 cells, p110 was catalytically active only when complexed with p85-alpha. Volinia et al. (Genomics 24:472-477, 1994) contributed to the structural and functional understanding of phosphatidylinositol 3-kinase by purifying, cloning, and subsequently elucidating the expression of the bovine enzyme. cDNA for the human PIK3CA encodes a protein 99% identical to the bovine p110. The chromosomal localization of the gene for human PIK3CA is shown to be at 3q21-qter as determined using somatic cell hybrids. *In situ* hybridization performed using Alu-PCR from the YAC DNA located the human gene in 3q26.3 [Chromosome 3: 178,865,902-178,957,881]. The sequence for the human PIK3CA gene was disclosed as early as 1994 by Volinia et al.

**Probe:** A nucleic acid molecule that is capable of hybridizing with a target nucleic acid molecule (e.g., genomic target nucleic acid molecule) and, when hybridized to the target, is capable of being detected either directly or indirectly. Thus probes permit the detection, and in some examples quantification, of a target nucleic acid molecule. In particular examples, a probe includes at least two segments complementary to uniquely specific nucleic acid sequences of a target nucleic acid molecule and are thus capable of specifically hybridizing to at least a portion of the target nucleic acid molecule. Generally, once at least one segment or portion of a segment has (and remains) hybridized to the target nucleic acid molecule other portions of the probe may (but need not) be physically constrained from hybridizing to those other portions' cognate binding sites in the target (e.g., such other portions are too far distant from their cognate binding sites); however, other nucleic acid molecules present in the probe can bind to one another, thus amplifying signal from the probe. A probe can be referred to as a "labeled nucleic acid probe," indicating that the probe is coupled directly or indirectly to a detectable moiety or "label," which renders the probe detectable.

**Sample:** A specimen containing DNA (for example, genomic DNA), RNA (including mRNA), protein, or combinations thereof, obtained from a subject. Examples include, but are not limited to, chromosomal preparations, peripheral blood, urine, saliva, tissue biopsy, fine needle aspirate, surgical specimen, bone marrow, amniocentesis samples, and autopsy material. In one example, a sample includes genomic DNA. In some examples, the sample is a cytogenetic preparation, for example which can be placed on microscope slides. In particular examples, samples are used directly, or can be manipulated prior to use, for example, by fixing (e.g., using formalin).

**Sequence identity:** The identity (or similarity) between two or more nucleic acid sequences is expressed in terms of the identity or similarity between the sequences. Sequence identity can be measured in terms of percentage identity; the higher the percentage, the more identical the sequences are. Sequence similarity can be measured in terms of percentage similarity (which takes into account conservative amino acid substitutions); the higher the percentage, the more similar the sequences are.

Methods of alignment of sequences for comparison are well known in the art. Various programs and alignment algorithms are described in: Smith & Waterman, Adv. Appl. Math. 2:482, 1981; Needleman & Wunsch, J. Mol. Biol. 48:443, 1970; Pearson & Lipman, Proc. Natl. Acad. Sci. USA 85:2444, 1988; Higgins & Sharp, Gene, 73:237-44, 1988; Higgins & Sharp, CABIOS 5:151-3, 1989; Corpet et al., Nuc. Acids Res. 16:10881-90, 1988; Huang et al. Computer Appls. in the Biosciences 8:155-65, 1992; and Pearson et al., Meth. Mol. Bio. 24:307-31, 1994. Altschul et al., J. Mol. Biol. 215:403-10, 1990, presents a detailed consideration of sequence alignment methods and homology calculations.

The NCBI Basic Local Alignment Search Tool (BLAST) (Altschul et al., J. Mol. Biol. 215:403-10, 1990) is available from several sources, including the National Center for Biotechnology and on the Internet, for use in connection with the sequence analysis programs blastp, blastn, blastx, tblastn and tblastx. Additional information can be found at the NCBI web site. BLASTN may be used to compare nucleic acid sequences, while BLASTP may be used to compare amino acid sequences. If the two compared sequences share homology, then the designated output file will present those regions of homology as aligned sequences. If the two compared sequences do not share homology, then the designated output file will not present aligned sequences. The BLAST-like alignment tool (BLAT) may also be used to compare nucleic acid sequences (Kent, Genome Res. 12:656-664, 2002). BLAT is available from several sources, including Kent Informatics (Santa Cruz, CA) and on the Internet (genome.ucsc.edu).

Once aligned, the number of matches is determined by counting the number of positions where an identical nucleotide or amino acid residue is presented in both sequences. The percent sequence identity is determined by dividing the number of matches either by the length of the sequence set forth in the identified sequence, or by an articulated length (such as 100 consecutive nucleotides or amino acid residues from a sequence set forth in an identified sequence), followed by multiplying the resulting value by 100. For example, a nucleic acid sequence that has 1166 matches when aligned with a test sequence having 1554 nucleotides is 75.0 percent identical to the test sequence (1166÷1554*100=75.0). The percent sequence identity value is rounded to the nearest tenth. For example, 75.11, 75.12, 75.13, and 75.14 are rounded down to 75.1, while 75.15, 75.16, 75.17, 75.18, and 75.19 are rounded up to 75.2. The length value will always be an integer. In another example, a target sequence containing a 20-nucleotide region that aligns with 15 consecutive nucleotides from an identified sequence as follows contains a region that shares 75 percent sequence identity to that identified sequence (that is, 15÷20*100=75).

**Subject:** Any multi-cellular vertebrate organism, such as human or non-human mammals (e.g., veterinary subjects).

**Target nucleic acid sequence or molecule:** A defined region or particular portion of a nucleic acid molecule, for example a portion of a genome (such as a gene or a region of mammalian genomic DNA containing a gene of interest). In an example where the target nucleic acid sequence is a target genomic sequence, such a target can be defined by its position on a chromosome (e.g., in a normal cell), for example, according to cytogenetic nomenclature by reference to a particular location on a chromosome; by reference to its location on a genetic map; by reference to a hypothetical or assembled contig; by its specific sequence or function; by its gene or protein name; or by any other means that uniquely identifies it from among other genetic sequences of a genome. In some examples, the target nucleic acid sequence is mammalian genomic sequence (for example human genomic sequence).

In some examples, alterations of a target nucleic acid sequence (e.g., genomic nucleic acid sequence) are "associated with" a disease or condition. In some examples, detection of the target nucleic acid sequence can be used to infer the status of a sample with respect to the disease or condition. For example, the target nucleic acid sequence can exist in two (or more) distinguishable forms, such that a first form correlates with absence of a disease or condition and a second (or different) form correlates with the presence of the disease or condition. The two different forms can be qualitatively distinguishable, such as by polynucleotide polymorphisms, and/or the two different forms can be quantitatively distinguishable, such as by the number of copies of the target nucleic acid sequence that are present in a cell.

**Uniquely specific sequence:** A nucleic acid sequence (for example, a sequence of at least of at least 20 basepairs (bp) (such as at least 20 bp, 30 bp, 40 bp, 50 bp, 60 bp, 70 bp, 80 bp, 90 bp, 100 bp, or more) that is present only one time in a haploid genome of an organism. In a particular example, a uniquely specific nucleic acid sequence is a nucleic acid sequence from a target nucleic acid that has 100% sequence identity with the target nucleic acid and has no significant identity to any other nucleic acid sequences present in the specific haploid genome that includes the target nucleic acid.

**Vector:** Any nucleic acid that acts as a carrier for other ("foreign") nucleic acid sequences that are not native to the vector. When introduced into an appropriate host cell a vector may replicate itself (and, thereby, the foreign nucleic acid sequence) or express at least a portion of the foreign nucleic acid sequence. In one context, a vector is a linear or circular nucleic acid into which a nucleic acid sequence of interest is introduced (for example, cloned) for the purpose of replication (e.g., production) and/or manipulation using standard recombinant nucleic acid techniques (e.g., restriction digestion). A vector can include nucleic acid sequences that permit it to replicate in a host cell, such as an origin of replication. A vector can also include one or more selectable marker genes and other genetic elements known in the art. Common vectors include, for example, plasmids, cosmids, phage, phagemids, artificial chromosomes (e.g., BAC, PAC, HAC, YAC), and hybrids that incorporate features of more than one of these types of vectors. Typically, a vector includes one or more unique restriction sites (and in some cases a multi-cloning site) to facilitate insertion of a target nucleic acid sequence.

### II. Systems for in situ Hybridization for Chromosome Enumeration

### A. CHROMOSOME 3

The most common target for a control region of chromosome 3 (CHR3) ISH is the centromeric region. The centromeric regions of all human chromosomes are characterized by distinct subsets of a diverse tandemly repeated DNA family, alpha satellite. Since alpha satellite DNA clusters most often contain monomer variants that differ from the consensus sequence by up to 40%, blocking DNA is usually included with the probes to suppress sequences contained within the target loci that are common to other chromosomes.

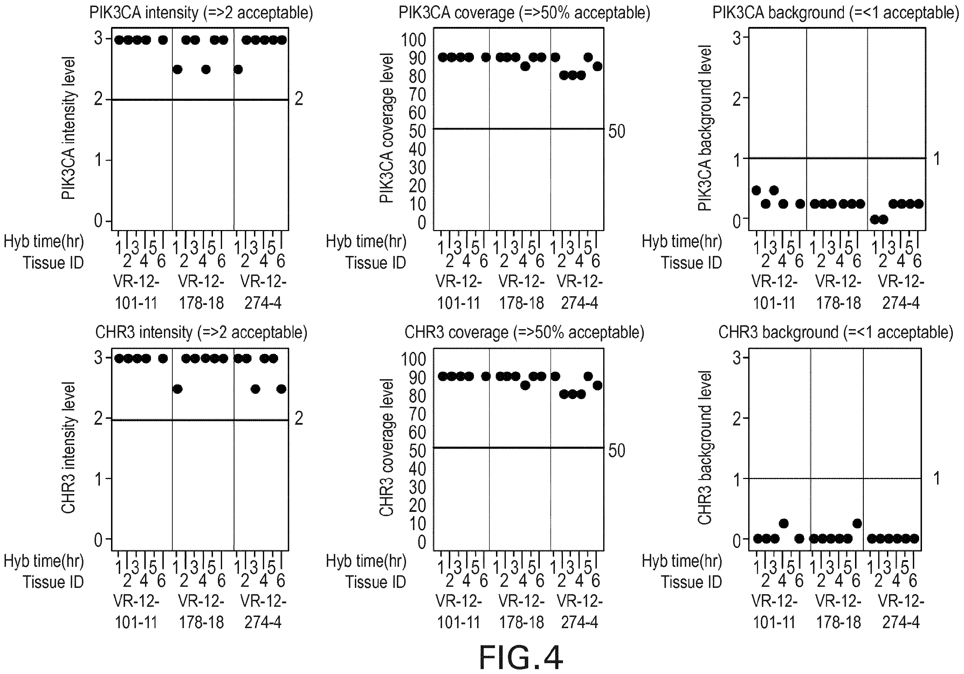

Single-stranded probes directed to the control region (centromeric region) of chromosome 3 were designed that achieved acceptable signal intensity levels and background levels within 1 hour of hybridization (see FIG. 4) and without the use of blocking DNA. For example, the probes are configured to achieve a staining intensity of greater than or equal to 2 and staining coverage of greater than or equal to 50% of nuclei. Also designed were single-stranded probes directed to a target region near and within the PIK3CA gene locus that also achieved acceptable signal intensity levels and background levels within 1 hour of hybridization (see FIG. 4) and without the use of blocking DNA. The criteria in Table 3 were used to evaluate whether the ISH assay is acceptable or not acceptable.

**TABLE 3**

| | **Acceptable (A)** | **Not Acceptable (N)** |
|---|---|---|
| **Signal Intensity** | **3,** Signals are bright and easily identified in > 80% of cells within the target region. | **1**, Specific signals are visible but too weak to reliably identify in ≥ 50% of the targeted region. |
| | **2**, Specific signals are sufficiently intense to reliably identify in > 50% of cells within the targeted region. | **0**.5, Signals are visible but absent or too weak to reliably identify in 80% of cells. |
| | | **0**, Signals are not visible. |
| **Background** | **1**, Background signals (either punctate signals or diffuse, hazy staining) are present but are sufficiently weak in intensity within the nuclei to permit reliable identification of specific signals in > 50% of cells within the target region. | **3**, Background signals (punctate signals, diffuse staining, haze) cover 75-100% of cells within the target region and are sufficiently intense to obscure specific signals. |
| | **0**, Background staining is not observed in > 80% of cells within the target region. | **2**, Background signals (punctate signals, diffuse staining, haze) cover 50-75% of cells within the target region and are sufficiently intense to obscure specific signals |

From the perspective of manufacturing and quality control, a single-stranded probe having an exact structure are more reproducibly manufactured using oligonucleotide synthesis compared to the approaches based on PCR, nick translation, or other random synthetic approaches. From the perspective of cost analysis, the probes that do not require blocking DNA provide for a less expensive assay.

The present disclosure describes systems for ISH featuring a control probe specific to a control region of a chromosome, e.g., a centromere target of a chromosome. The chromosome detected may be chromosome 3. The control probe is configured to achieve a staining intensity of greater than or equal to 2 and staining coverage of greater than or equal to 50% of the number of nuclei within 3 hours when applied to a control sample (e.g., as described above, TABLE 3). In some examples, a staining coverage of ≥55% of the number of nuclei may be achieved within 3 hours, e.g., ≥60% of the number of nuclei, ≥65% of the number of nuclei, ≥70% of the number of nuclei, ≥75% of the number of nuclei, ≥80% of the number of nuclei, ≥85% of the number of nuclei, ≥90% of the number of nuclei.

In some embodiments, the systems for ISH also feature a target probe specific for a target region (e.g., for detecting a target gene) on the corresponding chromosome.

In some embodiments, the control probe comprises a first plurality (e.g., a plurality of a single probe, a plurality of different probes such as a set or pool of probes) of single-stranded oligonucleotide probes. One or more of the plurality of probes may comprise a sequence selected from the group consisting of SEQ ID NOs: 1-18 (see Table 4 below). In some embodiments, one or more of the first plurality of probes comprise a truncated version (e.g., at least 30 contiguous bp, at least 35 contiguous bp, at least 40 contiguous bp, at least 45 contiguous bp, at least 50 contiguous bp, at least 55 contiguous bp, at least 60 contiguous bp, at least 65 contiguous bp, at least 70 contiguous bp, at least 75 contiguous bp, etc.) of one of the sequences in Table 4 (SEQ ID NOs: 1-18). In some embodiments, one or more of the first plurality of probes comprises a sequence that has at least 70% sequence identity, at least 75% sequence identity, at least 80% sequence identity, at least 85% sequence identity, at least 90% sequence identity, or at least 95% sequence identity to one of the sequences in Table 4 (SEQ ID NOs: 1-18). The first plurality of single-stranded oligonucleotide probes is configured to hybridize uniquely and specifically to a portion of the control region of human chromosome 3 so that other chromosomes or portions thereof are not evidently labeled.

As used herein, reference to use of SEQ ID NOs: 1-18 may also include the use of complementary sequences of SEQ ID NOs: 1-18.

In some embodiments, the probes target between 2 and 18 distinct portions within the control region. In some embodiments, the probes target between 4 and 18 distinct portions within the control region. In some embodiments, the probes target between 6 and 18 distinct portions within the control region. In some embodiments, the probes target between 8 and 18 distinct portions within the control region. In some embodiments, the probes target between 10 and 18 distinct portions within the control region. In some embodiments, the probes target between 12 and 18 distinct portions within the control region. In some embodiments, the probes target between 14 and 18 distinct portions within the control region. In some embodiments, the probes target between 16 and 18 distinct portions within the control region. In some embodiments, the probes target between 2 and 12 distinct portions within the control region. In some embodiments, the probes target between 4 and 12 distinct portions within the control region. In some embodiments, the probes target between 6 and 12 distinct portions within the control region. In some embodiments, the probes target between 8 and 12 distinct portions within the control region. In some embodiments, the probes target between 10 and 12 distinct portions within the control region.

**TABLE 4**

| **SEQ ID NO:** | **Sequences** | **Length** |
|---|---|---|
| 1 | TCTGTGGAATTTGCAAGGGGAGATTTCAAGCACTTTGAGGC CATTGGTGGAAAAGGAAATATCTTCGTATGAAAACTAGA | 80 |
| 2 | CAGAATCATTCTCAGGAACTACTTTGTGATATGTGCATTCA ACTCCCAGAGTTTAACCTTTCTTTTCATAGATGAGTTTG | 80 |
| 3 | AATGCTAGACAGAAGAATTCTCAGTAACTTCTTTTGGGATG TATGTATTCAAATCAGAGAGTTGAACCTTCCTTTAGACA | 80 |
| 4 | GAGCGGATTGGAAACACTCTTTTTGTGGAATTTGCAAGTGG AAAATTCTAGCAGTATGAGGCCAATGGTACAAAAGGAAA | 80 |
| 5 | TATCTTCGTATAAAAACTAGACAGTATCATTCTCAGAAACT GCTTTGTGATGTGTGTATTAAACTCACAGAGTTGAACAT | 80 |
| 6 | TTCTTTGCATAGAGCAGTTTGGAAAGACTTAGTTTGTGCAG TGTGCAAGTGGATATTTGGAACTCTTTGAGGCCTTCGTT | 80 |
| 7 | ATGTGTGCATTCGGCTCACAGAGTTGAACCTTACTTTGGAA AGAGCAGTTTTCTAACACTCTTTTTGTAAAAGTTCCAAG | 80 |
| 8 | TGAATACTTTGAGTGCTTTGAAGCCTACGGTTGACAACGAA ATATCTTCCTGTAAAAACTACAAAGAATCATTCGCAGAA | 80 |
| 9 | ACCACGTTGTGATCTCTGCATTCAACTCACAGAGTTCAACC TTTCTTCCTATAGAGCAGTTATGAAACAGTCTCTTTGTA | 80 |
| 10 | GAATTTGCAAGGGTGTATTTAGAGGGCATTGAAGCCTACG GTAGAAAAGGAAATATCTTACCATAAAATCTAGTCAGAAG | 80 |
| 11 | CATTCTCAGCAACTGAGTTGTGATGTTTGCATTCAACTCAC AGAGTTCAACATTCCTTTTAATGGAGCGGTTTTGAAACA | 80 |
| 12 | ATTGGAAACACTCTTTTGTGGAATTTTCAGGTGGAGGTAT CAAGCGCTTTGAGGCCAATGATAGAAAAGGAAATACCTT | 80 |
| 13 | CGTATAATAATTAGACGGAATCATTCTCAGAAACCGCTTTG CAATGTGTGCGTTCAACTCACAGTGTTTAACCTTTCTTT | 80 |
| 14 | TCATACAAGTTGTTTCGAAACACTCTTTTGCAGAATCTGC AAGTGGATATTTGGACCTCTTTGAAGTCTTCGTTGGAAA | 80 |
| 15 | GCCTTCGTAGTAAACGGGATTTCTTCGTGTAATGATAGACA ATAGAATTCTCAGTGAATTTTTTTCTGTGTGTGTGTATT | 80 |
| 16 | TTGAAGTGAATGGTAGGAAAGGAAATACCTTCGTATAAAA ACTAGACGGAGTCATTCTCAGAAACTACTTTGTGATGTTT | 80 |
| 17 | ATTTGCAAGTGGAGAATTCTAGCGCTTTGACGCCAATGGTA GAAAGGAAATATCTTCGTATAAAAACTAGACAGTATCAT | 80 |
| 18 | CGGTTGGAAACACTTTTTGTGGAATTTTCAGGGGGAGACTT CAAGCGCTTTGAAGTGAATGGTAGGAAAGGAAATACCTT | 80 |

The first plurality of single-stranded oligonucleotide probes may be constructed in a variety of lengths. For example, the probes each comprise between 40 to 100 nucleotides. In some embodiments, the probes each comprise between 50 to 100 nucleotides. In some examples, the probes each comprise between 60 to 110 nucleotides. In some examples, the probes each comprise between 40 to 120 nucleotides. In some examples, the probes each comprise at least 40 nucleotides. In some examples, the probes each comprise at least 50 nucleotides. In some examples, the probes each comprise at least 60 nucleotides. In some examples, the probes each comprise at least 70 nucleotides.

The present invention also features slides with a plurality of nuclei stained for human chromosome 3. The slide may be contacted with one or more of the above systems (e.g., probes). The slide features enumerable signals indicative of the number of chromosome 3 centromere regions present in a cell, e.g., cells should exhibit two copies of the CHR3 centromere normally.

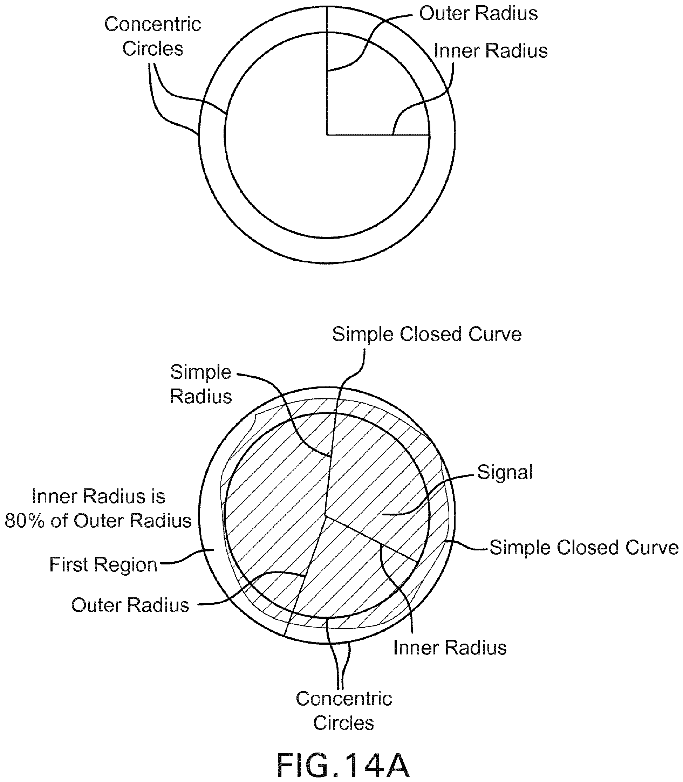

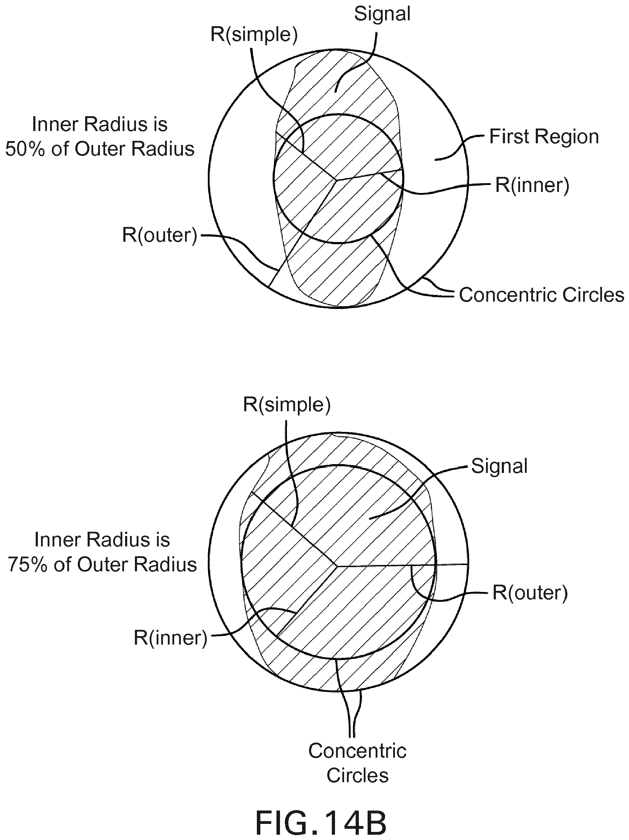

In some examples, more than 50% of the nuclei have enumerable signals for the chromosome. An enumerable signal may be a generally round shape. The round shape can be defined as shown in FIG. 14, wherein a round shape is a simple closed curve that fits within a first region, the first region lies on and outside an inner circle and on and inside a concentric outer circle, the inner circle has an inner radius (Rᵢₙ) and the outer circle has an outer radius (Rₒᵤₜ), wherein the simple close curve has a radius Rₛᵢₘₚₗₑ, wherein Rᵢₙ ≤ Rₛᵢₘₚₗₑ ≤ Rₒᵤₜ, and wherein, Rᵢₙ is ≥ 50% of Rₒᵤₜ. One aspect of the round shape is that it is a condensed signal compared to a diffuse signal. The hybridization of the probe to the target may not form a round signal at a very high magnification, but with a chromogenic deposition and at lower magnifications (e.g. those customary within the anatomic pathology laboratory, 100x, 600x) the signal appears round. The parameters of a generally round shape are specified so as to distinguish the signals associated with the present probes to those previously possible. Another aspect of the present disclosure is that the extent to which the signals are in a generally round shape, condensed, and regular, the ability to read the signals by either a pathologist or using a machine reader, is enhanced.

In some examples, the inner radius is no less than 40% of the outer radius. In some examples, the inner radius is no less than 50% of the outer radius. In some examples, the inner radius is no less than 55% of the outer radius. In some examples, the inner radius is no less than 60% of the outer radius. In some examples, the inner radius is no less than 65% of the outer radius. In some examples, the inner radius is no less than 70% of the outer radius. In some examples, the inner radius is no less than 75% of the outer radius. In some examples, the inner radius is no less than 80% of the outer radius. In some examples, the inner radius is no less than 85% of the outer radius. In some examples, the inner radius is no less than 90% of the outer radius.

In some examples, more than 60% of the nuclei have enumerable signals for the chromosome. In some examples, more than 70% of the nuclei have enumerable signals for the chromosome. In some examples, more than 80% of the nuclei have enumerable signals for the chromosome. In some examples, more than 90% of the nuclei have enumerable signals for the chromosome. The nuclei may not be enumerable if the tissue sectioning process has destroyed that portion of the cell, if that portion of the cell is divided between two slides, or if that portion of the cell is wholly within a separate slide. The nuclei may also be enumerable if the tissue condition prevents probe penetration to the specific binding site (i.e. the cell is not sufficiently accessible to the probe) or if the target region of DNA is substantially degraded.

In some examples, the sum of the surface area covered by staining signal is calculated and assigned a 100% value, and at least 50% of the sum of the surface area is derived from discrete round signals (or round shapes).

A round shape can be defined as shown in FIG. 14, wherein a round shape is a simple closed curve that fits within a first region, the first region lies on and outside an inner circle and on and inside a concentric outer circle, the inner circle has an inner radius (Rᵢₙ) and the outer circle has a outer radius (Rₒᵤₜ), wherein the simple close curve has a radius Rₛᵢₘₚₗₑ, wherein Rᵢₙ ≤ Rₛᵢₘₚₗₑ ≤ Rₒᵤₜ, and wherein, Rᵢₙ is ≥ 50% of Rₒᵤₜ.

In some examples, the inner radius is no less than 50% of the outer radius. In some examples, more than 60% of said sum of the surface area is derived from discrete round signals. In some examples, more than 70% of said sum of the surface area is derived from discrete round signals. In some examples, the inner radius is no less than 60% of the outer radius. In some examples, the inner radius is no less than 75% of the outer radius. In some examples, the inner radius is no less than 90% of the outer radius.

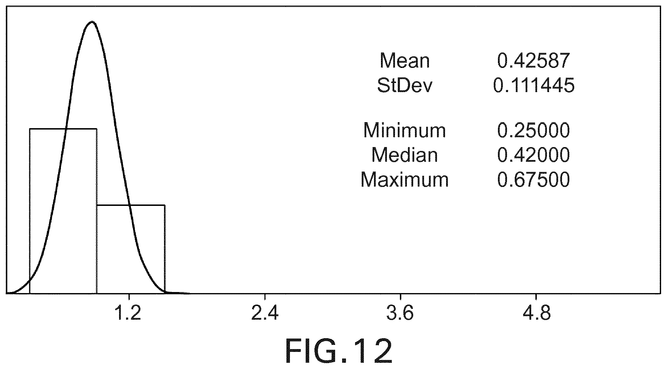

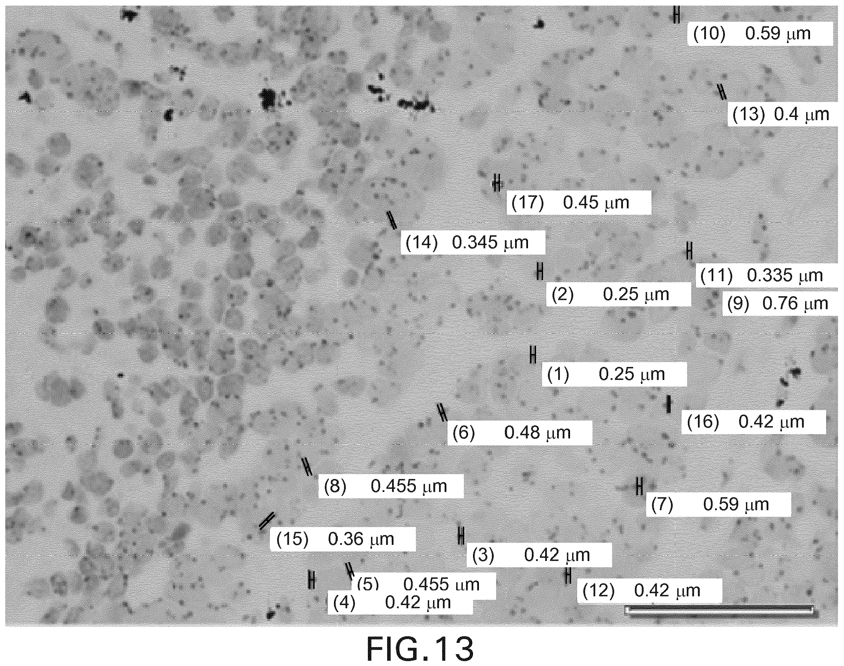

Referring to FIG. 12-13, the radii (e.g., outer radii) of a plurality of signals were measured. In some examples, the outer radius is between about 0.25 to 0.675 µm. In some examples, the outer radius is between about 0.2 to 0.75 µm. In some examples, the outer radius is between about 0.15 to 1 µm. In some examples, the average outer radius of the enumerable signals is between about 0.2 to 0.75 µm. In some examples, the average outer radius of the enumerable signals has a standard deviation of less than 0.5 µm. In some examples, the average outer radius of the enumerable signals has a standard deviation of less than 0.25 µm.

In some examples, the enumerable round signals are mono-sized. In particular, a population of "mono-sized" round signals may have the Rₛᵢₘₚₗₑ being within 15% plus or minus of each other. In some examples, the population of "mono-sized" round signals have the Rₛᵢₘₚₗₑ being within 10% plus or minus of each other. In some examples, the population of "mono-sized" round signals have the Rₛᵢₘₚₗₑ being within 5% plus or minus of each other.

### B. TARGET GENE (PIK3CA)

In some embodiments, the systems for ISH also feature a target probe specific for a target region (e.g., for detecting a target gene, for gene copy enumeration) on the corresponding chromosome.

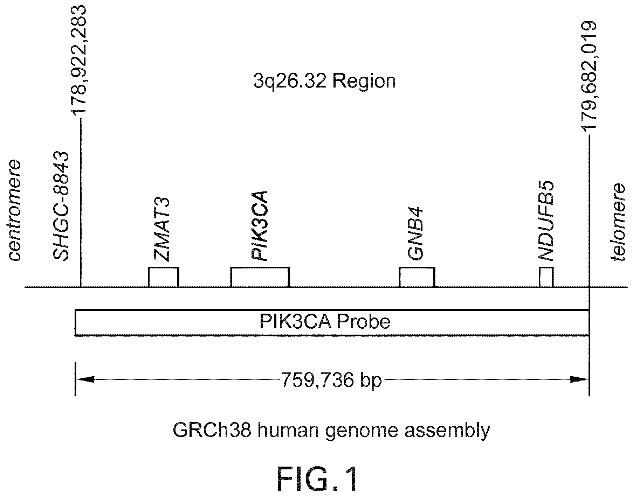

The target region may comprise the PIK3CA gene locus (or nearby nucleotides). Disclosed herein are probes directed to the human PIK3CA gene (e.g., Gene ID No. 5290; NC_000003.11 (178866311..178952500)), which is present in GENBANK® on April 30, 2012) and depicted in FIG. 1. As described below in detail in EXAMPLE 1, the PIK3CA target probe is specific to a region between nucleotides 178,640,071 and 179,399,807 of human chromosome 3.

In some examples, the target probe comprises a second plurality (e.g., a plurality of a single probe, a plurality of different probes such as a set or pool of probes) of single-stranded oligonucleotide probes. One or more of the plurality of probes may comprise a sequence selected from the group consisting of SEQ ID NOs: 19-1230 (see FIG. 14). The second plurality of single-stranded oligonucleotide probes is configured to hybridize uniquely and specifically to a portion of the target region of the corresponding chromosome so that other genes or chromosomes or portions thereof are not evidently labeled.

### III. Kits

Also disclosed are kits including one or more of the oligonucleotide probes (for example, one or more of SEQ ID NOs: 1-18). For example, kits can include at least one probe (such as at least 1, 2, 3, 4, 5, 6, 7, 8, 9, 10, or more probes) or at least one probe set (such as at least 1, 2, 3, 4, or 5 probe sets) as described herein. In one example, the kit comprises probes such as at least 1, 2, 3, 4, 5, 6, 7, 8, 9, 10, 11, 12, 13, 14, 15, 16, 17 or all of SEQ ID NOs: 1-18 (or sequences at least 60%, at least 70%, at least 75%, at least 80%, at least 85%, or at least 90% identical to SEQ ID NOs: 1-18; or truncated versions of SEQ ID NOs: 1-18). In other examples, the probes (or the probe set) are in a single container.

The kits may also comprise one or more reagents for detecting the probe (for example, by *in situ* hybridization), or for producing a detectably labeled probe. For example, a kit can include at least one of the disclosed nucleic acid probes or probe sets, along with one or more buffers, labeled dNTPs, a labeling enzyme (such as a polymerase), primers, nuclease free water, and instructions for producing a labeled probe. In another example, the kit includes one or more of the disclosed nucleic acid probes (unlabeled or labeled) along with buffers and other reagents for performing *in situ* hybridization. For example, if one or more unlabeled probes are included in the kit, labeling reagents can also be included, along with specific detection agents (for example, fluorescent, chromogenic, luminescent and/or radiometric) and other reagents for performing an *in situ* hybridization assay, such as paraffin pretreatment buffer, protease(s) and protease buffer, prehybridization buffer, hybridization buffer, wash buffer, counterstain(s), mounting medium, or combinations thereof. In some examples, such kit components are present in separate containers. The kit can optionally further include control slides (such as positive or negative controls) for assessing hybridization and signal of the probe(s).

In certain examples, the kits include avidin, antibodies, and/or receptors (or other anti-ligands). Optionally, one or more of the detection agents (including a primary detection agent, and optionally, secondary, tertiary or additional detection reagents) are labeled, for example, with a hapten or fluorophore (such as a fluorescent dye or quantum dot). In some instances, the detection reagents are labeled with different detectable moieties (for example, different fluorescent dyes, spectrally distinguishable quantum dots, different haptens, etc.). For example, a kit can include two or more nucleic acid probes or probe sets that correspond to and are capable of hybridizing to different target nucleic acids (for example, any of the target nucleic acids disclosed herein). The first probe or probe set can be labeled with a first detectable label (e.g., hapten, fluorophore, etc.), the second probe or probe set can be labeled with a second detectable label, and any additional probes or probe sets (e.g., third, fourth, fifth, etc.) can be labeled with additional detectable labels. The first, second, and any subsequent probes or probe sets can be labeled with different detectable labels, although other detection schemes are possible. If the probe(s) are labeled with indirectly detectable labels, such as haptens, the kits can include detection agents (such as labeled avidin, antibodies or other specific binding agents) for some or all of the probes. In one example, the kit includes probes and detection reagents suitable for multiplex ISH.

In one example, the kit also includes an antibody conjugate, such as an antibody conjugated to a label (e.g., an enzyme, fluorophore, or fluorescent nanoparticle). In some examples, the antibody is conjugated to the label through a linker, such as PEG, 6X-His, streptavidin, or GST.

### IV. Detectable Labels and Methods of Labeling

The probes disclosed herein may comprise one or more labels (e.g., at least 1 at least 2, at least 3, at least 4, at least 5, at least 6, etc.), for example to permit detection of the probe/nucleic acid sequence (or region) of interest. In various applications, such as *in situ* hybridization procedures, a nucleic acid probe includes a label (e.g., a detectable label). A "detectable label" is a molecule or material that can be used to produce a detectable signal that indicates the presence or concentration of the probe (particularly the bound or hybridized probe) in a sample. Thus, a labeled nucleic acid molecule provides an indicator of the presence or quantity (for example, gene copy number) of a target nucleic acid (to which the labeled uniquely specific nucleic acid molecule is bound or hybridized) in a sample. The disclosure is not limited to the use of particular labels, although examples are provided.

A label associated with one or more nucleic acid molecules (such as the disclosed probes) can be detected either directly or indirectly. A label can be detected by any known or yet to be discovered mechanism including absorption, emission and/or scattering of a photon (including radio frequency, microwave frequency, infrared frequency, visible frequency and ultra-violet frequency photons). Detectable labels include colored, fluorescent, phosphorescent and luminescent molecules and materials, catalysts (such as enzymes) that convert one substance into another substance to provide a detectable difference (such as by converting a colorless substance into a colored substance or vice versa, or by producing a precipitate or increasing sample turbidity), haptens that can be detected by antibody binding interactions, and paramagnetic and magnetic molecules or materials.

Particular examples of detectable labels include fluorescent molecules (or fluorochromes). Numerous fluorochromes are known to those of skill in the art, and can be selected, for example from Life Technologies, e.g., see, The Handbook - A Guide to Fluorescent Probes and Labeling Technologies. Examples of particular fluorophores that can be attached (for example, chemically conjugated) to a nucleic acid molecule (such as a uniquely specific binding region) are provided in U.S. Patent No. 5,866,366 to Nazarenko et al., such as 4-acetamido-4'-isothiocyanatostilbene-2,2'disulfonic acid, acridine and derivatives such as acridine and acridine isothiocyanate, 5-(2'-aminoethyl)aminonaphthalene-1-sulfonic acid (EDANS), 4-amino-N-[3-vinylsulfonyl)phenyl]naphthalimide-3,5 disulfonate (Lucifer Yellow VS), N-(4-anilino-1-naphthyl)maleimide, anthranilamide, Brilliant Yellow, coumarin and derivatives such as coumarin, 7-amino-4-methylcoumarin (AMC, Coumarin 120), 7-amino-4-trifluoromethylcouluarin (Coumarin 151); cyanosine; 4',6-diaminidino-2-phenylindole (DAPI); 5', 5"-dibromopyrogallol-sulfonephthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein, fluorescein isothiocyanate (FITC), and QFITC (XRITC); 2', 7'-difluorofluorescein (OREGON GREEN®); fluorescamine; IR144; IR1446; Malachite Green isothiocyanate; 4-methylumbelliferone; ortho-cresolphthalein; nitrotyrosine; pararosaniline; Phenol Red; B-phycoerythrin; o-phthaldialdehyde; pyrene and derivatives such as pyrene, pyrene butyrate and succinimidyl 1-pyrene butyrate; Reactive Red 4 (Cibacron Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, rhodamine green, sulforhodamine B, sulforhodamine 101 and sulfonyl chloride derivative of sulforhodamine 101 (Texas Red); N,N,N',N'-tetramethyl-6-carboxyrhodamine (TAMRA); tetramethyl rhodamine; tetramethyl rhodamine isothiocyanate (TRITC); riboflavin; rosolic acid and terbium chelate derivatives.

Other suitable fluorophores include thiol-reactive europium chelates, which emit at approximately 617 nm (Heyduk and Heyduk, Analyt. Biochem. 248:216-27, 1997; J. Biol. Chem. 274:3315-22, 1999), as well as GFP, Lissamine™, diethylaminocoumarin, fluorescein chlorotriazinyl, naphthofluorescein, 4,7-dichlororhodamine and xanthene (as described in U.S. Patent No. 5,800,996 to Lee et al.) and derivatives thereof. Other fluorophores known to those skilled in the art can also be used, for example those available from Life Technologies (Carlsbad, CA) and including the ALEXA FLUOR® series of dyes (for example, as described in U.S. Patent Nos. 5,696,157, 6,130,101 and 6, 716,979), the BODIPY series of dyes (dipyrrometheneboron difluoride dyes, for example as described in U.S. Patent Nos. 4,774,339, 5,187,288, 5,248,782, 5,274,113, 5,338,854, 5,451,663 and 5,433,896), Cascade Blue (an amine reactive derivative of the sulfonated pyrene described in U.S. Patent No. 5,132,432) and Marina Blue (U.S. Patent No. 5,830,912). In addition to the fluorochromes described above, a fluorescent label can be a fluorescent nanoparticle, such as a semiconductor nanocrystal, e.g., a quantum dot. Additional labels include, for example, radioisotopes (such as 3H), metal chelates such as DOTA and DPTA chelates of radioactive or paramagnetic metal ions like Gd3+, and liposomes.

Detectable labels that can be used with nucleic acid molecules (such as the disclosed probes) also include enzymes, for example horseradish peroxidase (HRP), alkaline phosphatase (AP), acid phosphatase, glucose oxidase, β-galactosidase, β-glucuronidase, or β-lactamase. Where the detectable label includes an enzyme, a chromogen, fluorogenic compound, or luminogenic compound can be used in combination with the enzyme to generate a detectable signal (numerous of such compounds are commercially available, for example, from Life Technologies). Particular examples of chromogenic compounds include diaminobenzidine (DAB), 4-nitrophenylphosphate (pNPP), fast red, fast blue, bromochloroindolyl phosphate (BCIP), nitro blue tetrazolium (NBT), BCIP/NBT, AP Orange, AP blue, tetramethylbenzidine (TMB), 2,2'-azino-di-[3-ethylbenzothiazoline sulphonate] (ABTS), o-dianisidine, 4-chloronaphthol (4-CN), nitrophenyl-β-D-galactopyranoside (ONPG), o-phenylenediamine (OPD), 5-bromo-4-chloro-3-indolyl-β-galactopyranoside (X-Gal), methylumbelliferyl-β-D-galactopyranoside (MU-Gal), p-nitrophenyl-α-D-galactopyranoside (PNP), 5-bromo-4-chloro-3-indolyl-β-D-glucuronide (X-Gluc), 3-amino-9-ethyl carbazol (AEC), fuchsin, iodonitrotetrazolium (INT), tetrazolium blue, and tetrazolium violet.

Alternatively, an enzyme can be used in a metallographic detection scheme. For example, silver *in situ* hybridization (SISH) procedures involve metallographic detection schemes for identification and localization of a hybridized genomic target nucleic acid sequence. Metallographic detection methods include using an enzyme, such as alkaline phosphatase, in combination with a water-soluble metal ion and a redox-inactive substrate of the enzyme. The substrate is converted to a redox-active agent by the enzyme, and the redox-active agent reduces the metal ion, causing it to form a detectable precipitate. (See, for example, U.S. Patent No. 7,632,652,). Metallographic detection methods also include using an oxido-reductase enzyme (such as horseradish peroxidase) along with a water soluble metal ion, an oxidizing agent and a reducing agent, again to form a detectable precipitate. (See, for example, U.S. Patent No. 6,670,113).

In non-limiting examples, the disclosed nucleic acid probes are labeled with dNTPs covalently attached to hapten molecules (such as a nitro-aromatic compound (e.g., 2,4-dinitrophenyl (DNP)), biotin, fluorescein, digoxigenin (DIG), etc.). Additional haptens suitable for labeling the disclosed probes include nitropyrazole, 3-hydroxyquinoxaline, thiazolesulfonamide, nitrocinnamic acid, rotenone, 7-(diethylamino)coumarin-3-carboxylic acid, benzodiazepine, or benzofuran haptens (see, e.g., International Pat. Publ. No. WO 2012/003476). Methods for conjugating haptens and other labels to dNTPs (e.g., to facilitate incorporation into labeled probes) are well known in the art. For examples of procedures, see, e.g., U.S. Patent Nos. 5,258,507, 4,772,691, 5,328,824, and 4,711,955. Indeed, numerous labeled dNTPs are available commercially, for example from Life Technologies (Carlsbad, CA). A label can be directly or indirectly attached to a dNTP at any location on the dNTP, such as a phosphate (e.g., α, β or γ phosphate) or a sugar.

Detection of labeled nucleic acid molecules can be accomplished by contacting the hapten-labeled nucleic acid molecules bound to the genomic target nucleic acid with a primary anti-hapten antibody. In one example, the primary anti-hapten antibody (such as a mouse anti-hapten antibody) is directly labeled with an enzyme. In another example, a secondary anti-species antibody (such as a goat anti-mouse IgG antibody) conjugated to an enzyme is used for signal amplification. In chromogenic *in situ* hybridization CISH a chromogenic substrate is added, for SISH, silver ions and other reagents as outlined in the referenced patents/applications are added.

In some examples, a probe is labeled by incorporating one or more labeled dNTPs using an enzymatic (polymerization) reaction. For example, the disclosed nucleic acid probes (for example, incorporated into a plasmid vector) can be labeled by nick translation (using, for example, biotin, DNP, digoxigenin, etc.) or by random primer extension with terminal transferase (e.g., 3' end tailing). In some examples, the nucleic probe is labeled by a modified nick translation reaction where the ratio of DNA polymerase I to deoxyribonuclease I (DNase I) is modified to produce greater than 100% of the starting material. In particular examples, the nick translation reaction includes DNA polymerase I to DNase I at a ratio of at least about 800:1, such as at least 2000:1, at least 4000:1, at least 8000:1, at least 10,000:1, at least 12,000:1, at least 16,000:1, such as about 800:1 to 24,000:1 and the reaction is carried out overnight (for example, for about 16-22 hours) at a substantially isothermal temperature, for example, at about 16°C to 25°C (such as room temperature). If the probe is included in a probe set (for example, multiple plasmids, such as 2, 3, 4, 5, 6, 7, 8, 9, 10, or more plasmids), the plasmids may be mixed in an equal molar ratio prior to performing the labeling reaction (such as nick translation or modified nick translation).

In other examples, chemical labeling procedures can also be employed. Numerous reagents (including hapten, fluorophore, and other labeled nucleotides) and other kits are commercially available for enzymatic labeling of nucleic acids, including the disclosed nucleic acid probes. As will be apparent to those of skill in the art, any of the labels and detection procedures disclosed above are applicable in the context of labeling a probe, e.g., for use in *in situ* hybridization reactions. For example, the Amersham MULTIPRIME® DNA labeling system, various specific reagents and kits available from Molecular Probes/Life Technologies, or any other similar reagents or kits can be used to label the nucleic acids disclosed herein. In particular examples, the disclosed probes can be directly or indirectly labeled with a hapten, a ligand, a fluorescent moiety (e.g., a fluorophore or a semiconductor nanocrystal), a chromogenic moiety, or a radioisotope. For example, for indirect labeling, the label can be attached to nucleic acid molecules via a linker (e.g., PEG or biotin). Additional methods that can be used to label probe nucleic acid molecules are provided in U.S. Pat. No. 7,541,455.

### V. Methods for in situ Hybridization for Chromosome Enumeration

The present invention also features *in situ* hybridization (ISH) assays, e.g., bright-field ISH assays, for detection of a gene target and a chromosome (e.g., centromere target of a chromosome) using single-strand oligonucleotide probes. For example, a method comprises contacting a tissue sample with a control probe specific to a control region of a chromosome (e.g., chromosome 3), wherein the control probe is a single-stranded oligonucleotide probe labeled with at least one first label. The control probe may be configured to achieve a staining intensity of ≥2 and staining coverage of ≥50% of nuclei within 3 hours when applied to a control sample. The method may further comprise hybridizing the control probe to the control region under conditions for a period of time less than about 3 hours (e.g., ≤ about 2.5 hours, ≤ about 2 hours, ≤ about 1.5 hour, or ≤ about 1 hour), rinsing the sample to remove unbound probe, and detecting the presence of the hybridized probe.

The method may further comprise contacting the tissue sample with a target probe specific to a target region (e.g., PIK3CA) of the chromosome, wherein the target probe is a single-stranded oligonucleotide probe labeled with at least one second label.

In some examples, the method further comprises applying chromogenic detection reagents that recognize the first label and amplify the signal associated with said first label. The method may feature the use of one or more probes (e.g., SEQ ID NOs: 1-18) or systems as described herein.

Genome-specific blocking DNA (such as human DNA, for example, total human placental DNA or Cot-1™ DNA) is usually included in a hybridization solution (such as for *in situ* hybridization) to suppress probe hybridization to repetitive DNA sequences or to counteract probe hybridization to highly homologous (frequently identical) off target sequences when a probe complementary to a human genomic target nucleic acid is utilized. In hybridization with standard probes, in the absence of genome-specific blocking DNA, an unacceptably high level of background staining (for example, non-specific binding, such as hybridization to non-target nucleic acid sequence) is usually present, even when a "repeat-free" probe is used. The disclosed nucleic acid probes exhibit reduced background staining, even in the absence of blocking DNA. In particular examples, the hybridization solution including the disclosed probes does not include genome-specific blocking DNA (for example, total human placental DNA or Cot-1™ DNA, if the probe is complementary to a human genomic target nucleic acid). This advantage is derived from the uniquely specific nature of the target sequences included in the nucleic acid probe; each labeled probe sequence binds only to the cognate uniquely specific genomic sequence. This results in dramatic increases in signal to noise ratios for ISH techniques.

As such, some methods herein may be free from the use of blocking DNA. However, in some examples, blocking DNA may be used. In some examples, an amount of blocking DNA is used but the amount of blocking DNA is sufficient to block out no more than a specified percent of the non-specific binding, e.g., no more than 50%, 40%, 30%, 20%, or 10%.

In order to determine an amount of blocking DNA that is sufficient to block out no more than a specified percent (e.g., 50%) of the non-specific binding, the following tests may be conducted. Set up an *in situ* hybridization assay, contact a tissue sample with a double strand (e.g. DNA) control probe specific to a control region of a chromosome (in combination with zero to a serially, gradually increasing amount of blocking DNA); hybridize the double strand control probe to the control region; rinse the sample to remove unbound double strand probe; and detect the presence of the hybridized probe. Then observe the amount of background that is blocked by the serially increasing blocking DNA in each assay. The amount of blocking DNA that achieves a specified percent of the blocking of the background corresponds to the amount of blocking DNA that is sufficient to block out no more than a specified percent (e.g., 50%) of the non-specific binding. For example, the amount of blocking DNA that achieves blocking out 50% of percent of the background corresponds to the amount of blocking DNA that is sufficient to block out no more than 50% of the non-specific binding.

In some examples, said amount of blocking DNA is between about 1 pg/ml to 1 mg/ml. In some examples, said amount of blocking DNA is between about 1 pg/ml to 0.5 mg/ml. In some examples, said amount of blocking DNA is between about 1 pg/ml to 0.25 mg/ml. In some examples, said amount of blocking DNA is between about 1 pg/ml to 1 µg/ml.

In some illustrative examples, methods for obtaining two bright-field chromogenic *in situ* hybridization signals per cell may comprise contacting a tissue sample containing a plurality of cells with a control probe specific to a control region of a single chromosome, the probe selected so as to not evidently bind non-specifically in the absence of blocking DNA; hybridizing the control probe to the control region of said chromosome; rinsing the sample to remove unbound probe; and detecting the presence of the hybridized probe via a chromogenic reagent so as to generate two bright-field chromogenic *in situ* hybridization signals per cell. In order to determine that the selected probe does not evidently bind non-specifically in the absence of blocking DNA, a comparative assay (Assay 2) may be conducted along side with the aforementioned assay (Assay 1), wherein the same selected probe is employed in both Assay 1 and Assay 2. Assay 1 is free of the blocking DNA and Assay 2 employs a blocking DNA. Then the respective data of the two assays are compared. The selected probe does not evidently bind non-specifically in the absence of blocking DNA when the data of the two respective assays are the same or substantially the same.

In some examples the hybridization solution may contain carrier DNA from a different organism (for example, salmon sperm DNA or herring sperm DNA, if the genomic target nucleic acid is a human genomic target nucleic acid) to reduce non-specific binding of the probe to non-DNA materials (for example to reaction vessels or slides) with high net positive charge which can non-specifically bind to the negatively charged probe DNA.

Methods of the present invention may comprise detecting signals wherein more than 50% of the nuclei of the tissue sample have enumerable signals for said chromosome, wherein an enumerable signal is a generally round shape (e.g., as described above). In some examples, background signals are not observed in > 70% of cells of the tissue sample. In some examples, background signals are not observed in > 80% of cells of the tissue sample. In some examples, background signals are not observed in > 90% of cells of the tissue sample. In some examples, background signals are present but are sufficiently weak in intensity so as to permit identification of enumerable signals in > 50% of the nuclei.

In some examples, more than 60% of the nuclei have enumerable chromosome signals. In some examples, more than 70% of the nuclei have enumerable chromosome signals. In some examples, the inner radius is no less than 60% of the outer radius. In some examples, the inner radius is no less than 75% of the outer radius. In some examples, the inner radius is no less than 90% of the outer radius.

*In situ* hybridization (ISH) involves contacting a sample containing a target nucleic acid (e.g., a genomic target nucleic acid) in the context of a metaphase or interphase chromosome preparation (such as a cell or tissue sample mounted on a slide) with a labeled probe specifically hybridizable or specific for the target nucleic acid (for example, one or more of the probes disclosed herein). The slides are optionally pretreated, e.g., to remove paraffin or other materials that can interfere with uniform hybridization. The chromosome sample and the probe are both treated, for example by heating to denature the double stranded nucleic acids. The probe (formulated in a suitable hybridization buffer) and the sample are combined, under conditions and for sufficient time to permit hybridization to occur (typically to reach equilibrium). The chromosome preparation is washed to remove excess probe, and detection of specific labeling of the target is performed using standard techniques.

For example, a biotinylated probe can be detected using fluorescein-labeled avidin or avidin-alkaline phosphatase. For fluorochrome detection, the fluorochrome can be detected directly, or the samples can be incubated, for example, with fluorescein isothiocyanate (FITC)-conjugated avidin. Amplification of the FITC signal can be effected, if necessary, by incubation with biotin-conjugated goat anti-avidin antibodies, washing and a second incubation with FITC-conjugated avidin. For detection by enzyme activity, samples can be incubated, for example, with streptavidin, washed, incubated with biotin-conjugated alkaline phosphatase, washed again and pre-equilibrated (e.g., in alkaline phosphatase (AP) buffer). The enzyme reaction can be performed in, for example, AP buffer containing NBT/BCIP and stopped by incubation in 2 X SSC. For a general description of *in situ* hybridization procedures, see, e.g., U.S. Patent No. 4,888,278.

Numerous procedures for FISH, CISH, and SISH are known in the art. For example, procedures for performing FISH are described in U.S. Patent Nos. 5,447,841; 5,472,842; and 5,427,932 CISH is described in U.S. Patent No. 6,942,970, and additional detection methods are provided in U.S. Patent No. 6,280,929.

Numerous reagents and detection schemes can be employed in conjunction with FISH, CISH, and SISH procedures to improve sensitivity, resolution, or other desirable properties. As discussed above, probes labeled with fluorophores (including fluorescent dyes and quantum dots) can be directly optically detected when performing FISH. Alternatively, the probe can be labeled with a non-fluorescent molecule, such as a hapten (such as the following non-limiting examples: biotin, digoxigenin, DNP, and various oxazoles, pyrrazoles, thiazoles, nitroaryls, benzofurazans, triterpenes, ureas, thioureas, rotenones, coumarin, courmarin-based compounds, Podophyllotoxin, Podophyllotoxin-based compounds, and combinations thereof), ligand or other indirectly detectable moiety. Probes labeled with such non-fluorescent molecules (and the target nucleic acid sequences to which they bind) can then be detected by contacting the sample (e.g., the cell or tissue sample to which the probe is bound) with a labeled detection reagent, such as an antibody (or receptor, or other specific binding partner) specific for the chosen hapten or ligand. The detection reagent can be labeled with a fluorophore (e.g., quantum dot) or with another indirectly detectable moiety, or can be contacted with one or more additional specific binding agents (e.g., secondary or specific antibodies), which can in turn be labeled with a fluorophore. Optionally, the detectable label is attached directly to the antibody, receptor (or other specific binding agent).

Alternatively, the detectable label is attached to the binding agent via a linker, such as a hydrazide thiol linker, a polyethylene glycol linker, or any other flexible attachment moiety with comparable reactivities. For example, a specific binding agent, such as an antibody, a receptor (or other anti-ligand), avidin, or the like can be covalently modified with a fluorophore (or other label) via a heterobifunctional polyalkyleneglycol linker such as a heterobifunctional polyethyleneglycol (PEG) linker. A heterobifunctional linker combines two different reactive groups selected, e.g., from a carbonyl-reactive group, an amine-reactive group, a thiol-reactive group and a photo-reactive group, the first of which attaches to the label and the second of which attaches to the specific binding agent.

In other examples, the probe, or specific binding agent (such as an antibody, e.g., a primary antibody, receptor or other binding agent) is labeled with an enzyme that is capable of converting a fluorogenic or chromogenic composition into a detectable fluorescent, colored or otherwise detectable signal (e.g., as in deposition of detectable metal particles in SISH). As indicated above, the enzyme can be attached directly or indirectly via a linker to the relevant probe or detection reagent. Examples of suitable reagents (e.g., binding reagents) and chemistries (e.g., linker and attachment chemistries) are described in U.S. Patent Application Publication Nos. 2006/0246524; 2006/0246523, and 2007/0117153.

In further examples, a signal amplification method is utilized, for example, to increase sensitivity of the probe. For example, tyramide signal amplification may be utilized (See U.S. Pat. No. 5,196,306). In one variation of this method a biotinylated nucleic acid probe detects the presence of a target by binding thereto. Next a streptavidin-peroxidase conjugate is added. The streptavidin binds to the biotin. A substrate of biotinylated tyramide (tyramine is 4-(2- aminoethyl)phenol) is used, which presumably becomes a free radical when interacting with the peroxidase enzyme. The phenolic radical then reacts quickly with the surrounding material, thus depositing or fixing biotin in the vicinity. This process is repeated by providing more substrate (biotinylated tyramide) and building up more localized biotin. Finally, the "amplified" biotin deposit is detected with streptavidin attached to a fluorescent molecule. Alternatively, the amplified biotin deposit can be detected with avidin-peroxidase complex, that is then fed 3,3'-diaminobenzidine to produce a brown color. It has been found that tyramide attached to fluorescent molecules also serve as substrates for the enzyme, thus simplifying the procedure by eliminating steps. Yet another amplification approach is described in U.S. Patent Publ. No. 2013/0260379.

In other examples, the signal amplification method utilizes branched DNA (bDNA) signal amplification. In some examples, target-specific oligonucleotides (label extenders and capture extenders) are hybridized with high stringency to the target nucleic acid. Capture extenders are designed to hybridize to the target and to capture probes, which are attached to a microwell plate. Label extenders are designed to hybridize to contiguous regions on the target and to provide sequences for hybridization of a preamplifier oligonucleotide. Signal amplification then begins with preamplifier probes hybridizing to label extenders. The preamplifier forms a stable hybrid only if it hybridizes to two adjacent label extenders. Other regions on the preamplifier are designed to hybridize to multiple bDNA amplifier molecules that create a branched structure. Finally, alkaline phosphatase (AP)-labeled oligonucleotides, which are complementary to bDNA amplifier sequences, bind to the bDNA molecule by hybridization. The bDNA signal is the chemiluminescent product of the AP reaction See, e.g., Tsongalis, Microbiol. Inf. Dis. 126:448-453, 2006; U.S. Pat. No. 7,033,758.

In further examples, the signal amplification method utilizes polymerized antibodies. In some examples, the labeled probe is detected by using a primary antibody to the label (such as an anti-DIG or anti-DNP antibody). The primary antibody is detected by a polymerized secondary antibody (such as a polymerized HRP-conjugated secondary antibody or an AP-conjugated secondary antibody). The enzymatic reaction of AP or HRP leads to the formation of strong signals that can be visualized.

It will be appreciated by those of skill in the art that by appropriately selecting labeled probe-specific binding agent pairs, multiplex detection schemes can be produced to facilitate detection of multiple target nucleic acids (e.g., genomic target nucleic acids) in a single assay (e.g., on a single cell or tissue sample or on more than one cell or tissue sample). For example, a first probe that corresponds to a first target nucleic acid can be labeled with a first hapten, such as biotin, while a second probe that corresponds to a second target nucleic acid can be labeled with a second hapten, such as DNP. Following exposure of the sample to the probes, the bound probes can be detected by contacting the sample with a first specific binding agent (in this case avidin labeled with a first fluorophore, for example, a first spectrally distinct quantum dot, e.g., that emits at 585 nm) and a second specific binding agent (in this case an anti-DNP antibody, or antibody fragment, labeled with a second fluorophore (for example, a second spectrally distinct quantum dot, e.g., that emits at 705 nm). Additional probes/binding agent pairs can be added to the multiplex detection scheme using other spectrally distinct fluorophores. Numerous variations of direct, and indirect (one step, two step or more) can be envisioned, all of which are suitable in the context of the disclosed probes and assays.

Additional details regarding certain detection methods, e.g., as utilized in CISH and SISH procedures, can be found in Bourne, The Handbook of Immunoperoxidase Staining Methods, published by Dako Corporation, Santa Barbara, CA.

Difficulties frequently encountered in ISH testing may result from the manner in which the tissues are typically preserved. The mainstay of the diagnostic pathology laboratory has been for many decades the formalin-fixed, paraffin-embedded block of tissue, sectioned and mounted upon glass slides. Fixation in such a preservative causes cross-linking of macromolecules, both amino acids and nucleic acids. These cross-linked components must be removed to allow access of the probe to the target nucleic acid and to allow the antibody to recognize the corresponding antigen. "Unmasking" the antigen and/or nucleic acid is typically accomplished manually with multiple pretreatment, proteolytic digestion, and wash steps. Prior to or staining, complete removal of the paraffin is also required so that it does not interfere with antibody or probe binding. Deparaffinization may be achieved by the use of multiple (e.g., two or three) successive clearing reagents that are paraffin solvents (e.g., xylene, xylene substitutes, or toluene).

In some examples, preparing the sample includes the step of cell conditioning. Cell conditioning is discussed in greater detail in U.S. Patent 6,855,552, Towne, et al. "Automated immunohistochemical and *in situ* hybridization assay formulations". In illustrative cell conditioning steps, a cell conditioning reagent is applied and the sample is contacted at the appropriate temperature for an appropriate duration of time so that the antigens and/or nucleic acid targets are sufficiently expressed for detection. One aspect of the present disclosure is that the automated instrument can automatically adjust the cell conditioning duration and/or temperature in response to the user inputs. Cell conditioning may further include applying a protease reagent. Illustratively, a protease treatment may involve the step of contacting a protease solution to a biological sample. The protease treatment, as with cell conditioning, is intended to increase the expression of target antigens and/or nucleic acids.

Cell conditioning reagents such as ethylenediaminetetraacetic acid (EDTA) for nucleic acid targets (ISH) may be used. The contacting may be done at a temperature of about 95° C for between about 2 and about 90 minutes. A partial list of possible reagents appears in Analytical Morphology, Gu, ed., Eaton Publishing Co. (1997) at pp. 1-40. Sodium dodecyl sulfate (SDS) and/or ethylene glycol may be included in the conditioning solution. Furthermore, metal ions or other materials may be added to these reagents to increase effectiveness of the cell conditioning. Exemplary cell conditioning solutions are available from Ventana Medical Systems, Inc., Tucson, AZ (Cell Conditioning 1 (CC1) catalog #: 950-124; Cell Conditioning 2 (CC2) catalog #: 950-123; SSC (10X) catalog #: 950-110; ULTRA Cell Conditioning (ULTRA CC1) catalog #: 950-224; ULTRA Cell Conditioning (ULTRA CC2) catalog #: 950-223, Protease 1 catalog #: 760-2018; Protease 2 catalog #: 760-2019; Protease 3 catalog #: 760-2020). In some examples, applying the *in situ* hybridization binding reagent occurs subsequent to applying the cell conditioning reagent and prior to applying the chromogenic reagent.

In illustrative examples, the method includes applying a rinsing reagent. Between various steps described herein and as part of the system described herein, rinse steps may be added to remove unreacted residual reagents from the prior step. Rinse steps may further include incubations, which include maintaining a rinsing reagent on the sample for a pre-determined time at a pre-determined temperature with or without mixing. The conditions appropriate for the rinsing steps may be distinct between the various steps. Exemplary rinsing reagents are available from Ventana Medical Systems, Inc., Tucson, AZ (Reaction Buffer (10x) catalog #: 950-300; Special Stains Wash (10x) catalog #: 860-015).

Exemplary automated systems available through Ventana Medical Systems, Inc., Tucson, AZ include SYMPHONY® Staining System, catalog #: 900-SYM3, VENTANA® BenchMark Automated Slide Preparation Systems, catalog #s: N750-BMKXT-FS, N750-BMKU-FS, VENTANA, and VENTANA® BenchMark Special Stains automated slide stainer. These systems employ a microprocessor controlled system including a revolving carousel supporting radially positioned slides. A stepper motor rotates the carousel placing each slide under one of a series of reagent dispensers positioned above the slides. Bar codes on the slides and reagent dispensers permits the computer controlled positioning of the dispensers and slides so that different reagent treatments can be performed for each of the various tissue samples by appropriate programming of the computer.

While EXAMPLE 1 below describes a single-stranded oligonucleotide-based PIK3CA/CHR3 dual ISH assay, it is understood that those of ordinary skill in the art could apply the discoveries disclosed herein to other gene/centromere combination of interest.

In some embodiments, the disclosed systems (e.g., probes) can be used in methods of determining the copy number of a target nucleic acid (such as PIK3CA) in a biological sample (such as a tissue sample). Methods of determining the copy number of a gene or chromosomal region are well known to those of skill in the art. In some examples, the methods include *in situ* hybridization (such as fluorescent, chromogenic, or silver *in situ* hybridization), comparative genomic hybridization, or polymerase chain reaction (such as real-time quantitative PCR). In some examples, methods of determining gene copy number include counting the number of ISH signals (such as fluorescent, colored, or silver spots) for the target nucleic acid in one or more individual cells. The methods may also include counting the number of ISH signals (such as fluorescent, colored, or silver spots) for a reference (such as a chromosome-specific probe) in the cells. In particular examples, the number of copies of the gene (or chromosome) may be estimated by the person (or computer, in the case of an automated method) scoring the slide. In some examples, an increased copy number relative to a control (such as an increase of about 1.5-fold, 2-fold, 3-fold, 5-fold, 10-fold, 20-fold, or more relative to a control sample or reference value) indicates an increase in the target nucleic acid copy number.

In some examples, the method includes counting the number of copies per cell or nucleus of a reference, such as a chromosomal locus known not to be abnormal, for example a centromere. In some examples, the reference is on the same chromosome as the gene of interest. Exemplary reference chromosomes that can be used for particular human genes of interest are provided in Table 5. In particular examples, the reference locus is detected by using a centromere-specific probe. Such probes are known in the art and are commercially available, for example, Vysis CEP probes (Abbott Molecular, Des Plaines, IL) and SPOTLIGHT centromeric probes (Invitrogen, Carlsbad, CA). In some examples, a ratio of target nucleic acid copy number to reference copy number greater than about two (such as greater than about 2, 3, 4, 5, 10, 20, or more) indicates an increase in the target nucleic acid copy number.

**TABLE 5. EXEMPLARY REFERENCE CHROMOSOMES FOR PARTICULAR TARGET NUCLEIC ACIDS**

| **Target Nucleic Acid** | **Reference Chromosome** |
|---|---|
| PTEN | 10 |
| PIK3CA | 3 |
| TOP2A | 17 |
| MET | 7 |
| MDM2 | 12 |

### VI. Methods of Scoring

The present invention also features methods of scoring gene copy number of a target region and optionally comparing it to the copy number of a control region. For additional methods of scoring, which may be used with the methods described herein, reference is made to U.S. Publ. Appl. No. 2012/0141472 for disclosure related to scoring ISH.

In some examples, an increased gene copy number includes the gene copy number per nucleus (such as average gene copy number per nucleus) in the sample of greater than about two copies of the gene per nucleus (such as greater than 2, 3, 4, 5, 10, or 20 copies). In other examples, an increased gene copy number includes a ratio of gene copy number to its corresponding chromosome copy number (such as an average gene:chromosome ratio) in the sample of greater than about 2 (such as a ratio of greater than 2, 3, 4, 5, 10, or 20). In further examples, an increased gene copy number includes an increase in gene copy number relative to a control (such as an increase of about 1.5-fold, about 2-fold, about 3-fold, about 5-fold, about 10-fold, about 20-fold, or more). Therefore, in some examples, the method includes comparing the gene copy number in the sample from the subject to the gene copy number in a control or a reference value or range of values expected for the gene copy number in an appropriate normal tissue.

Also disclosed herein is a method of scoring (for example, enumerating) copy number of a gene in a sample from a subject, wherein the sample is stained by ISH (such as FISH, SISH, CISH, or a combination of two or more thereof) for the gene of interest and wherein individual copies of the gene are distinguishable in cells in the sample. In particular examples, the sample is a biological sample from a subject, such as a tumor sample (for example, a tumor biopsy). Methods of determining gene copy number by ISH are well known in the art.

In some examples, the method includes identifying individual cells in a sample with the highest number of signals per nucleus for the gene (such as the strongest signal in the sample), counting the number of signals for the gene in the identified cells, and determining an average number of signals per cell, thereby scoring the gene copy number in the sample. In additional examples, the method further includes counting the number of signals for a reference (such as a chromosomal locus known not to be abnormal, for example, centromeric DNA) and determining an average ratio of the number of signals for the gene to the number of signals for the reference per cell.

The scoring method may include identifying individual cells in the sample (such as a tissue section or tumor core) having the highest number of signals (such as the highest number of spots per cell or the brightest intensity of staining) for the gene of interest in the cells in the sample. Thus, the disclosed method may not determine gene copy number in a random sampling of cells in the sample. Rather, the method may include specifically counting gene copy number in those cells that have the highest gene copy number in the sample. In some examples, identifying the individual cells having the highest number of signals for the gene includes examining a sample stained by ISH for the gene under low power microscopy (such as about 20>< magnification). Cells with the strongest signal (for example, highest amplification signal under higher power) are identified for counting by eye or by an automated imaging system. In some examples, such as when the sample is a tissue section, the sample is examined (for example, visually scanned) to identify a region that has a concentration of tumor cells that has amplification of the gene. Gene copy number in the cells with highest amplification in the selected region is then counted. In other examples, such as when the sample is a tumor core (such as a tumor microarray), most of the sample is visible in the field of view under low power magnification and the individual cells (such as tumor cells) with the strongest signal (for example, highest amplification signal under high power) are separately identified for counting. In particular examples, the cells chosen for counting the gene copy number may be non-consecutive cells, such as cells that are not adjacent to or in contact with one another. In other examples, at least some of the cells chosen for counting the gene copy number may be consecutive cells, such as cells that are adjacent to or in contact with one another.

The disclosed methods may include counting the number of ISH signals (such as fluorescent, colored, or silver spots) for the gene in the identified cells. The methods may also include counting the number of ISH signals (such as fluorescent, colored or silver spots) for a reference (such as a chromosome-specific probe) in the identified cells. In some examples, the number of spots per cells is distinguishable in the identified cells and the number of spots are counted (or enumerated) and recorded. In other examples, one or more of the identified cells may include a cluster, which is the presence of multiple overlapping signals in a nucleus that cannot be counted (or enumerated). In particular examples, the number of copies of the gene (or chromosome) may be estimated by the person (or computer, in the case of an automated method) scoring the slide. For example, one of skill in the art of pathology may estimate that a cluster contains a particular number of copies of a gene (such as 10, 20, or more copies) based on experience in enumerating gene copy number in a sample. In other examples, the presence of a cluster may be noted as a cluster, without estimating the number of copies present in the cluster.

The number of cells identified for counting is a sufficient number of cells that provides for detecting a change (such as an increase or decrease) in gene copy number. In some examples, the number of cells identified for counting is at least about 20, for example, at least 25, 30, 40, 50, 75, 100, 200, 500, 1000 cells, or more. In a particular example, about 50 cells are counted. In other examples, every cell in the sample or every cell in a microscope field of vision, or in a number of microscope fields (such as at least 2 microscope fields, at least 3, at least 4, at least 5, at least 6 microscope fields, and the like) which contains 3 or more copies of the gene of interest (such as 3, 4, 5, 6, 7, 8, 9, 10, 15, 20, or more) is counted.

Methods may feature obtaining a sample having undergone ISH according to methods disclosed herein. An area of neoplastic nuclei with the most copy numbers is identified and the enumerable signals for the chromosome/target are counted in 50-100 neoplastic nuclei and either 50 adjacent mesenchymal nuclei or 50 adjacent normal epithelial nuclei.

Scoring criteria may be as follows: no staining or <1 dot/ 10 cells is scored as 0; 1-3 dots/cell is scored as 1; 4-9 dots/cell, none or very few dot clusters is scored as 2; 10-15 dots/cell and <10% dots are in clusters is scored as 3; and >15 dots/cell and >10% dots are in clusters is scored as 4.

In some embodiments, the average number of target signals (e.g., PIK3CA) per nuclei is calculated. In some examples, the average number of chromosome (e.g., CHR3) copies per nuclei is calculated. In some embodiments, the target signal to chromosome signal ratio is calculated.

The disclosure is further illustrated by the following non-limiting Examples.

### EXAMPLES

### Example 1 - Automated Bright-field PIK3CA Copy Number Enumeration with Oligo Dual in situ Hybridization and Its Correlation with Overexpression of Phosphatidylinositol 3 Kinase on Human Lung Tumors

This example describes the use of CHR3 probes and PIK3CA probes for ISH assays. The example also describes the use of said probes for copy number enumeration. One skilled in the art will appreciate that methods that deviate from these specific methods can also be used to successfully detect a target nucleic acid and/or chromosome.

### MATERIALS AND METHODS

### Specimens

Individual lung tissue samples obtained from a tissue specimen archive maintained at Ventana Medical Systems, Inc. (Tucson, AZ) were used for developing and optimizing PIK3CA dual ISH (DISH), mRNA ISH, and p110α IHC assays. These samples were redundant clinical specimens that had been de-identified and unlinked from patient information. A tissue microarray slide containing 102 lung tissues was obtained from Pantomics Inc. (Richmond, CA, USA). Paraffin sections (4 µm) containing tissue cores of formalin-fixed, paraffin-embedded breast tissue were mounted on Superfrost® Plus glass slides.

### PIK3CA and Chromosome 3 oligo probes

The single-stranded oligonucleotide PIK3CA probe (PIK3CA oligo probe) is a DNP-labeled, repeat-free genomic probe that specifically targets the PIK3CA gene region. The PIK3CA oligo probe spans 759,736 nucleotides (nt) (178,640,071 - 179,399,807) of genomic DNA from human Chromosome 3, encompassing the PIK3CA target region (UCSC Genome Browser on GRCh37/hg19 human genome Assembly) (FIG. 1). A bioinformatic search was used to identify PIK3CA specific nucleic acid sequences around the PIK3CA target region. The selected genomic target nucleic acid sequence was separated into consecutive non-overlapping 80 nt segments. One thousand two hundred and twelve (1212) ∼80mer oligonucleotides each carrying 5 DNP haptens were synthesized. The oligonucleotides were purified and verified with Mass Spec and gel electrophoresis. The single-stranded oligonucleotide Chr3 probe (Chr3 oligo probe) is a pool of 18 oligonucleotides with a length as specified in Table 4. Each oligonucleotide was labeled with 2 DIG haptens; the oligonucleotides were PAGE purified and their molecular weights were verified by mass spectrometer. The PIK3CA oligo probe (5.0 ug/ml) and the Chr3 oligo probe (0.75 ug/ml) were combined in a formamide-containing buffer without human blocking DNA.

### Automated bright-field PIK3CA/CHR3 Oligo Dual ISH for interphase slides

The BenchMark ULTRA automated slide processing system (Ventana Medical Systems, Inc., Tucson, Arizona, USA) was used for designing and evaluating the performance of the single-stranded oligonucleotide PIK3CA and CHR3 dual ISH assays (PIK3CA/CHR3 Oligo DISH) for PIK3CA and CHR3 DNA targets. The ultraView SISH and ultraView Alkaline Phosphatase Red ISH detection kits (Ventana) were used for silver (PIK3CA) and red (CHR3) detection. The slides were deparaffinized at 69°C, followed by incubation with pH 6 citrate buffer at 82°C and by digestion with ISH Protease 3 for 20 minutes. The probe mixture was then deposited onto the slide so as to contact the sample. The probe(s) were heated to 80°C for 8 minutes to ensure the probes were denatured. Subsequently, conditions suitable for hybridizing the probes were maintained for 1 hour (e.g. temperature was lowered to 44°C and maintained). The unbound probes were washed from the sample using 3 stringency washes (pH 6.0 citrate buffer at 72°C). The presence of the probes on the sample was then detected by contacting the sample with a horseradish peroxidase-labeled rabbit anti-DNP antibody. Following a rinsing step to remove the unbound antibody-enzyme conjugate, the ultraView SISH reagent was contacted with the sample to cause silver metal precipitate to form. The specific hybridization of the DNP-linked PIK3CA probe to its target was detected by visualizing the insoluble precipitate of silver chromogen. The ultraView Alakaline Phosphatase reagents were then contacted with the sample according to its normal usage, first contacting the sample with an alkaline phosphatase (AP)-labeled mouse anti-DIG antibody, followed by rinsing and contacting the sample with the Fast Red chromogen system. The Chromosome 3 probe was detected by visualizing the soluble precipitate of the AP-based Fast Red chromogenic system. The slides were counterstained by contacting the samples with hematoxylin for 4 minutes and post-counterstained with bluing reagent for 4 minutes so that the morphology of the tissue could be better visualized.

### Automated bright-field PIK3CA/CHR3 Oligo DISH chromosome metaphase spread staining:

Metaphase chromosomes (CGH Metaphase Target Slides, Abbott Molecular) were UV cross-linked on Stratalinker 2400 (Stratagene Model # C00518) at energy level 200 mJ. They were then treated with 1% trypsin (Sigma cat#T1426) at room temperature for 5 seconds. The slides were then processed for PIK3CA/CHR3 Oligo DISH staining under the same conditions as described above except that the steps for baking, deparaffin, cell conditioning and counterstaining were omitted. After ISH staining was completed on the instrument, slides were stained with 4% Giemsa (Gibco, cat#10092-03) diluted in Gurr buffer (Gibco, cat#10582-013) at room temperature for 5 min, and staining was visualized with a regular light microscope.

### Bright-field PIK3CA mRNA ISH

Homo sapiens phosphatidylinositol-4,5-bisphosphate 3-kinase, catalytic subunit alpha (PIK3CA), mRNA (GenBank accession number: NM_006218.2) was targeted with Hs-PIK3CA (Cat. No: 603041, Advanced Cell Diagnostics, (ACD), Hayward, CA). Thirty of double Z oligonucleotide probe pairs were designed to cover the region 420-3261 nt. The signal amplification system consists of the preamplifier, amplifier, and enzyme conjugated label probe, which assemble into a tree-like structure via sequential hybridization. To ensure specificity, only when both ZZ probes bind to the preamplifier binding site can the signal amplification occurs at the target site under given assay conditions. Hs-PPIB was used as positive control probe to target Peptidylprolyl Isomerase B (Cyclophilin B) (ACD Cat. 313901); and dapB was used as negative control probe to target bacteria B. subtilis gene dihydrodipicolinate reductase (ACD Cat. 310043). The RNA *in situ* hybridization was performed manually using RNAscope® 2.0 FFPE Reagent Kit (RNAscope® 2.0 HD Red Reagent Kit, ACD Cat. No 310036) according to the manufacturer's instructions. Briefly, FFPE tissue sections were pretreated with heat and protease prior to hybridization with the target oligo probes. Preamplifier, amplifier and AP-labeled oligos were then hybridized sequentially, followed by chromogenic precipitate development. Each sample was quality controlled for RNA integrity with the probe specific to PPIB RNA and for background with the probe specific to bacterial dapB RNA. Samples with >70% of cells stained with moderate and strong signals for PPIB were considered pass for RNA integrity. Specific RNA staining signal was identified as red, punctate dots mainly in the cytoplasm. Samples were counterstained with Gill's Hematoxylin. Signals were granular and discrete red signals corresponding to individual PIK3CA mRNA targets. Stained slides were scored semi-quantitatively using conventional bright-field microscopy

### Immunohistochemistry of p110α protein

PI3Kinase p110α (C73F8) rabbit monoclonal antibody (Cell signaling Cat # 4249) was diluted 1:50 in 50mM Tris buffer (pH 7.7) with 1% BSA and 1% normal goat serum. Staining was done on automated BenchMark®Ultra stainers. Antigen recovery was conducted using heat retrieval and CC1 standard, a high pH Tris/borate/EDTA buffer (Ventana, Cat# 950-224). The PI3Kinase p110α antibody was incubated at 37 °C for 8 min. The primary antibody was detected using OptiView DAB IHC detection kit (Ventana, Cat# 760-700). Slides were counterstained with hematoxylin II (Ventana, Cat# 790-2208) for 8 min, followed by Bluing reagent (Ventana, Cat# 760-2037) for 4 min.

### PIK3CA/CHR3 Oligo DISH Stain Scoring Criteria:

Three board-certified pathologists (W.C., J. J., and T. G.) that had no previous knowledge of the genetic, clinical and IHC results evaluated the DISH stained slides. The readers first identify an area of neoplastic nuclei with most copy numbers, then count PIK3CA and CHR3 signals in 50-100 neoplastic nuclei and 50 adjacent mesenchymal nuclei (or normal epithelial nuclei) if possible. If one area does not have enough neoplastic nuclei to count (e.g. focal amplification), pathologists may move to another area with most copy numbers.

### Bright-field PIK3CA mRNA ISH Stain Semi-quantitative Scoring Criteria

Scoring performed at 20x magnification. The scoring system is as following: 0, no staining or <1 dot/ 10 cells; 1, 1-3 dots/cell; 2, 4-9 dots/cell, none or very few dot clusters; 3. 10-15 dots/cell and <10% dots are in clusters; 4, >15 dots/cell and >10% dots are in clusters. If <5% of cells score 1 and >95% of cells score 0, a score of 0 will be given. If 5-30% of cells score 1 and >70% of cells score 0, a score of 0.5 will be given. ACD's H-Score analysis was also performed to evaluate heterogeneity in PIK3CA expression. The RNA signal is binned into 5 groups (bin 0: 0 dots/cell, bin 1: 1-3 dots/cell, bin 2: 4-9 dots/cell, bin 3: 10-15 dots/cell, and bin 4: >15 dots/cell with >10% of dots in clusters). Each sample is evaluated for the percentage of cells in each bin. The H-Score is calculated by adding up the percentage of cells in each bin, with a weight assigned to each bin, according to the formula below. H-Scores are given on a scale of 0-400. H scores = 0*(% of cells in bin 0) + 1*(% of cells in bin 1) + 2*(% of cells in bin 2) + 3*(% of cells in bin 3) + 4*(% of cells in bin 4)

### p110α IHC stain scoring criteria

By light microscopy, representative viable tissue sections were scored semiquantitatively for cytoplasmic staining. The dominant staining intensity in tumor cells was scored as: 0=negative;

1=weak; 2=intermediate; 3=strong. The level of staining in adjacent mesenchymal cells was used as baseline references. Most p110α IHC stain stains were homogeneous with more than 30% of cells showing the dominant staining intensity.

### RESULTS:

### PIK3CA/CHR3 Oligo DISH assay with 1 hr hybridization and no use of human blocking DNA: specific, robust, and superior CHR3 staining

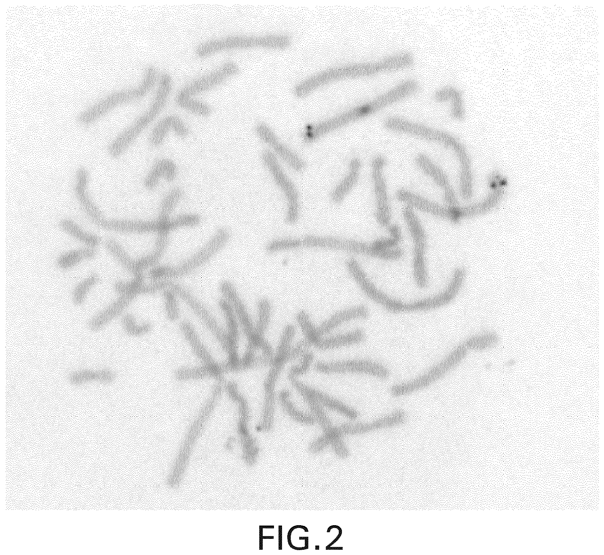

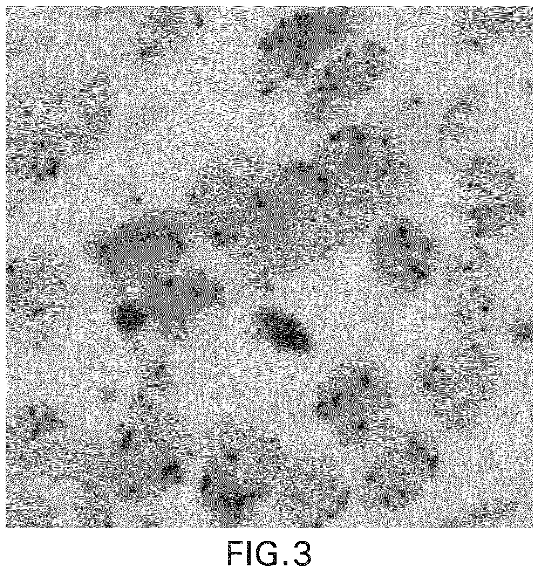

Specificity of the PIK3CA/CHR3 Oligo DISH assay was first verified on chromosomal metaphase spreads slides without human blocking DNA. The PIK3CA oligo probe (black signal) and the CHR3 oligo probe (red signal) were localized to the same chromosome. No cross-hybridization of either the PIK3CA oligo probe or the CHR3 oligo probe to other chromosomes was observed (Figure 2). The specificity of PIK3CA/CHR3 Oligo DISH assay was further assessed on 5 non-tumor lung tissues (3 normal lung and 2 tuberculosis, TB granuloma cases). The average PIK3CA copy number ranges 1.69 to 1.86 per nuclei, the average CHR3 copy number ranges 1.75 to 1.97 per nuclei, and the average PIK3CA/CHR3 ratio ranges 1.701to 1.08 per nuclei (Table 6). Furthermore, PIK3CA/CHR3 Oligo DISH assay was tested on Calu 3 cell line xenograft which has been characterized PIK3CA amplification (Spoerke, O'Brien et al. 2012) (Figure 3).

**TABLE 6 PIK3CA AND CHROMOSOME 3 COPY NUMBERS IN NORMAL AND BENIGN LUNG TISSUES**

| **Cases** | **Type** | **Nuclei Number** | **PIK3CA copies** | **Chr3 copies** | **PIK3CA/CHR3 Ratio** | **Ave PIK3CA copies** | **Ave CHR3 copies** |
|---|---|---|---|---|---|---|---|
| Case 1 | Normal | 100 | 182 | 197 | 1.02 | 1.82 | 1.97 |
| Case 2 | Normal | 100 | 186 | 192 | 1.01 | 1.86 | 1.92 |
| Case 3 | Normal | 100 | 175 | 172 | 1.08 | 1.75 | 1.72 |
| Case 4 | Tuberculosis, TB granuloma | 100 | 173 | 176 | 1.05 | 1.73 | 1.76 |
| Case 5 | Tuberculosis, TB granuloma | 100 | 169 | 175 | 1.03 | 1.69 | 1.75 |

All the aforementioned staining was performed with 1hr hybridization, a time course study was designed to test if 1hr is sufficient to generate adequate staining results when comparing to the longer hybridization times (2, 3, 4, 5, and 6hrs). All the time points including 1hr generate PIK3CA and CHR3 staining intensity 2.5 and above (≥2 acceptable), and staining coverage 80% and above (≥50% acceptable), and less than 0.5 background (≤1 acceptable) (Figure 4).

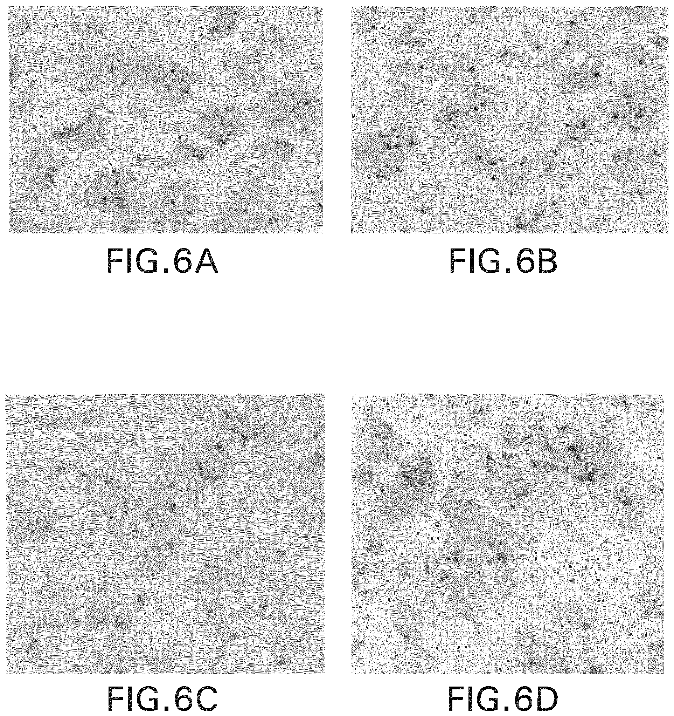

Most FISH studies targeted chromosome 3 centromere alpha satellite (D3Z1) sequence for CHR3 enumeration with plasmid pHS05 (EMBL accession number Z12006) (Alexandrov, Mitkevich et al. 1988, Alexandrov, Mashkova et al. 1993). These assays all require human blocking DNA to reduce background from cross-reactivity to other chromosome alpha satellite. We labeled plasmid pHS05 with DIG and compared its performance to that of the CHR3 Oligo Probe. First of all, in the absence of human blocking DNA, the plasmid pHS05 probe generated excessive number of red signals in the epithelial and stromal nuclei in a normal lung tissue (Figure 5). Next, we bulked the plasmid pHS05 probe with human placenta blocking DNA (2mg/ml) and stained with the CHR3 Oligo Probe pair-wise on the same lung specimens. Whereas the chromosome 3 signals stained with the plasmid pHS05 probe appeared to be specific (1-2 dots/nuclei), the signals often looked diffuse or smudge-like (Figure 6A&B). Moreover, in severe scenario, enumeration of discrete dots was a big challenge. In contrast, the CHR3 Oligo Probe usually generated discrete signals with regular size and shape (Figure 6C&D).

### Scoring 50 neoplastic nuclei with most copy numbers for the PIK3CA/CHR3 Oligo DISH assay

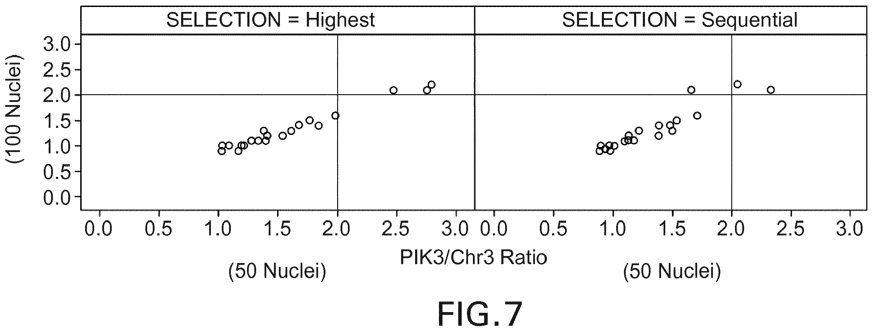

Up to now, no scoring criterion is available for bright-field PIK3CA/CHR dual ISH assay on lung tissues. FISH studies usually counted ∼100 nuclei on average. The minimal number is 20 on 419 primary tumor samples (Jehan, Bavi et al. 2009) (Kiyose, Nagura et al. 2012) counted 30 to 50 nuclei, and Costa C. et al.(Costa, Espinet et al. 2009) counted 200 in 26 primary tumor samples. PIK3CA and CHR3 signals were enumerated on 100 nuclei on 5 non-neoplastic lung tissues, 15 SCC tissues, and 6 other lung tumor types. PIK3CA/CHR3 ratio from the highest scores of 50 nuclei was consistent with the scores from the entire 100 nuclei enumeration (Figure 7). When sequentially selecting 50 nuclei, 25 out of 26 cases demonstrated consistent results (either ratio < 2 as normal or >2 as amplified) except for one case. This case had a ratio >2 with 100 nuclei counts, but <2 with 50 nuclei that were sequentially selected (Figure 7). These results prove 50 nuclei count is sufficient enough to generate consistent results as 100 nuclei; and also underscore the importance of counting neoplastic nuclei with most copy numbers (See MATERIALS AND METHODS "PIK3CA/CHR3 Oligo DISH Stain Scoring Criteria"). Counting 50 neoplastic nuclei with most copy numbers was therefore implemented for the rest of clinical specimen assessment.

### PIK3CA gene amplification mainly occurs in SCC (37%) by the PIK3CA/CHR3 Oligo DISH assay

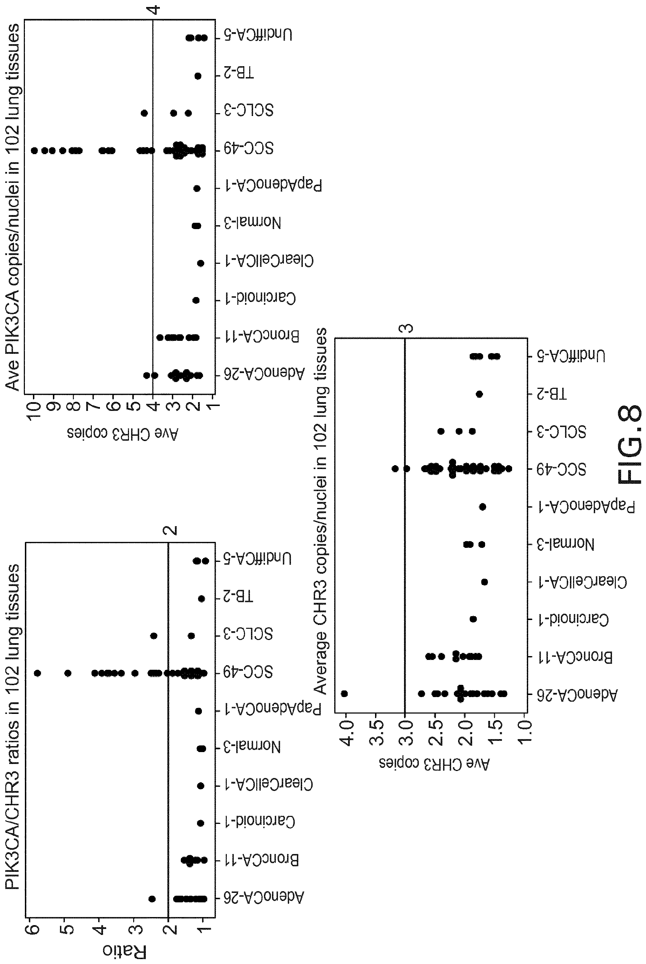

PIK3CA gene status was analyzed on a cohort of 102 lung tissue microarray specimens: 49 SCC, 26 adenocarcinomas, 11 bronchial carcinomas, 1 carcinoid, 1 clear cell carcinomas, 3 normal, 1 papillary adenocarcinoma, 3 small cell lung carcinomas (SCLC), 2 tuberculosis, TB granulomas, and 5 undifferentiated carcinomas. 100% 1st pass rate was achieved on the 102 tissues, in which all the staining is interpretable for PIK3CA and CHR3 copy numbers. The PIK3CA/CHR3 ratio, average PIK3CA copy per nuclei, and average CHR3 copy per nuclei for each of the 102 lung tissues are illustrated in Figure 8. In this cohort, SCC stands out with the highest incidence of elevated ratio and PIK3CA copy number from other tumor types. Only 1 adenocarcinoma with 4 copies of CHR3 and 1 SCC with 3.16 copies of CHR3 (Figure 8), chromosome 3 polysomy seems unlikely the driving force for PIK3CA copy number gain.

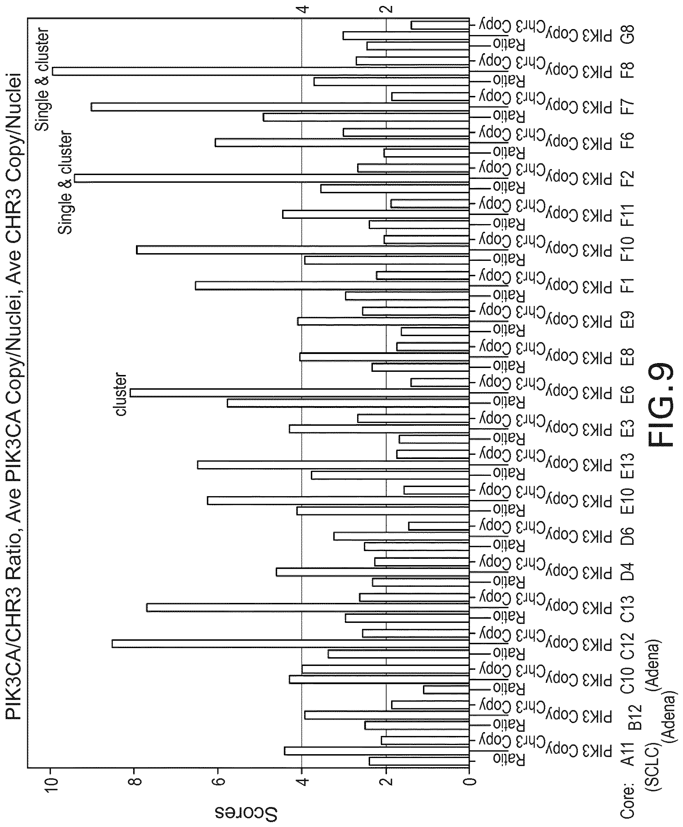

A total of 21 lung tissues have PIK3CA/CHR3 ratio greater than 2 and/or average PIK3CA copy number greater than 4, among which 15 have both ratio>2 and copy number >4. These include 18 SCC (36.7% of SCC), 2 adenocarcinomas (7.7% of adenocarcinomas) and 1 SCLC (1 out of 3 SCLC) (Figure 9). The PIK3CA/CHR3 ratio ranges from 2.03 to 5.77; the average PIK3 copy number per nuclei ranges from 3.00 to 9.96; the average CHR3 copy number per nuclei ranges from 1.38 to 4.02. Majority (18 out of 21) cases with PIK3CA copy number gain manifest multiple copies of discrete signals. Among the 21 cases, 20 demonstrate focal PIK3CA amplification in chromosome 3, only 1 case (C10, adenocarcinoma) has chromosome 3 polysomy.

Heterogeneity of PIK3CA gene copy gain in the 21 cases is presented in Table 7. In Case C12, C13, E6, F1, F2, F7, F8, and F10, almost all nuclei carry abnormally high copy number (4-9 and above per nuclei). In the remaining 13 cases, 12-80% of nuclei with normal (0-3) PIK3CA copies are mixed with abnormal (4-9 and above) nuclei. Regarding the copy number ranges of 4-9/nuclei, >10/nuclei, and >15/nuclei, 4-9 copies per nuclei represents the largest population of nuclei with PIK3CA gene amplification.

**TABLE 7. HETEROGENEITY OF PIK3CA GENE COPY NUMBER GAIN IN THE 21 LUNG TUMORS**

| Cores | Tumor type | # Nuclei | % 0-3 copies | % 4-9 copies | % >10 copies | % >15 copies |
|---|---|---|---|---|---|---|
| A11 | SCLC | 100 | 41 | 55 | 4 | |
| B12 | AdenoCa | 50 | 40 | 60 | | |
| C10 | AdenoCA | 50 | 38 | 60 | 2 | |
| C12 | SCC | 50 | | 68 | 28 | 4 |
| C13 | SCC | 50 | 2 | 76 | 14 | 8 |
| D4 | SCC | 100 | 33 | 62 | 5 | |
| D6 | SCC | 100 | 66 | 34 | | |
| E3 | SCC | 50 | 42 | 58 | | |
| E6 | SCC | 50 | 2 | 68 | 24 | 6 |
| E8 | SCC | 50 | 38 | 62 | | |
| E9 | SCC | 50 | 42 | 56 | 2 | |
| E10 | SCC | 50 | 22 | 56 | 20 | 2 |
| E13 | SCC | 50 | 12 | 74 | 14 | |
| F1 | SCC | 50 | 2 | 92 | 4 | |
| F2 | SCC | 50 | 0 | 50 | 42 | 8 |
| F6 | SCC | 50 | 10 | 76 | 14 | |
| F7 | SCC | 50 | 0 | 58 | 36 | 6 |
| F8 | SCC | 50 | 0 | 52 | 32 | 14 |
| F10 | SCC | 50 | 2 | 82 | 8 | 8 |
| F11 | SCC | 50 | 26 | 72 | 2 | |
| G8 | SCC | 50 | 80 | 20 | | |

With these 102 lung tissues, we also evaluated the CHR3 staining morphology with pHS05 plasmid in comparison to the CHR3 Oligo Probe. Thirty-seven (37) tissues were commented for inferior CHR3 staining quality (e.g. more red smudges, more red smears, increased red background), while only 2 cases stained with CHR3 Oligo Probe were commented for red signal variability.

### PIK3CA mRNA upregulation and protein overexpression mainly occurs in SCC by the PIK3CA mRNA ISH (45.3%) and p110 αIHC (75.5%)

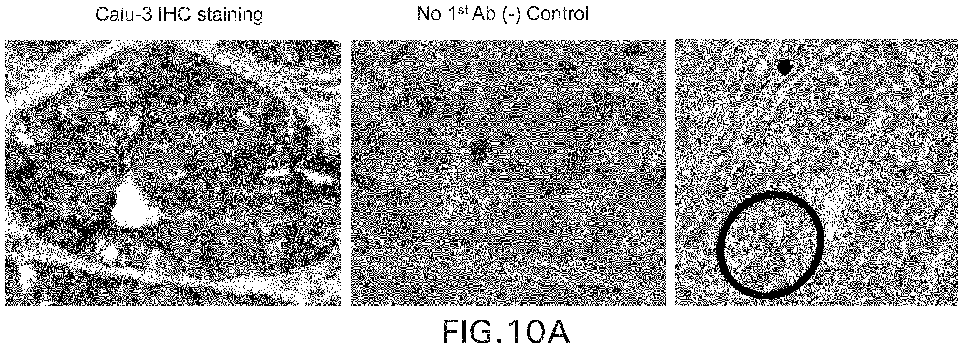

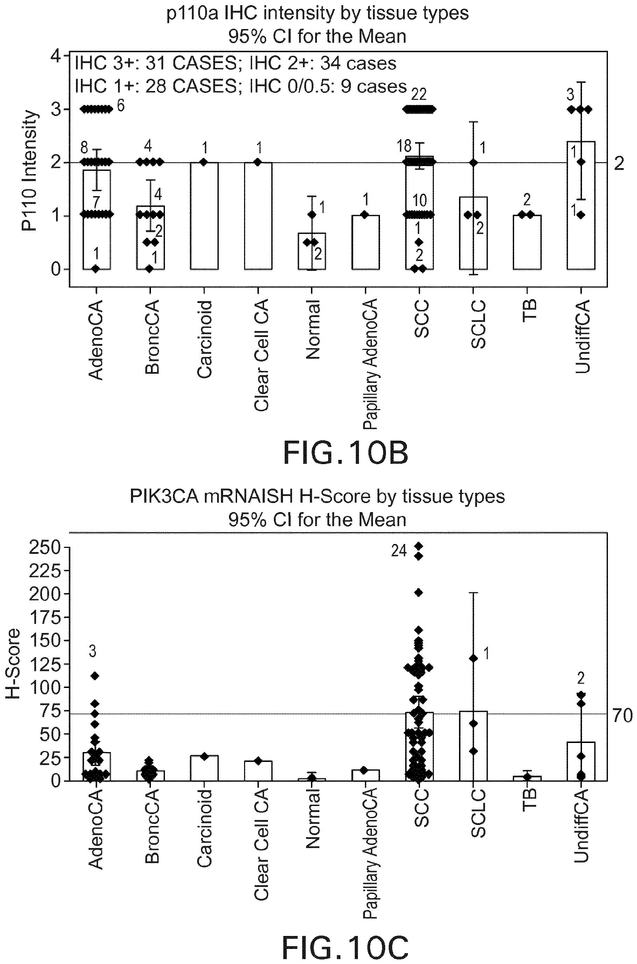

The PIK3CA mRNA ISH staining achieved 87.3% (89/102) pass rate. Thirteen (13) tissues failed QC as <70% of cells with 2+ intensity for PPIB staining. Thirty (30) cases have mRNA H score 70 and above, among which 24 are SCC (24/49, 49.0% of SCC), 3 adenocarcinoma (3/26, 11.5%), 1 SCLC and 2 Undifferentiated. Non-tumor tissues have the lowest mRNA expression (3 normal and 2 TB) (Figure 10C). To optimize p110α IHC assay condition, human kidney was selected for its known IHC staining patterns (Uhlen M 2010), and Calu-3 cell line was chosen for its known PIK3CA gene amplification. Cells in glomeruli showed low-to-medium staining (<25% of cells with strong intensity), while cells in tubules had high staining (>75% of cells with strong intensity). Calu-3 had high immunoreactive staining. p110α IHC staining featured a predominantly cytoplasmic and in some cases an accentuated membranous staining (Figure 10A). Focal and weak positive staining was found in some bronchiolar epithelial cells of normal lung tissue, and some of the lymphocytes and macrophages of tumor area, likely due to the role of PI3K in normal cell proliferation processes.

Ninety-nine (99) cases staining with p110α IHC demonstrated certain level of staining (0.5-3), 3 cases exhibited no staining in the entire tissue area. 65 cases exhibited p110α IHC intensity 2+ (34 cases) and 3+ (31 cases), among which 40 were SCC (40/49, 81.6% of SCC), 14 were adenocarcinomas (14/26, 53.8%), and 11 for other tumor types. Non-tumor tissues (3 normal and 2 TB) had the lowest p110α IHC intensity (0.5-1) (Figure 10B).

### 17 out of the 21 amplified cases (80.9%) have both mRNA and protein overexpression (15 cases) and protein overexpression (2 cases)

For the 21cases with PIK3CA copy number gain, fifteen cases (A11, C12, C13, D4, D6, E3, E6, E8, E10, E13, F2, F8, F10, F11, and G8) have both elevated mRNA level (H score >70) and protein overexpression (intensity 2 and 3). Two cases (C10 and F1) have p110alpha overexpression (intensity 2 and3), but mRNA H scores are below 70 (22 and 44, respectively). Three cases (B12, E9 and F7) have no over-expression at both mRNA and protein levels. One case (F6) has a low H score (5) for mRNA, and the IHC staining is not interpretable. Taken together, ∼80% of the cases with PIK3CA copy number gain have overexpression at mRNA and/or protein levels. Figure 10A showed the images of PIK3CA/CHR3 DISH, PIK3CA mRNA ISH and p100α IHC staining on a SCC (F3) with normal gene copy numbers (2.16, ratio 0.98), borderline mRNA expression (H score 75) and normal p110α expression (intensity 1, 80%). Figure 10B showed the DISH, mRNA ISH and IHC images on a SCC (F2) with gene copy gain (9.04, ratio 3.55), increased mRNA (H score 160) and protein expression (intensity 3, 80%).

While a number of exemplary aspects and embodiments have been discussed above, those of skill in the art will recognize certain modifications, permutations, additions and sub-combinations thereof as being present in the disclosure.

### REFERENCES

Yurov YB, Mitkevich SP, Alexandrov IA: Application of cloned satellite DNA-sequences to molecular-cytogenetic analysis of constitutive heterochromatin heteromorphisms in man. Hum Genet 1987, 76: 157e164.
Woenckhaus J, Steger K, Sturm K, Münstedt K, Franke FE, Fenic I: Prognostic value of PIK3CA and phosphorylated AKT expression in ovarian cancer. Virchows Arch 2007, 450:387e395.
Agell L, Hernández S, Salido M, de Muga S, Juanpere N, Arumi-Uria M, Menendez S, Lorenzo M, Lorente JA, Serrano S, Lloreta J:PI3K signaling pathway is activated by PIK3CA mRNA overexpression and copy gain in prostate tumors, but PIK3CA, BRAF, KRAS and AKT1 mutations are infrequent events. Mod Pathol 2011, 24:443e452.
Jang W, Yonescu R, Knutsen T, Brown T, Reppert T, Sirotkin K, Schuler GD, Ried T, Kirsch IR. Linking the human cytogenetic map with nucleotide sequence: the CCAP clone set. Cancer Genet Cytogenet 2006; 168(2): 89-97.
De Marco C, Rinaldo N, Bruni P, Malzoni C, Zullo F, Fabiani F, Losito S, Scrima M, Marino FZ, Franco R, Quintiero A, Agosti V, Viglietto G: Multiple genetic alterations within the PI3K pathway are responsible for AKT activation in patients with ovarian carcinoma. PLoS One 2013, 8:e55362.
Nakayama K, Nakayama N, Kurman RJ, Cope L, Pohl G, Samuels Y, Velculescu VE, Wang TL, Shih IM: Sequence mutations and amplification of PIK3CA and AKT2 genes in purified ovarian serous neoplasms. Cancer Biol Ther 2006, 5:779e785.
Taneja K, Singer RH: Use of oligodeoxynucleotide probes for quantitative in situ hybridization to actin messenger-RNA. Anal Biochem 1987, 166:389e398.
Lewis ME, Sherman TG, Watson SJ: In situ hybridization histochemistry with synthetic oligonucleotides: strategies and methods. Peptides 1985, 6(Suppl 2):75e87.
Strachan T., Read AP. Human molecular genetics 3. 1st ed. New York : Garland Science/Taylor & Francis Group, 1999.[Book]
Kourilsky P, Mercereau O, Gros D, Tremblay G.Biochimie. Hybridization of filters with competitor DNA in the liquid phase in a standard and a micro-assay. 1974;56(9):1215-21.
An SF, Franklin D, Fleming KA. Generation of digoxigenin-labelled double-stranded and single-stranded probes using the polymerase chain reaction. Mol Cell Probes. 1992 Jun;6(3):193-200.
Hannon K, Johnstone E, Craft LS, Little SP, Smith CK, Heiman ML, Santerre RF. Synthesis of PCR-derived, single-stranded DNA probes suitable for in situ hybridization. Anal Biochem. 1993 Aug 1;212(2):421-7.
Cox KH, DeLeon DV, Angerer LM, Angerer RC. Detection of mrnas in sea urchin embryos by in situ hybridization using asymmetric RNA probes. Dev Biol. 1984 Feb;101(2):485-502.
Alexandrov IA, Mashkova TD, Romanova LY, Yurov YB, Kisselev LL: Segment substitutions in alpha satellite DNA. Unusual structure of human chromosome 3-specific alpha satellite repeat unit. J Mol Biol 1993, 231:516e520.
Minna JD, Roth JA, Gazdar AF. Focus on lung cancer. Cancer Cell. 2002 Feb;1(1):49-52.
Zochbauer-Muller S, Gazdar AF, Minna JD. Molecular pathogenesis of lung cancer. Annu Rev Physiol. 2002;64:681-708.
Lam DCL, Clinical testing for molecular targets for personalized treatment in lung cancer. Respirology. 2013; 18(2): 233-237.
Villaruz LC, Burns TF, Ramfidis VS, Socinski MA. Personalizing therapy in advanced non-small cell lung cancer. Semin Respir Crit Care Med. 2013 Dec;34(6):822-36.
Ji M, Guan H, Gao C, Shi B, and Hou P. Highly frequent promoter methylation and PIK3CA amplification in non-small cell lung cancer (NSCLC). BMC Cancer. 2011; 11: 147.
Lockwood WW, Wilson IM, Coe BP, Chari R, Pikor LA, Thu KL, Solis LM, Nunez MI, Behrens C, Yee J, English J, Murray N, Tsao MS, Minna JD, Gazdar AF, Wistuba II, MacAulay CE, Lam S, Lam WL. Divergent genomic and epigenomic landscapes of lung cancer subtypes underscore the selection of different oncogenic pathways during tumor development. PLoS One. 2012;7(5):e37775.
Kok K, Geering B, Vanhaesebroeck B: Regulation of phosphoinositide 3-kinase expression in health and disease. Trends Biochem Sci 2009, 34:115e127.
Massion PP, Kuo WL, Stokoe D, Olshen AB, Treseler PA, Chin K, Chen C, Polikoff D, Jain AN, Pinkel D, Albertson DG, Jablons DM, Gray JW: Genomic copy number analysis of non-small cell lung cancer using array comparative genomic hybridization: implications of the phosphatidylinositol 3-kinase pathway. Cancer Res 2002, 62: 3636e3640.
Okudela K, Suzuki M, Kageyama S, Bunai T, Nagura K, Igarashi H, Takamochi K, Suzuki K, Yamada T, Niwa H, Ohashi R, Ogawa H, Mori H, Kitamura H, Kaneko T, Tsuneyoshi T, Sugimura H: PIK3CA mutation and amplification in human lung cancer. Pathol Int 2007, 57: 664e671.
Yamamoto H, Shigematsu H, Nomura M, Lockwood WW, Sato M, Okumura N, Soh J, Suzuki M, Wistuba II, Fong KM, Lee H, Toyooka S, Date H, Lam WL, Minna JD, Gazdar AF: PIK3CA mutations and copy number gains in human lung cancers. Cancer Res 2008, 68:6913e6921.
Spoerke JM, O'Brien C, Huw L, Koeppen H, Fridlyand J, Brachmann RK, Haverty PM, Pandita A, Mohan S, Sampath D, Friedman LS, Ross L, Hampton GM, Amler LC, Shames DS, Lackner MR: Phosphoinositide 3-kinase (PI3K) pathway alterations are associated with histologic subtypes and are predictive of sensitivity to PI3K inhibitors in lung cancer preclinical models. Clin Cancer Res 2012, 18:6771e6783.
Woenckhaus J, Steger K, Werner E, Fenic I, Gamerdinger U, Dreyer T, Stahl U: Genomic gain of PIK3CA and increased expression of p110alpha are associated with progression of dysplasia into invasive squamous cell carcinoma. J Pathol 2002, 198:335e342.
Wee S, Lengauer C, Wiederschain D: Class IA phosphoinositide 3-kinase isoforms and human tumorigenesis: implications for cancer drug discovery and development. Curr Opin Oncol 2008, 20:77e82.
Salphati L, Wong H, Belvin M, Bradford D, Edgar KA, Prior WW, Sampath D, and Wallin JJ. Pharmacokinetic-Pharmacodynamic Modeling of Tumor Growth Inhibition and Biomarker Modulation by the Novel Phosphatidylinositol 3-Kinase Inhibitor GDC-0941. Drug Metabolism and Disposition. 2010; 38 (9):1436-1442.
Blumenthal GM, Orbach RC, Zineh I, Cortazar P, Justice RL, Pazdur R. Early targeted drug development: Pilot FDA review of PI3K inhibitor phase I studies using a knowledge management database. J Clin Oncol 29: 2011 (suppl; abstr 3060).
Salphati L, Pang J, Plise EG, Chou B, Halladay JS, Olivero AG, Rudewicz PJ, Tian Q, Wong S, Zhang X. Preclinical pharmacokinetics of the novel PI3K inhibitor GDC-0941 and prediction of its pharmacokinetics and efficacy in human. Xenobiotica. 2011 Dec;41(12):1088-99.
Angulo B, Suarez-Gauthier A, Lopez-Rios F, Medina PP, Conde E, Tang M, Soler G, Lopez-Encuentra A, Cigudosa JC, Sanchez-Cespedes M. Expression signatures in lung cancer reveal a profile for EGFR-mutant tumours and identify selective PIK3CA overexpression by gene amplification. J Pathol. 2008 Feb;214(3):347-56.
Scrima M, De Marco C, Fabiani F, Franco R, Pirozzi G, Rocco G, Ravo M, Weisz A, Zoppoli P, Ceccarelli M, Botti G, Malanga D, Viglietto G. Signaling Networks Associated with AKT Activation in Non-Small Cell Lung Cancer (NSCLC): New Insights on the Role of Phosphatydil-Inositol-3 kinase. PLOS One. February 17, 2012DOI: 10.1371/journal.pone.0030427.
Shayesteh L, Lu Y, Kuo WL, Baldocchi R, Godfrey T, Collins C, Pinkel D, Powell B, Mills GB, Gray JW: PIK3CA is implicated as an oncogene in ovarian cancer. Nat Genet 1999, 21:99e102.
Psyrri A, Papageorgiou S, Liakata E, Scorilas A, Rontogianni D, Kontos CK, Argyriou P, Pectasides D, Harhalakis N, Pappa V, Kolialexi A, Economopoulou C, Kontsioti F, Maratou E, Dimitriadis G, Economopoulou P, Economopoulos T. Phosphatidylinositol 3'-kinase catalytic subunit alpha gene amplification contributes to the pathogenesis of mantle cell lymphoma. Clin Cancer Res. 2009 Sep 15;15(18):5724-32.
Jehan Z, Bavi P, Sultana M, Abubaker J, Bu R, Hussain A, Alsbeih G, Al-Sanea N, Abduljabbar A, Ashari LH, Alhomoud S, Al-Dayel F, Uddin S, Al-Kuraya KS. Frequent PIK3CA gene amplification and its clinical significance in colorectal cancer. J Pathol. 2009 Nov;219(3):337-46.
Kiyose S, Nagura K, Tao H, Igarashi H, Yamada H, Goto M, Maeda M, Kurabe N, Suzuki M, Tsuboi M, Kahyo T, Shinmura K, Hattori N, Sugimura H. Detection of kinase amplifications in gastric cancer archives using fluorescence in situ hybridization. Pathol Int. 2012 Jul;62(7):477-84.
Costa C, Espinet B, Molina MA, Salgado R, Salido M, Baró T, Fusté P, Mancebo G, Carreras R, Solé F, Serrano S, Alameda F. Analysis of gene status in cervical dysplastic lesions and squamous cell carcinoma using tissue microarrays. Histol Histopathol. 2009 Jul;24(7):821-9.

## Claims

1. A system for *in situ* hybridization comprising
a control probe specific to a control region of chromosome 3, wherein the control probe is labeled with at least one first label, wherein the control probe is a first plurality of single-stranded oligonucleotide probes, each oligonucleotide probe comprising:
a sequence selected from the group consisting of SEQ ID NOs: 1-18; or
a sequence selected from the group consisting of a truncated version of SEQ ID NOs: 1-18, the truncated version being at least 40 contiguous basepairs (bp) of said SEQ ID NOs:1-18; or
a sequence selected from the group consisting of a sequence that has at least 70% sequence identity to one of SEQ ID NOs: 1-18.

2. The system of claim 1, wherein each oligonucleotide probe comprises
a sequence selected from the group consisting of a sequence that has at least 80% sequence identity to one of SEQ ID NOs: 1-18, or
a sequence selected from the group consisting of a sequence that has at least 90% sequence identity to one of SEQ ID NOs: 1-18.

3. The system of any of claims 1 or 2, wherein the control probes each comprise between 50 to 100 nucleotides.

4. The system of any of claims 1 or 2, wherein the first plurality of single-stranded oligonucleotide probes target between 2 and 18 distinct portions within the control region
or wherein the first plurality of single-stranded oligonucleotide probes target between 4 and 18 distinct portions within the control region
or wherein the first plurality of single-stranded oligonucleotide probes target between 6 and 18 distinct portions within the control region or wherein the first plurality of single-stranded oligonucleotide probes target between 8 and 18 distinct portions within the control region
or wherein the first plurality of single-stranded oligonucleotide probes target between 10 and 18 distinct portions within the control region.

5. The system of any of claims 1 to 4, wherein the control probes are each labeled with at least 2, at least 3, at least 4, or at least 5 first labels.

6. The system of any of claims 1 to 5, wherein the first label comprises a digoxigenin (DIG).

7. The system of any of claims 1 to 6, further comprising a target probe specific to a target region of human chromosome 3, wherein the target probe is labeled with at least one second label.

8. The system of claim 7, wherein the target probe is specific to a target region near or around the PIK3CA gene locus, or wherein the target probe is specific to a region between nucleotides 178,640,071 and 179,399,807 of human chromosome 3.

9. The system of any of claims 1 to 8, further comprising an *in situ* hybridization staining instrument, the instrument is configured to contact the control probe to a tissue sample.

10. A kit comprising a vessel containing a system according to any of claims 1 to 9 and instructions for use.

11. A slide comprising a plurality of nuclei chromogenically stained for human chromosome 3, wherein the slide is made using a system according to any of the claims 1 to 9.

12. A method for *in situ* hybridization of a tissue sample, the method comprising contacting the tissue sample with a system according to any of claims 1 to 9.

13. A method of scoring for a chromosome for PIK3CA gene copy amplification, said method comprising:
obtaining a tissue sample having undergone *in situ* hybridization according to claim 12, wherein a control probe and target probe are used;
identifying an area of neoplastic nuclei with most copy numbers; and
counting enumerable signals for PIK3CA signal in 50-100 neoplastic nuclei and either 50 adjacent mesenchymal nuclei or 50 adjacent normal epithelial nuclei;
wherein scoring criteria comprises: no staining or <1 dot/ 10 cells is scored as 0; 1-3 dots/cell is scored as 1; 4-9 dots/cell, none or very few dot clusters is scored as 2; 10-15 dots/cell and <10% dots are in clusters is scored as 3; and >15 dots/cell and >10% dots are in clusters is scored as 4.

14. The method of claim 13 further comprising calculating the ratio of PIK3CA signal to control signal.

15. The method of claim 13, further comprising calculating the average number of PIK3CA copies per nuclei.

## Patentansprüche

1. System für *In-situ*-Hybridisierung, das Folgendes umfasst:
eine Kontrollsonde, die spezifisch für eine Kontrollregion des Chromosoms 3 ist, wobei die Kontrollsonde mit mindestens einer ersten Markierung markiert ist, wobei die Kontrollsonde eine erste Mehrzahl von einzelsträngigen Oligonukleotidsonden ist, wobei jede Oligonukleotidsonde Folgendes umfasst:
eine Sequenz, die aus der Gruppe ausgewählt ist, die aus den SEQ ID NO: 1-18 besteht; oder
eine Sequenz, die aus der Gruppe ausgewählt ist, die aus einer verkürzten Version der SEQ ID NO: 1-18 besteht, wobei die verkürzte Version aus mindestens 40 zusammenhängenden Basenpaaren (bp) dieser SEQ ID NO: 1-18 besteht; oder
eine Sequenz, die aus der Gruppe ausgewählt ist, die aus einer Sequenz besteht, die mindestens 70 % Sequenzidentität mit einer der SEQ ID NO: 1-18 aufweist.

2. System nach Anspruch 1, wobei jede Oligonukleotidsonde Folgendes umfasst:
eine Sequenz, die aus der Gruppe ausgewählt ist, die aus einer Sequenz besteht, die mindestens 80 % Sequenzidentität mit einer der SEQ ID NO: 1-18 aufweist, oder
eine Sequenz, die aus der Gruppe ausgewählt ist, die aus einer Sequenz besteht, die mindestens 90 % Sequenzidentität mit einer der SEQ ID NO: 1-18 aufweist.

3. System nach einem der Ansprüche 1 oder 2, wobei jede Kontrollsonde zwischen 50 bis 100 Nukleotide umfasst.

4. System nach einem der Ansprüche 1 oder 2, wobei die erste Mehrzahl von einzelsträngigen Oligonukleotidsonden auf zwischen 2 und 18 verschiedene Abschnitte innerhalb der Kontrollregion abzielt
oder wobei die erste Mehrzahl von einzelsträngigen Oligonukleotidsonden auf zwischen 4 und 18 verschiedene Abschnitte innerhalb der Kontrollregion abzielt od
er wobei die erste Mehrzahl von einzelsträngigen Oligonukleotidsonden auf zwischen 6 und 18 verschiedene Abschnitte innerhalb der Kontrollregion abzielt
oder wobei die erste Mehrzahl von einzelsträngigen Oligonukleotidsonden auf zwischen 8 und 18 verschiedene Abschnitte innerhalb der Kontrollregion abzielt
oder wobei die erste Mehrzahl von einzelsträngigen Oligonukleotidsonden auf zwischen 10 und 18 verschiedene Abschnitte innerhalb der Kontrollregion abzielt.

5. System nach einem der Ansprüche 1 bis 4, wobei jede Kontrollsonde mit mindestens 2, mindestens 3, mindestens 4 oder mindestens 5 ersten Markierungen markiert ist.

6. System nach einem der Ansprüche 1 bis 5, wobei die erste Markierung ein Digoxigenin (DIG) umfasst.

7. System nach einem der Ansprüche 1 bis 6, das weiterhin eine Zielsonde umfasst, die spezifisch für eine Zielregion des humanen Chromosoms 3 ist, wobei die Zielsonde mit mindestens einer zweiten Markierung markiert ist.

8. System nach Anspruch 7, wobei die Zielsonde spezifisch für eine Zielregion in der Nähe oder in der Umgebung von dem PIK3CA-Genlocus ist, oder wobei die Zielsonde spezifisch für eine Region zwischen Nukleotiden 178 640 071 und 179 399 807 des humanen Chromosoms 3 ist.

9. System nach einem der Ansprüche 1 bis 8, das weiterhin ein *In-situ*-Hybridisierungs-Färbeinstrument umfasst, wobei das Instrument dafür ausgelegt ist, die Kontrollsonde mit einer Gewebeprobe in Kontakt zu bringen.

10. Kit, das ein Gefäß umfasst, das ein System nach einem der Ansprüche 1 bis 9 und Gebrauchsanweisungen enthält.

11. Objektträger, der eine Mehrzahl von Nuklei umfasst, die für das humane Chromosom 3 chromogen gefärbt wurden, wobei der Objektträger mittels eines Systems nach einem der Ansprüche 1 bis 9 hergestellt wurde.

12. Verfahren für die *In-situ*-Hybridisierung einer Gewebeprobe, wobei das Verfahren umfasst, die Gewebeprobe mit einem System nach einem der Ansprüche 1 bis 9 in Kontakt zu bringen.

13. Verfahren für das Bewerten eines Chromosoms für die PIK3CA-Genkopieamplifikation, wobei das Verfahren Folgendes umfasst:
Erhalten einer Gewebeprobe, die *In-situ*-Hybridisierung nach Anspruch 12 durchlaufen hat, wobei eine Kontrollsonde und eine Zielsonde verwendet werden;
Identifizieren eines Bereichs von neoplastischen Nuklei mit den größten Kopienanzahlen; und
Zählen von zählbaren Signalen für das PIK3CA-Signal in 50-100 neoplastischen Nuklei und entweder 50 benachbarten mesenchymalen Nuklei oder 50 benachbarten normalen epithelialen Nuklei;
wobei die Bewertungskriterien Folgendes umfassen: keine Färbung oder <1 Fleck/10 Zellen wird mit 0 bewertet; 1-3 Flecke/Zelle wird mit 1 bewertet; 4-9 Flecke/Zelle, keine oder sehr wenige Fleckcluster werden mit 2 bewertet; 10-15 Flecke/Zelle und wenn <10 % der Flecke in Clustern vorliegen, wird dies mit 3 bewertet; und > 15 Flecke/Zelle und wenn >10 % der Flecke in Clustern vorliegen, wird dies mit 4 bewertet.

14. Verfahren nach Anspruch 13, das weiterhin das Berechnen des Verhältnisses des PIK3CA-Signals zu dem Kontrollsignal umfasst.

15. Verfahren nach Anspruch 13, das weiterhin das Berechnen der durchschnittlichen Anzahl von PIK3CA-Kopien pro Nukleus umfasst.

## Revendications

1. Système pour hybridation *in situ,* comprenant
une sonde de contrôle spécifique d'une région de contrôle du chromosome 3, laquelle sonde de contrôle est marquée avec au moins un premier marqueur, laquelle sonde de contrôle est une première pluralité de sondes oligonucléotidiques monocaténaires, chaque sonde oligonucléotidique comprenant :
une séquence choisie dans le groupe constitué par les SEQ ID NO: 1-18 ; ou
une séquence choisie dans le groupe constitué par une version tronquée des SEQ ID NO: 1-18, la version tronquée étant au moins 40 paires de bases (pb) contiguës desdites SEQ ID NO: 1-18 ; ou
une séquence choisie dans le groupe constitué par une séquence qui présente au moins 70 % d'identité de séquence avec l'une des SEQ ID NO: 1-18.

2. Système selon la revendication 1, dans lequel chaque sonde oligonucléotidique comprend
une séquence choisie dans le groupe constitué par une séquence qui présente au moins 80 % d'identité de séquence avec l'une des SEQ ID NO: 1-18, ou
une séquence choisie dans le groupe constitué par une séquence qui présente au moins 90 % d'identité de séquence avec l'une des SEQ ID NO: 1-18.

3. Système selon l'une quelconque des revendications 1 ou 2, dans lequel les sondes de contrôle comprennent chacune entre 50 à 100 nucléotides.

4. Système selon l'une quelconque des revendications 1 ou 2, dans lequel la première pluralité de sondes oligonucléotidiques monocaténaires cible entre 2 et 18 parties distinctes au sein de la région de contrôle
ou dans lequel la première pluralité de sondes oligonucléotidiques monocaténaires cible entre 4 et 18 parties distinctes au sein de la région de contrôle
ou dans lequel la première pluralité de sondes oligonucléotidiques monocaténaires cible entre 6 et 18 parties distinctes au sein de la région de contrôle
ou dans lequel la première pluralité de sondes oligonucléotidiques monocaténaires cible entre 8 et 18 parties distinctes au sein de la région de contrôle
ou dans lequel la première pluralité de sondes oligonucléotidiques monocaténaires cible entre 10 et 18 parties distinctes au sein de la région de contrôle.

5. Système selon l'une quelconque des revendications 1 à 4, dans lequel les sondes de contrôle sont chacune marquées avec au moins 2, au moins 3, au moins 4, ou au moins 5 premiers marqueurs.

6. Système selon l'une quelconque des revendications 1 à 5, dans lequel le premier marqueur comprend une digoxigénine (DIG).

7. Système selon l'une quelconque des revendications 1 à 6, comprenant en outre une sonde cible spécifique d'une région cible du chromosome 3 humain, dans lequel la sonde cible est marquée avec au moins un second marqueur.

8. Système selon la revendication 7, dans lequel la sonde cible est spécifique d'une région cible à proximité ou autour du locus du gène PIK3CA, ou dans lequel la sonde cible est spécifique d'une région entre les nucléotides 178 640 071 et 179 399 807 du chromosome 3 humain.

9. Système selon l'une quelconque des revendications 1 à 8, comprenant en outre un instrument de coloration d'hybridation *in situ,* l'instrument étant configuré pour mettre en contact la sonde de contrôle avec un échantillon de tissu.

10. Kit comprenant un récipient contenant un système selon l'une quelconque des revendications 1 à 9 et une notice d'utilisation.

11. Lame comprenant une pluralité de noyaux colorés de manière chromogène pour le chromosome 3 humain, la lame étant préparée en utilisant un système selon l'une quelconque des revendications 1 à 9.

12. Méthode pour l'hybridation *in situ* d'un échantillon de tissu, la méthode comprenant la mise en contact de l'échantillon de tissu avec un système selon l'une quelconque des revendications 1 à 9.

13. Méthode de notation d'un chromosome pour l'amplification des copies du gène PIK3CA, ladite méthode comprenant :
l'obtention d'un échantillon de tissu ayant subi une hybridation *in situ* selon la revendication 12, dans laquelle sont utilisées une sonde de contrôle et une sonde cible ;
l'identification d'une zone de noyaux néoplasiques comprenant la plupart des nombres de copies ; et
le comptage de signaux dénombrables pour le signal de PIK3CA dans 50 à 100 noyaux néoplasiques et soit 50 noyaux mésenchymateux adjacents, soit 50 noyaux épithéliaux normaux adjacents ;
dans laquelle les critères de notation comprennent : aucune coloration ou <1 point/10 cellules est noté 0 ; 1 à 3 points/cellule est noté 1 ; 4 à 9 points/cellule, absence ou présence de très peu d'amas de points est noté 2 ; 10 à 15 points/cellule avec <10 % des points en amas est noté 3 ; et > 15 points/cellule avec > 10 % de points en amas est noté 4.

14. Méthode selon la revendication 13, comprenant en outre le calcul du rapport du signal de PIK3CA au signal de contrôle.

15. Méthode selon la revendication 13, comprenant en outre le calcul du nombre moyen de copies de PIK3CA par noyau.
